# EUROPÄISCHE PATENTSCHRIFT

(11) **EP 3 479 895 B1**
(45) Veröffentlichungstag und Bekanntmachung des Hinweises auf die Patenterteilung: **25.03.2020**
(21) Anmeldenummer: 18203106.2
(22) Anmeldetag: 29.10.2018
(51) Int. Cl.: B01F 15/02

(54) **PULVER-FLÜSSIGKEITS-KNOCHENZEMENTMISCHER MIT DRUCKGASANSCHLUSS UND VERFAHREN**
POWDER LIQUID BONE CEMENT MIXER WITH CONNECTOR FOR PRESSURISED GAS AND METHOD
MÉLANGEUR DE CIMENT OSSEUX, DE LIQUIDE ET DE POUDRE POURVU DE RACCORDEMENT DE GAZ DE PRESSION ET PROCÉDÉ

(30) Priorität: 02.11.2017 DE 102017125592
(43) Veröffentlichungstag der Anmeldung: 08.05.2019
(73) Patentinhaber: Heraeus Medical GmbH, 61273 Wehrheim (DE)
(72) Erfinder: Vogt, Sebastian, Dr., 99092 Erfurt (DE); Kluge, Thomas, Dr., 56179 Vallendar (DE)
(74) Vertreter: Schultheiss & Sterzel Patentanwälte PartG mbB

(56) Entgegenhaltungen:
- EP-A1- 1 886 648
- WO-A1-95/01809
- WO-A1-97/18031
- US-A- 5 193 907
- US-A1- 2001 008 968
- US-A1- 2004 066 706

## Beschreibung

Die Erfindung betrifft eine Vorrichtung zum Herstellen eines Knochenzementteigs aus einer Monomerflüssigkeit und einem Zementpulver als Ausgangskomponenten des Knochenzementteigs.

Die Erfindung betrifft auch ein Verfahren zur Herstellung eines Knochenzementteigs, insbesondere eines pastenförmigen Polymethylmethacrylat(PMMA)-Knochenzementteigs.

Gegenstand der Erfindung ist insbesondere eine Vorrichtung zum separaten Lagern des Zementpulvers und der Monomerflüssigkeit von Polymethylmethacrylat-Knochenzement, zum anschließenden Vermischen des Zementpulvers mit der Monomerflüssigkeit zur Bildung eines Knochenzementteigs und zum Austragen des gemischten Knochenzementteigs. Die Vorrichtung ist besonders für die Befüllung von Spritzen mit PMMA-Knochenzementteig für die Vertebroplastie bestimmt. Weiterhin ist die Befüllung von Kyphoplastie-Systemen mit PMMA-Knochenzementteig möglich. Es handelt sich bei der erfindungsgemäßen Vorrichtung bevorzugt um ein Full-Prepacked-Zementiersystem.

Polymethylmethacrylat-(PMMA)-Knochenzemente gehen auf die grundlegenden Arbeiten von Sir Charnley zurück (Charnley, J.: Anchorage of the femoral head prosthesis of the shaft of the femur. J. Bone Joint Surg. 42 (1960) 28-30.). Die Monomerkomponente enthält im Allgemeinen das Monomer Methylmethacrylat und einen darin gelösten Aktivator (N,N-Dimethyl-p-toluidin). Die Pulverkomponente, auch als Zementpulver oder Knochenzementpulver bezeichnet, weist ein oder mehrere Polymere auf, die auf Basis von Methylmethacrylat und Comonomeren, wie Styren, Methylacrylat oder ähnlichen Monomeren durch Polymerisation, vorzugsweise Suspensionspolymerisation, hergestellt sind, einen Röntgenopaker und den Initiator Dibenzoylperoxid auf. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement beziehungsweise Knochenzementteig. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylats. Mit fortschreitender Polymerisation des Methylmethacrylats erhöht sich die Viskosität des Knochenzementteigs, bis dieser erstarrt.

PMMA-Knochenzemente können in geeigneten Mischbechern mit Hilfe von Spateln durch manuelles Vermischen des Zementpulvers mit der Monomerflüssigkeit vermischt werden.

Zur Vermeidung von Lufteinschlüssen im Knochenzementteig wurden eine Vielzahl von Vakuum-Zementiersystemen beschrieben, von denen exemplarisch folgende genannt sind: US 6 033 105 A, US 5 624 184 A, US 4 671 263 A, US 4 973 168 A, US 5 100 241 A, WO 99/67015 A1, EP 1 020 167 A2, US 5 586 821 A, EP 1 016 452 A2, DE 36 40 279 A1, WO 94/26403 A1, EP 1 005 901 A2, EP 1 886 647 A1, US 5 344 232 A.

Aus den Patenten DE 10 2010 019 220 B4, EP 2 596 873 B1 und DE 10 2013 226 118 B3 sowie der Patentanmeldung DE 10 2014 101 305 A1 sind Vorrichtungen zum Mischen von PMMA-Knochenzementen aus zwei pastösen Ausgangskomponenten bekannt.

Eine Weiterentwicklung in der Zementiertechnik stellen Zementiersysteme dar, in denen sowohl das Zementpulver als auch die Monomerflüssigkeit bereits in separaten Kompartimenten der Mischvorrichtungen verpackt sind und die erst unmittelbar vor der Zementapplikation im Zementiersystem miteinander vermischt werden. Solche geschlossenen Full-Prepacked-Mischvorrichtungen wurden mit der EP 0 692 229 A1, der DE 10 2009 031 178 B3, der US 5 997 544 A, der US 6 709 149 B1, der DE 698 12 726 T2, der EP 0 796 653 A2 und der US 5 588 745 A vorgeschlagen.

Das Patent DE 10 2009 031 178 B3 offenbart eine Lager- und Mischvorrichtung als Full-Prepacked-Zementiersystem, in dem die zur Herstellung des Knochenzementteigs notwendigen Ausgangskomponenten bereits in der Lager- und Mischvorrichtung gelagert sind und in der Lager- und Mischvorrichtung zusammengeführt und gemischt werden können. Die Lager- und Mischvorrichtung weist einen zweiteiligen Austragskolben zum Verschluss einer Zementkartusche auf. Dabei wird eine Kombination aus einem gasdurchlässigen Sterilisationskolben und einem gasundurchlässigen Dichtungskolben verwendet.

Polymethylmethacrylat-Knochenzemente werden nach Vermischung des Zementpulvers mit der flüssigen Monomerkomponente im noch nicht ausgehärteten, pastenförmigen Zustand als Knochenzementteig appliziert. Bei Verwendung von Mischvorrichtungen befindet sich der Knochenzementteig im Fall von Pulver-Flüssigkeits-Zementen in einer Kartusche. Bei der Applikation solcher konventioneller PMMA-Knochenzemente, wird nach der Vermischung der beiden Ausgangskomponenten der gebildete Knochenzementteig mit Hilfe von manuell bedienbaren Auspressvorrichtungen ausgepresst. Aus der Kartusche wird der Knochenzementteig durch Bewegung eines Austragskolbens herausgedrückt.

Bei der Verwendung aller bisher bekannten Full-Prepacked-Zementiersysteme muss der medizinische Anwender mehrere Arbeitsschritte in einer vorbestimmten Reihenfolge an den Vorrichtungen nacheinander durchführen bis der gemischte Knochenzementteig vorliegt und appliziert werden kann. Eine Verwechslung der Arbeitsschritte kann zum Versagen der Mischvorrichtung führen und daher Störungen im OP-Ablauf verursachen. Kostenintensive Schulungen der medizinischen Anwender sind deshalb notwendig, um Anwenderfehler zu vermeiden.

In der WO 00/35506 A1 wird eine Vorrichtung vorgeschlagen, bei der Polymethylmethacrylat-Zementpulver in einer Kartusche gelagert wird, wobei das Zementpulver das gesamte Volumen der Kartusche ausfüllt und die Zwischenräume zwischen den Partikeln des Zementpulvers ein solches Volumen hat, das dem Volumen der Monomerflüssigkeit entspricht, das zur Herstellung von Knochenzementteig mit dem in der Kartusche gelagerten Zementpulver notwendig ist. Diese Vorrichtung ist so aufgebaut, dass durch Vakuumeinwirkung die Monomerflüssigkeit von oben in die Kartusche eingeleitet wird, wobei hierzu ein Vakuum an einem Vakuumanschluss an der Unterseite der Kartusche angelegt wird. Dadurch wird die Monomerflüssigkeit durch das Zementpulver gezogen, wobei die in den Zwischenräumen der Zementpulverpartikel befindliche Luft durch die Monomerflüssigkeit verdrängt wird. Auf eine mechanische Durchmischung des gebildeten Zementteigs mit einem Rührer wird dabei verzichtet.

Nachteilig an diesem System ist, dass Zementpulver, die schnell mit der Monomerflüssigkeit anquellen, mit dieser Vorrichtung nicht angemischt werden können, weil die schnell anquellenden Zementpulverpartikel nach einem Eindringen der Monomerflüssigkeit in das Zementpulver von ungefähr 1 bis 2 cm eine gelartige Barriere bilden und die Migration der Monomerflüssigkeit durch das gesamte Zementpulver behindern. Konventionelle Zementpulver zeigen zudem das Phänomen, dass auf Grund der unterschiedlichen Oberflächenenergien die Zementpulverpartikel durch Methylmethacrylat nur schlecht benetzt werden. Dadurch dringt das Methylmethacrylat nur relativ langsam in das Zementpulver ein. Weiterhin kann bei Vakuumeinwirkung nicht ausgeschlossen werden, dass nach vollständiger Durchdringung des Zementpulvers durch die Monomerflüssigkeit die Monomerflüssigkeit über den Vakuumanschluss abgesaugt wird. Dann steht nicht genügend Monomerflüssigkeit für die Aushärtung durch radikalische Polymerisation zur Verfügung beziehungsweise das Mischungsverhältnis wird ungewollt verändert und damit auch die Konsistenz des Knochenzementteigs. Weiterhin ist es problematisch, dass die zwischen den Zementpulverpartikeln eingeschlossen Luft durch die Monomerflüssigkeit von oben nach unten verdrängt werden soll, weil die gegenüber der Monomerflüssigkeit spezifisch leichtere Luft auf Grund der Schwerkraft das Bestreben hat, im Zementpulver nach oben zu wandern und nicht nach unten in Richtung Vakuumanschluss zu migrieren.

Aus dem Kleb- und Dichtstoffbereich sind auch elektrisch angetriebene Auspressvorrichtungen bekannt. Diese Vorrichtungen können sowohl mit Akkumulatoren und mit Batterien als auch mit Hilfe einer stationären Stromversorgung angetrieben werden. Diese Vorrichtungen können mit ihren zum Teil sehr großen Auspresskräften besonders zähe pastenförmige Massen auspressen. Nachteilig ist jedoch bei der Verwendung von Elektromotoren, dass diese Buntmetalle enthalten und kostenintensiv in der Beschaffung sind. Im steril zu haltenden OP-Bereich müssen derartige Vorrichtungen aufwendig sterilisiert oder sogar ausgetauscht werden. Bei einer elektrischen Verkabelung kann die Bewegung des Anwenders im OP behindert werden.

Weiterhin wurden auch pneumatische Vorrichtungen vorgeschlagen. Diese Geräte erfordern einen stationären oder mobilen Druckluftanschluss (US 2 446 501 A, DE 20 2005 010 206 U1). Dazu sind Druckluftschläuche notwendig, welche die Bewegung des Anwenders behindern können.

Alternativ dazu ist auch die Verwendung von Druckgaspatronen zur Bereitstellung von Druckgas zum Auspressen eines fertigen Klebstoffs oder Dichtungsmittels möglich. Dazu wurden Vorrichtungen vorgeschlagen, bei denen der Druckgaszufluss durch ein Ventil und zusätzlich durch ein zweites Ventil der Strom der viskosen Masse gesteuert werden (US 2004/0074927 A1, US 6 935 541 B1). Bei diesen Vorrichtungen sind die Gaspatronen in den Vorrichtungen integriert. Bei derartigen an Druckluft angeschlossenen oder Druckgaspatronen enthaltenden Systemen ist immer eine Druckgasquelle erforderlich, ohne die die Systeme nicht mehr anwendbar sind.

Die EP 1 886 648 A1 offenbart eine Vorrichtung und ein Verfahren zur Herstellung eines Knochenzementteigs und beschreibt dabei eine Monomerzufuhr durch ein Druckgas.

Für die Behandlung von Impressionsfrakturen von Wirbelkörpern wurden eine Reihe von speziellen Polymethylmethacrylat-Knochenzementen entwickelt. Diese zeichnen sich dadurch aus, dass sie einen relativ hohen Anteil an Röntgenopakern, zum Beispiel Zirkoniumdioxid oder Bariumsulfat, enthalten. Dadurch soll eine fortlaufende Kontrolle der Ausbreitung des Knochenzementteigs im frakturierten Wirbel durch Fluoroskopie erleichtert werden. Die gegenwärtig hauptsächlich angewandten Verfahren zur Augmentation von frakturierten Wirbelkörpern sind die Vertebroplastie und die Kyphoplastie. Bisher ist dabei die manuelle Vermischung der Zementkomponenten in Mischbechern oder in einfachen Mischsystemen üblich. Der gebildete Zementteig wird dann in Spritzen gefüllt und im Rahmen der Vertebroplastie zur Augmentation von frakturierten Wirbelkörpern eingesetzt. Alternativ dazu kann der Zementteig auch in Kyphoplastie-Systeme eingebracht werden. Bei diesen Systemen wird eine Kavität im frakturierten Wirbelkörper aufgefüllt, wobei der Wirbelkörper zuvor durch einen Ballon aufgerichtet wurde. Bei einer Vielzahl von Kyphoplastie-Systemen erfolgt die Applikation des Zementteigs unter Verwendung von Hydraulik-Systemen. Das bedeutet, dass der Anwender (der Operateur) manuell ein hydraulisches System über ein Handstück bedient, das einen Kolben oder eine Membran hydraulisch bewegt, der den Zementteig durch einen Trokar in den Wirbelkörper einpresst. Das Handstück ist durch einen Schlauch ca. 60 cm bis 80 cm vom Kolben oder von der Membran entfernt. Der Vorteil dieser Systeme besteht darin, dass sich die Hände des Operateurs durch das Hydrauliksystem außerhalb der Röntgenstrahlen befinden.

Die Aufgabe der vorliegenden Erfindung besteht darin, die Nachteile des Stands der Technik zu überwinden. Insbesondere besteht die Aufgabe der Erfindung in der Entwicklung einer Vorrichtung und eines Verfahrens zur Vermischung der Ausgangskomponenten von Polymethylmethacrylat-Knochenzement, bei der der Knochenzementteig schnell und mit möglichst geringem Aufwand aus einem Zementpulver und einer Monomerflüssigkeit gemischt wird. Die Vorrichtung soll dabei ein Herstellungsverfahren ermöglichen, beziehungsweise das Verfahren soll dabei derart gestaltet sein, dass es möglichst weitgehend automatisiert von alleine abläuft. Elektrisch betriebene Bauteile sollen dabei möglichst nicht zur Anwendung kommen. Die Handhabung der Vorrichtung soll sehr einfach im Vergleich zu gegenwärtig auf dem Markt befindlichen Zementiersystemen sein.

Die Aufgabe der Erfindung besteht insbesondere in der Entwicklung einer Vorrichtung zum Vermischen von Zementpulver und Monomerflüssigkeit, und bevorzugt auch zum vorherigen Lagern dieser Ausgangskomponenten, wobei der durch Vermischung der Zementkomponenten gebildete PMMA-Knochenzementteig bevorzugt zur Augmentation von frakturierten Wirbelkörpern bestimmt ist. Die Handhabung der Vorrichtung soll maximal vereinfacht sein, um Anwendungsfehler infolge von fehlerhaft durchgeführten Montageschritten grundsätzlich zu vermeiden. Die Vorrichtung soll bevorzugt eine sichere Lagerung von Zementpulver und Monomerflüssigkeit in voneinander getrennten Kompartimenten ermöglichen, so dass während der Lagerung der Vorrichtung eine unbeabsichtigte Vermischung der Zementkomponenten ausgeschlossen ist. Die Vorrichtung soll eine Sterilisation mit dem Gas Ethylenoxid ermöglichen. Das in der Vorrichtung gelagerte Zementpulver muss für Ethylenoxid zugänglich sein. Die Vermischung der Monomerflüssigkeit soll ohne einen von außen manuell zu bewegenden Mischer erfolgen. Die Vorrichtung soll nach einer erfolgten manuellen Aktivierung vorzugsweise auch das Öffnen des Monomerflüssigkeitsbehälters ermöglichen, sowie den nachfolgenden Monomertransfer in das Zementpulver und die Vermischung der Zementkomponenten unter Bildung des Knochenzementteigs selbsttätig unter Verwendung einer internen Energiequelle durchführen können. Weiterhin ist es wichtig, dass an der Vorrichtung ein geeigneter Konnektor oder ein Konnektor mit einem Schlauch angebracht werden kann, durch die der gebildete Knochenzementteig in eine Spritze für die Vertebroplastie oder in eine Kartusche eines Kyphoplastie-Systems appliziert werden kann.

Die Erfindung soll auch ein Verfahren zur Herstellung eines Knochenzementteigs, insbesondere eines pastenförmigen Polymethylmethacrylat-Knochenzementteigs, bereitstellen, wobei der Knochenzementteig aus einem Zementpulver und einer Monomerflüssigkeit hergestellt wird, mit dem die Nachteile der bisherigen Vorrichtungen und Verfahren überwunden werden. Die Erfindung hat insofern auch die Aufgabe, die Bildung von Monomerblasen im erzeugten Knochenzementteig zu verhindern. Mit der erfindungsgemäßen Vorrichtung und dem erfindungsgemäßen Verfahren soll ferner erreicht werden, dass auch bei einem sehr einfachen und kostengünstigen Aufbau der Vorrichtung und bei gleichzeitig sehr einfacher und unkomplizierter Anwendbarkeit der Vorrichtung von Anfang bis Ende des Auspressvorgangs ein homogener Knochenzementteig erzeugt und abgefüllt werden kann.

Der Aufbau der Vorrichtung soll kostengünstig sein, damit die Vorrichtung aus hygienischen Gründen nur einmalig verwendet werden kann. Gleichzeitig soll die Vorrichtung nach der Anwendung problemlos und gefahrlos entsorgt werden können. Es sollen möglichst viele oder alle in der Vorrichtung ablaufenden Prozesse, wie die Vermischung der Ausgangskomponenten, das Austragen des Knochenzementteigs und gegebenenfalls auch das Öffnen des Monomerflüssigkeitsbehälters und gegebenenfalls auch das Öffnen der Kartusche mit möglichst wenigen Arbeitsschritten und so weit als möglich automatisiert ablaufen und vorzugsweise mit einem einzigen Antrieb anzutreiben sein.

Die Aufgaben der Erfindung werden gelöst durch eine Vorrichtung zum Herstellen eines Knochenzementteigs aus einer Monomerflüssigkeit und einem Zementpulver als Ausgangskomponenten des Knochenzementteigs, die Vorrichtung aufweisend eine Kartusche mit einem zylindrischen Innenraum zum Mischen der Ausgangskomponenten, wobei der Innenraum der Kartusche an der Vorderseite bis auf eine Austragsöffnung zum Austreiben des Knochenzementteigs aus dem Innenraum geschlossen ist, einen Austragskolben, der im Innenraum der Kartusche angeordnet ist und der in Richtung der Austragsöffnung linear bewegbar gelagert ist, das Zementpulver, das im Innenraum der Kartusche zwischen der Austragsöffnung und dem Austragskolben angeordnet ist, eine Monomeraufnahme mit einem Innenraum, in dem ein Monomerflüssigkeitsbehälter enthaltend die Monomerflüssigkeit enthalten ist, wobei in der Monomeraufnahme ein in Längsrichtung der Monomeraufnahme beweglicher Förderkolben angeordnet ist, einen Druckgasanschluss, der direkt oder über eine Druckgasleitung druckdicht mit dem Innenraum der Monomeraufnahme verbunden ist, wobei der Förderkolben zwischen dem Monomerflüssigkeitsbehälter und dem Druckgasanschluss oder der Druckgasleitung in der Monomeraufnahme angeordnet ist, und eine Verbindung, die den Innenraum der Monomeraufnahme und den Innenraum der Kartusche für die Monomerflüssigkeit durchlässig aber für das Zementpulver undurchlässig miteinander verbindet, wobei der Monomerflüssigkeitsbehälter zwischen dem Förderkolben und der Verbindung angeordnet ist.

Die Vorrichtung ist erfindungsgemäß bevorzugt auch zum Lagern des Zementpulvers und der Monomerflüssigkeit geeignet. Hierzu kann besonders bevorzugt vorgesehen sein, dass der Monomerflüssigkeitsbehälter eine Glasampulle, eine Plastikampulle, ein Kunststofffolienbeutel oder ein Aluminium-Kunststoff-Verbund-Beutel ist. In derartigen Monomerflüssigkeitsbehältern kann die Monomerflüssigkeit besonders lange gelagert werden. Bevorzugt ist der Monomerflüssigkeitsbehälter eine Glasampulle oder eine Kunststoffampulle, da diese gut und zuverlässig durch die Bewegung des Förderkolbens aufgebrochen werden können und unanfällig für mögliche Beeinträchtigungen sind.

Es kann ferner vorgesehen sein, dass der Innenraum der Monomeraufnahme und der Innenraum der Kartusche über die Verbindung für Gase durchlässig verbunden sind.

Die Richtungsbezeichnungen in Rahmen der vorliegenden Erfindung werden auf die Flussrichtung des Druckgases, der Monomerflüssigkeit und des Knochenzementteigs beziehungsweise auf die Austragsöffnung der Vorrichtung bezogen, wobei die Austragsöffnung vorne an der Vorrichtung angeordnet beziehungsweise vorne so definiert ist. Der Austragskolben wird also von hinten angetrieben und nach vorne in Richtung der Austragsöffnung bewegt und dabei der Knochenzementteig in Richtung der Vorderseite durch die Austragsöffnung herausgedrückt, beziehungsweise gepresst.

Der Innenraum der Kartusche hat eine zylindrische Geometrie. Die zylindrische Form ist die einfachste, mit der sich der Innenraum der Kartusche realisieren lässt. Unter einer zylindrischen Form ist geometrisch die Form eines allgemeinen Zylinders mit einer beliebigen Grundfläche zu verstehen, also nicht nur ein Zylinder mit einer kreisförmigen Grundfläche. Die Innenwand des Innenraums der Kartusche kann also durch den Zylindermantel eines Zylinders mit beliebiger Grundfläche realisiert sein, insbesondere mit unterschiedlicher Grundfläche realisiert sein, das heißt auch mit nicht kreisförmigen oder nicht runden Grundflächen. Erfindungsgemäß wird jedoch eine zylindrische Geometrie mit rotationssymmetrischer und insbesondere kreisrunder Grundfläche für den Innenraum bevorzugt, da diese am einfachsten zu fertigen ist.

Die Kartusche, die Monomeraufnahme, der Austragskolben, der Förderkolben und die Verbindung sind vorzugsweise aus einem thermoplastischen Kunststoff gefertigt, insbesondere mit einem Spritzgussverfahren.

Es kann erfindungsgemäß vorgesehen sein, dass der Förderkolben mit einem über den Druckgasanschluss in den Innenraum der Monomeraufnahme geleiteten Gasdruck in Richtung der Verbindung drückbar ist und der Monomerflüssigkeitsbehälter durch die Bewegung des Förderkolbens zu öffnen ist, insbesondere aufbrechbar ist, und die Monomerflüssigkeit aus dem Innenraum der Monomeraufnahme durch die Verbindung in den Innenraum der Kartusche drückbar ist.

Hierdurch kann der Gasdruck als Antrieb beziehungsweise als Energiequelle zum Öffnen des Monomerflüssigkeitsbehälters und zum Auspressen der Monomerflüssigkeit in das Zementpulver verwendet werden.

Ferner kann vorgesehen sein, dass der Förderkolben für Gase undurchlässig ist und gegen die Innenwände der Monomeraufnahme gasdicht abgedichtet ist, vorzugsweise mit wenigstens einer umlaufenden Dichtung.

Hiermit wird sichergestellt, dass der Gasdruck gut zum Antreiben des Förderkolbens verwendet werden kann und dass kein Druckgas in die Monomerflüssigkeit gepresst wird.

Besonders bevorzugt kann vorgesehen sein, dass die Verbindung wenigstens einen Durchgang im Austragskolben aufweist, wobei bevorzugt der wenigstens eine Durchgang für die Monomerflüssigkeit und für Gase durchlässig ist und für das Zementpulver undurchlässig ist, wobei besonders bevorzugt die zum Zementpulver ausgerichtete Oberfläche des Austragskolbens für das Zementpulver undurchlässig ist.

Hierzu kann erfindungsgemäß bevorzugt eine Porenscheibe aus Kunststoff verwendet werden.

Hierdurch kann der wenigstens eine Durchgang zum Einleiten der Monomerflüssigkeit verwendet werden. Gleichzeitig kann das Zementpulver nicht in den oder durch den Durchgang vordringen, dort mit der Monomerflüssigkeit reagieren und dadurch den Durchgang oder die Verbindung ungewollt verstopfen, wenn das Zementpulver in der Verbindung mit der Monomerflüssigkeit reagiert und quillt.

Damit ist es möglich, dass der Monomerflüssigkeitsbehälter enthaltend die Monomerflüssigkeit zwischen dem Förderkolben und dem Austragskolben angeordnet ist.

Hierdurch ist es möglich, dass der Monomerflüssigkeitsbehälter zwischen dem Förderkolben und dem Austragskolben zusammengepresst und dadurch geöffnet und ausgepresst wird. Der Austragskolben ist dabei zunächst durch das Zementpulver in der Kartusche gehalten, das im nicht benetzten Zustand, also wenn es nicht mit der Monomerflüssigkeit benetzt ist, nicht fließfähig ist und deswegen den Austragskolben zunächst in Position hält.

Ferner kann vorgesehen sein, dass der Druckgasanschluss ein Dichtmittel zum druckdichten Anschließen einer Druckgasquelle aufweist, insbesondere zum druckdichten Anschließen einer Druckgaspatrone aufweist.

Hiermit kann sichergestellt werden, dass das Druckgas aus der Druckgasquelle vollständig für den Antrieb des Förderkolbens zur Verfügung steht und nicht entweicht.

Bevorzugt kann auch vorgesehen sein, dass die Vorrichtung eine Druckgaspatrone aufweist, die an dem Druckgasanschluss druckdicht angeschlossen ist oder anschließbar ist, wobei vorzugsweise die Druckgaspatrone eine CO₂-Patrone ist.

Hiermit wird erreicht, dass die Vorrichtung ohne weitere Bauteile und ohne ein externes Druckgasnetz unmittelbar verwendbar ist.

Mit der Erfindung wird auch vorgeschlagen, dass der Druckgasanschluss einen Hohldorn zum Durchstechen einer Membran einer Druckgaspatrone aufweist. Die Membran dient als Verschluss für die Druckgaspatrone.

Hiermit kann die Druckpatrone leicht innerhalb der Vorrichtung geöffnet werden.

Es kann erfindungsgemäß auch vorgesehen sein, dass der Druckgasanschluss ein Innengewinde aufweist, in das eine Druckpatrone oder eine andere Druckgasquelle mit einem Außengewinde schraubbar ist.

Besonders bevorzugt kann vorgesehen sein, dass der Druckgasanschluss ein Ventil oder eine Öffnungseinrichtung umfasst, wobei die Öffnungseinrichtung zum Öffnen einer verschlossenen Druckgaspatrone und zum Herstellen einer druckdichten Verbindung zwischen dem Druckgasanschluss und der Druckgaspatrone geeignet ist, wobei vorzugsweise die Druckgaspatrone und die Öffnungseinrichtung gegeneinander verschiebbar in der Vorrichtung gelagert sind und sich durch Zusammenschieben der Druckgaspatrone und der Öffnungseinrichtung die Druckgaspatrone in der Vorrichtung zu öffnen ist, so dass Druckgas aus der Druckgaspatrone in den Innenraum der Monomeraufnahme strömt.

Dadurch kann die Vorrichtung bequem durch das Bedienen des Ventils oder der Öffnungseinrichtung aktiviert werden, so dass sie anschließend den Knochenzementteig mischt.

Ferner kann vorgesehen sein, dass die Vorrichtung ferner einen Behälter für eine Druckgaspatrone oder eine andere Druckgasquelle aufweist, wobei eine in den Behälter eingesetzte Druckgaspatrone beziehungsweise Druckgasquelle in der Vorrichtung durch eine Bewegung der Druckgaspatrone beziehungsweise der Druckgasquelle gegen den Druckgasanschluss derart zu öffnen ist, dass das Druckgas aus der Druckgaspatrone beziehungsweise der Druckgasquelle in den Druckgasanschluss strömt, wobei vorzugsweise die Druckgaspatrone beziehungsweise die Druckgasquelle durch eine Schraubbewegung gegen den Druckgasanschluss zu bewegen ist.

Vorzugsweise ist die Schraubbewegung durch manuelles Bedienen eines Betätigungselements erzeugbar, insbesondere durch manuelles Drehen eines Flügelschraubenkopfs erzeugbar.

Durch diese Maßnahmen kann die Vorrichtung bequem durch das Bewegen des Behälters für die Druckgaspatrone beziehungsweise die Druckgasquelle aktiviert werden, so dass sie anschließend den Knochenzementteig mischt. Die Vorrichtung ist mit der eingesetzten Druckgaspatrone beziehungsweise Druckgasquelle vollständig einsatzbereit.

Es kann erfindungsgemäß vorgesehen sein, dass im Druckgasanschluss oder in der Druckgasleitung ein Ablassventil zum Ablassen eines Überdrucks an die Umgebung angeordnet ist, insbesondere ein geschlossenes von außen manuell bedienbares Ablassventil oder ein geschlossenes mechanisch oder elektrisch öffenbares Ablassventil.

Dadurch kann die Vorrichtung nach dem Herstellen des Knochenzementteigs druckfrei gemacht werden und damit nach Gebrauch gefahrlos entsorgt werden.

Es kann auch vorgesehen sein, dass die Verbindung eine Fluidleitung aufweist, wobei die Fluidleitung den Innenraum der Monomeraufnahme mit dem Innenraum der Kartusche verbindet.

Hierdurch können die Kartusche und die Monomeraufnahme parallel zueinander oder nebeneinander angeordnet werden und so die Vorrichtung kompakt gestaltet werden.

Es kann erfindungsgemäß ferner vorgesehen sein, dass zwischen der Verbindung und dem Monomerflüssigkeitsbehälter ein elastisch verformbarer Abstandhalter angeordnet ist, wobei bevorzugt der Abstandhalter den Monomerflüssigkeitsbehälter zumindest 3 mm von der Verbindung beabstandet, besonders bevorzugt zumindest 6 mm von der Verbindung beabstandet, ganz besonders bevorzugt zumindest 10 mm von der Verbindung beabstandet Mit dem Abstandhalter kann der Monomerflüssigkeitsbehälter stoßsicher in der Monomeraufnahme gelagert werden.

Ferner kann vorgesehen, dass die Verbindung über eine Einmündung in einer Seitenwand der Kartusche in den Innenraum der Kartusche mündet, wobei die Einmündung von einer Seitenfläche des Austragskolbens, die parallel zur Bewegungsrichtung des Austragskolbens liegt, abgedeckt ist, wobei der Austragskolben eine für die Monomerflüssigkeit durchlässige Durchführung in den Innenraum der Kartusche zum Zementpulver aufweist, die sich von einer Seitenfläche des Austragskolbens bis zu einer vorderen Grundfläche des Austragskolbens erstreckt, wobei die Grundfläche des Austragskolbens senkrecht zur Bewegungsrichtung des Austragskolbens liegt, wobei bevorzugt die Durchführung für das Zementpulver undurchlässig ist und besonders bevorzugt für Gase durchlässig ist.

Hierdurch können die Kartusche und die Monomeraufnahme parallel zueinander oder nebeneinander angeordnet werden und so die Vorrichtung kompakt gestaltet werden. Zudem wird so die Öffnung in den Innenraum der Kartusche durch Vortreiben des Austragskolbens geschlossen, so dass keine Monomerflüssigkeit mehr in den Innenraum der Kartusche nachfließen kann und so eine gleichbleibende Konsistenz des Knochenzementteigs besser gewährleistet werden kann.

Es kann bei einer solchen Ausführung erfindungsgemäß vorgesehen sein, dass der Austragskolben über eine Antriebsstange oder eine Gewindestange in der Kartusche in Richtung der Austragsöffnung vortreibbar ist.

Hiermit kann der Knochenzementteig aus der Kartusche bequem in einen Applikator, wie eine Spritze, eingefüllt werden oder direkt appliziert werden.

Es kann auch vorgesehen sein, dass an der Austragsöffnung ein Rohr oder ein Schlauch angeschlossen ist, wobei vorzugsweise an der Spitze des Applikationsrohrs oder des Schlauchs ein Luer-Lock-Adapter vorgesehen ist.

Hierdurch kann die Vorrichtung auch zum Applizieren des Knochenzementteigs durch ein Schlauchsystem oder einen Trokar verwendet werden.

Des Weiteren kann vorgesehen sein, dass in dem Zementpulver ein die Monomerflüssigkeit leitendes Additiv verteilt ist, wobei vorzugsweise das Zementpulver mit dem Additiv beschichtet oder mit dem Additiv gemischt ist.

Als Additiv kann beispielsein biokompatibler Zellstoff verwendet werden, der eine ausreichende Saugfähigkeit für die Monomerflüssigkeit aufweist. Das Additiv kann partikulär in dem Zementpulver verteilt sein.

Hiermit kann erreicht werden, dass die Monomerflüssigkeit sich schnell in dem Zementpulver verteilt und so eine vollständige Mischung entsteht, bevor das anquellende Zementpulver eine weitere Ausbreitung der Monomerflüssigkeit verhindern würde. Dadurch ist es möglich, die Monomerflüssigkeit auch über längere Strecken durch das Zementpulver zu leiten und so einen homogenen Knochenzementteig zu erzeugen.

Ferner kann vorgesehen sein, dass in der Kartusche eine Monomerflüssigkeit als erste Ausgangskomponente und ein Pulver als zweite Ausgangskomponente enthalten ist, aus denen der Knochenzementteig innerhalb der Kartusche gemischt wird, wobei in dem Pulver ein hydrophiles Additiv verteilt ist, mit dem die Monomerflüssigkeit in dem gesamten Pulver verteilbar ist, bevorzugt ohne dass zuvor eine Polymerisation des Knochenzements die weitere Verteilung der Monomerflüssigkeit in dem Pulver verhindert.

Hiermit wird erreicht, dass die Monomerflüssigkeit in dem Pulver schnell verteilt wird, bevor eine Polymerisation des in dem Pulver enthaltenen Zementpulvers mit der Monomerflüssigkeit stattfindet und dadurch eine weitere Verteilung der Monomerflüssigkeit unterbunden wird. Nur hierdurch ist der erfindungsgemäße Aufbau in einer einzelnen Kartusche überhaupt möglich, dass nämlich die Monomerflüssigkeit von einer Seite in das Pulver gepresst wird und sich dennoch in dem gesamten Pulver verteilen kann, bevor die Polymerisierung eine weitere Verteilung der Monomerflüssigkeit in dem Pulver unterbindet.

Das Additiv ist vorzugsweise Partikulär oder Faserförmig. Bevorzugt weist das Additiv eine chemische Substanz mit zumindest einer OH-Gruppe auf. Das Additiv hat bevorzugt ein Aufsaugvermögen von mindestens 0,6 g Methylmethacrylat pro Gramm Additiv.

Es kann erfindungsgemäß vorgesehen sein, dass das Pulver mindestens ein partikuläres Polymethylmethacrylat oder Polymethylmethacrylat-Copolymer der Siebfraktion kleiner 100 µm, einen Initiator, und mindestens ein in Methylmethacrylat unlösliches, partikuläres oder faserförmiges Additiv aufweist, wobei das Additiv ein Aufsaugvermögen größer gleich 0,6 g Methylmethacrylat pro Gramm Additiv bei Raumtemperatur besitzt.

Ein solches Pulver ist besonders gut zur Verteilung der Monomerflüssigkeit in dem Pulver geeignet, so dass ein Aufbau des Knochenzementapplikators ermöglicht wird, mit dem ein einseitiges Einpressen der Monomerflüssigkeit auch auf einer Schmalseite des Innenraums der Kartusche möglich ist. Dabei wurde überraschend gefunden, dass es möglich ist, durch einfaches In-Kontakt-Bringen eines solchen und insbesondere eines im folgenden definierten Pulvers mit einer Monomerflüssigkeit, insbesondere mit einer nachstehend definierten Monomerflüssigkeit, einen klebfreien, plastisch verformbaren Knochenzementteig herzustellen, der selbstständig durch radikalische Polymerisation aushärtet, ohne dass es notwendig ist, den Zementteig manuell oder mit Hilfe von technischen Hilfsmitteln zu vermischen. Es wurde beobachtet, dass durch Zusatz eines in Methylmethacrylat unlöslichen, partikulären oder faserförmigen Additivs, das ein Aufsaugvermögen von größer 0,6 g Methylmethacrylat pro Gramm Additiv bei Raumtemperatur besitzt, zu einem Zementpulver eines niedrig-viskosen Knochenzements, ein modifiziertes Pulver als Zementpulver erhalten wird, in das Monomerflüssigkeit über eine Strecke von mindestens 5 cm eingepresst werden kann. Das Additiv verbessert überraschend auch die Benetzung des Zementpulvers mit Monomerflüssigkeit. Das Additiv hat dabei einen "Docht-Effekt" und leitet schon in sehr geringen Mengen ab 0,1 Gew.-% die Monomerflüssigkeit in das Innere des Pulvers. Weiterhin verzögert das Additiv das Verkleben der Polymerpartikel im Pulver, wodurch die Ausbildung einer blockierenden Gelschicht verzögert wird und das Eindringen der Monomerflüssigkeit in das Pulver begünstigt wird. Die Monomerflüssigkeit kann dabei in das Pulver eingepresst oder auch eingesaugt werden.

Dabei kann bevorzugt vorgesehen sein, dass das Additiv kovalent gebundene Hydroxylgruppen an seiner Oberfläche besitzt. Das Additiv kann erfindungsgemäß bevorzugt ausgewählt sein aus der Gruppe, die aus mikrokristalliner Cellulose, Oxycellulose, Stärke, Titandioxid und Siliziumdioxid besteht, wobei pyrogenes Siliziumdioxid besonders bevorzugt wird. Das Additiv kann eine Partikelgröße der Siebfraktion kleiner 100 µm, bevorzugt der Siebfraktion kleiner 50 µm und ganz besonders bevorzugt von der Siebfraktion kleiner 10 µm aufweisen. Ferner kann bevorzugt vorgesehen sein, dass das Additiv im Pulver in einer Menge von 0,1 bis 2,5 Gew.-% bezogen auf das Gesamtgewicht des Pulvers enthalten ist. Des Weiteren kann vorgesehen sein, dass das Polymerpulver Dibenzoylperoxid als Initiator enthält.

Es kann vorgesehen sein, dass die Monomerflüssigkeit mindestens ein Methylmethacrylat und einen Aktivator enthält. Des Weiteren kann vorgesehen sein, dass die Monomerflüssigkeit mindestens einen Aktivator aus der Gruppe der aromatischen Amine enthält. Ferner kann vorgesehen sein, dass die Monomerflüssigkeit mindestens einen radikalischen Stabilisator aus der Gruppe der Chinone oder der sterisch gehinderten Phenole enthält.

Vorteilhaft ist es, wenn das Additiv kovalent gebundene Hydroxylgruppen an seiner Oberfläche besitzt. Besonders vorteilhaft sind dabei besonders Si-OH-Gruppen und alkoholische OH-Gruppen. Durch die oberflächlich angeordneten OH-Gruppen hat das Additiv eine hohe Oberflächenenergie, wodurch eine gute Benetzbarkeit des Additivs mit Methylmethacrylat erreicht wird. Die pyrogenen Kieselsäuren Aerosil® 380 und Aerosil® 300 sind besonders geeignet. Daneben ist es auch möglich, durch Sol-Gel-Prozesse erzeugtes Siliziumdioxid als Additiv zu verwenden.

Hierzu kann auch vorgesehen sein, dass der Innenraum der Kartusche und der Innenraum der Monomeraufnahme einen gemeinsamen zylindrischen Innenraum bilden und miteinander fluchten, so dass der Austragskolben mit dem Förderkolben in dem Innenraum der Kartusche vortreibbar ist und der Förderkolben in den Innenraum der Kartusche drückbar ist.

Hierdurch kann der Austragskolben durch Vortreiben des Förderkolbens mit dem Druckgas angetrieben werden. Dadurch kann durch das sich entspannende Druckgas nicht nur der Knochenzementteig hergestellt werden, sondern auch aus der Kartusche zur weiteren Verwendung ausgetrieben werden.

Erfindungsgemäß kann bevorzugt vorgesehen sein, dass an der Innenwand der Kartusche im Bereich der Vorderseite ein Bypass oder eine Nut vorgesehen ist, durch den oder durch die das Druckgas an dem Förderkolben und dem Austragskolben vorbei strömen kann, wenn der Förderkolben eine Öffnung zum Bypass oder eine Nut zumindest bereichsweise überfahren hat und dadurch zum Druckgasanschluss freilegt.

Dadurch entweicht am Ende des Auspressvorgangs das Druckgas durch den Bypass oder die Nut aus der Vorrichtung und die Vorrichtung wird damit druckfrei und kann gefahrlos entsorgt werden.

Dabei kann vorgesehen sein, dass die Öffnung für den Bypass oder das Ende der Nut von dem vorderen Ende der Kartusche weiter beabstandet ist als die Summe der Höhe des Förderkolbens und des Austragskolbens, vorzugsweise zumindest mehr als 5 mm und maximal 20 mm weiter von dem vorderen Ende der Kartusche beabstandet ist als die Summe der Höhe des Förderkolbens und des Austragskolbens.

Hiermit wird sichergestellt, dass der Knochenzementteig vollständig aus der Kartusche ausgetrieben wird, bevor die Vorrichtung druckfrei wird.

Bevorzugt kann des Weiteren ebenfalls vorgesehen sein, dass an dem Druckgasanschluss oder in der Druckgasleitung ein Sterilfilter angeordnet ist, der ein in die Monomeraufnahme strömendes Druckgas steril filtert.

Hiermit wird eine mögliche Verunreinigung oder Kontaminierung durch das verwendete Druckgas vermieden.

Bevorzugt kann vorgesehen sein, dass an dem Druckgasanschluss oder in der Druckgasleitung ein geschlossenes Überdruckventil angeordnet ist, das bei Überschreiten eines Grenzdrucks den Druckgasanschluss oder die Druckgasleitung nach außen zur Umgebung öffnet.

Hiermit kann verhindert werden, dass die Vorrichtung, insbesondere die Monomeraufnahme explodiert.

Ferner kann vorgesehen sein, dass die Vorrichtung einen Verschluss aufweist, der die Austragsöffnung verschließt und der gegen die Austragsöffnung beweglich gelagert ist, wobei ein Leitungselement an der Vorderseite der Austragsöffnung angeordnet ist, wobei das Leitungselement eine Verschlussaufnahme zum Aufnehmen zumindest eines Teils des Verschlusses umfasst, und wobei der Verschluss durch einen Druck auf den Knochenzementteig derart in die Verschlussaufnahme hinein drückbar ist, dass die Austragsöffnung geöffnet ist, wobei das Leitungselement mit dem in die Verschlussaufnahme gedrückten Verschluss einen freien Leitungsquerschnitt bereitstellt, durch den der Knochenzementteig aus der Austragsöffnung hindurch und aus der Vorrichtung heraus drückbar ist.

Hiermit ist die Vorrichtung zunächst verschlossen und öffnet sich selbsttätig durch Vortreiben des Knochenzementteigs mit dem Austragskolben.

Es kann vorgesehen sein, dass der Verschluss für Gase durchlässig aber für das Zementpulver undurchlässig ist.

Hierdurch kann das Innere der Kartusche mit einem sterilisierenden Gas, wie Ethylenoxid sterilisiert werden.

Es kann bei Vorrichtungen mit Verschluss vorgesehen sein, dass der Knochenzementteig den Verschluss in der Verschlussaufnahme umfließt, wenn der Knochenzementteig durch das Leitungselement fließt, vorzugsweise der Knochenzementteig entlang wenigstens einer Seitenfläche oder Mantelfläche des Verschlusses an dem Verschluss vorbeifließt.

Dass der Knochenzementteig den Verschluss in der Verschlussaufnahme umfließt, bedeutet, dass der Knochenzementteig in Längsrichtung des Verschlusses an dem Verschluss vorbeifließt.

Hierdurch wird erreicht, dass der Aufbau sehr einfach gehalten werden kann, da keine zusätzlichen Kanäle bereitgestellt werden müssen, durch die der Knochenzementteig den Verschluss im Leitungsmittel umfließt. Zudem wird der Knochenzementteig in die Bewegungsrichtung des Verschlusses gedrückt, so dass die Kraft, die mit dem Knochenzementteig übertragen wird und die zur Bereitstellung des Flusses des Knochenzementteigs genutzt wird, nicht umgeleitet werden muss, wodurch die notwendige Kraft zum Öffnen der Vorrichtung und zum Austreiben des Knochenzementteigs gering gehalten werden kann.

Bei erfindungsgemäßen Vorrichtungen mit Verschluss kann auch vorgesehen sein, dass der freie Leitungsquerschnitt auf einer Seite zumindest bereichsweise durch den Verschluss begrenzt ist, vorzugsweise durch eine Seitenfläche oder eine Mantelfläche des Verschlusses begrenzt ist.

Auch hierdurch wird erreicht, dass der Knochenzementteig in die Bewegungsrichtung des Verschlusses gedrückt werden kann, auch um durch das Leitungselement zu fließen, so dass die Kraft, die mit dem Knochenzementteig übertragen wird und die zur Bereitstellung des Flusses des Knochenzementteigs genutzt wird, nicht umgeleitet werden muss, wodurch die notwendige Kraft zum Öffnen der Vorrichtung und zum Austreiben des Knochenzementteigs gering gehalten werden kann.

Des Weiteren kann vorgesehen sein, dass der Verschluss fest in der Verschlussaufnahme steckt, wenn er aus der Austragsöffnung in die Verschlussaufnahme hineingedrückt ist.

Hiermit wird verhindert, dass sich der Verschluss in der Verschlussaufnahme bewegt, wenn er im fließenden Knochenzementteig angeordnet ist. Dadurch wird eine Veränderung des Strömungswiderstands des Knochenzementteigs und eine zeitliche Veränderung des Volumenstroms des Knochenzementteigs verhindert.

Zur Vereinfachung des Aufbaus kann vorgesehen sein, dass der Verschluss zumindest bereichsweise zylindrisch ist, insbesondere vollständig zylindrisch ist, und die Verschlussaufnahme eine hohlzylinderförmige Hülse bildet, wobei vorzugsweise in der Mantelfläche der hohlzylinderförmigen Hülse zumindest ein Kanal vorgesehen, wobei der zumindest eine Kanal den freien Leitungsquerschnitt bereitstellt.

Diese Ausführung ist besonders leicht zu fertigen. Zudem kann der Verschluss in axialer Richtung seiner Zylindergeometrie bewegt werden, so dass die Bewegung besonders leicht geführt werden kann.

Dabei kann vorgesehen sein, dass der Innendurchmesser der hohlzylinderförmigen Hülse größer ist als der Außendurchmesser des Verschlusses, vorzugsweise wenigstens 1 mm größer ist als der Außendurchmesser des Verschlusses, besondere bevorzugt zwischen 1 mm und 10 mm größer ist als der Außendurchmesser des Verschlusses.

Die sich hierdurch ergebenden freien Leitungsquerschnitte sind derart angeordnet beziehungsweise derart groß, dass sie den Fluss des Knochenzementteigs nur wenig behindern.

Gemäß einer bevorzugten Weiterbildung der vorliegenden Erfindung kann vorgesehen sein, dass in der Verschlussaufnahme Abstandhalter zur Beabstandung des Verschlusses von der Innenwand der Verschlussaufnahme vorgesehen sind, wobei vorzugsweise die Abstandhalten Leisten sind, die besonders bevorzugt in der Bewegungsrichtung des Verschlusses ausgerichtet sind und/oder die in Flussrichtung des Knochenzementteigs ausgerichtet sind.

Hierdurch wird erreichet, dass der freie Leitungsquerschnitt durch Beabstandung des Verschlusses von der Innenwand der Verschlussaufnahme erreicht wird, wenn der Verschluss in die Verschlussaufnahme gedrückt ist.

Des Weiteren kann auch vorgesehen sein, dass der freie Leitungsquerschnitt zumindest halb groß ist wie der Querschnitt der Austragsöffnung, vorzugsweise zumindest so groß ist wie der Querschnitt der Austragsöffnung.

Hierdurch wird erreicht, dass der Strömungswiderstand für den Knochenzementteig nicht durch einen zu geringen freien Leitungsquerschnitt des Leitungselements beeinträchtigt wird und gleichzeitig der Aufbau der Vorrichtung kompakt ist.

Bevorzugt kann auch vorgesehen sein, dass in der Verschlussaufnahme an der von der Austragsöffnung abgewandten vorderen Stirnwand ein Anschlag zur Begrenzung der Bewegung des Verschlusses angeordnet ist, wobei der Anschlag den Verschluss im vollständig eingedrücktem Zustand von der Stirnwand an der Vorderseite der Verschlussaufnahme beabstandet, so dass zwischen der Vorderseite des Verschlusses und der vorderen Stirnwand der freie Leitungsquerschnitt verbleibt.

Damit wird erreicht, dass der Knochenzementteig hinter dem Leitungselement in die gleiche Richtung weitergeleitet werden beziehungsweise strömen kann, in die er beim Eindrücken des Verschlusses in die Verschlussaufnahme fließt.

Es kann auch vorgesehen sein, dass die Rückseite der Kartusche mit der Vorderseite der Monomeraufnahme verbunden ist, vorzugsweise derart verbunden ist, dass der Innenraum der Kartusche mit dem Innenraum der Monomeraufnahme fluchtet.

Dadurch kann der Förderkolben auch zum Antreiben des Austragskolbens verwendet werden und so mit dem vom Druckgas angetriebenen Förderkolben auch der Knochenzementteig aus der Kartusche ausgetrieben werden. Die Vorrichtung ist dabei ein Full-Prepacked-Zementiersystem. Durch die fluchtenden Innenräume der Kartusche und der Monomeraufnahme kann sichergestellt werden, dass zunächst der Förderkolben durch einen auf die Rückseite des Förderkolbens wirkenden Gasdruck bewegt werden kann und anschließend der Förderkolben zum Antreiben des Austragskolbens genutzt werden kann, indem der Förderkolben gemeinsam mit dem Austragskolben (und gegebenenfalls der Scherben einer Glas oder Kunststoffampulle als Monomerflüssigkeitsbehälter dazwischen) weiter in Richtung der Austragsöffnung gedrückt wird.

Bevorzugt kann vorgesehen sein, dass die Monomeraufnahme einen zylindrischen Innenraum aufweist. Die zylindrische Form ist auch hier die einfachste, mit der sich der Innenraum der Aufnahme realisieren lässt. Unter einer zylindrischen Form ist geometrisch die Form eines allgemeinen Zylinders mit einer beliebigen Grundfläche zu verstehen, also nicht nur ein Zylinder mit einer kreisförmigen Grundfläche.

Es kann vorgesehen sein, dass an der Vorderseite des Förderkolbens zumindest eine vortretende Spitze, Kante und/oder Schneide zum Brechen des Monomerflüssigkeitsbehälters angeordnet ist.

Durch die Anwendung einer definierten Kraft an einer vorherbestimmten und räumlich begrenzten Stelle kann der Druck an dieser Stelle bei gleicher Kraft erhöht werden und so ein definiertes Brechen des Monomerflüssigkeitsbehälters erreicht werden. Dadurch wird der Ablauf des Aufbrechens des Monomerflüssigkeitsbehälters reproduzierbarer.

Ferner kann vorgesehen sein, dass in dem Druckgasanschluss oder in der Monomeraufnahme eine Belüftungsöffnung vorgesehen ist, wobei die Belüftungsöffnung durch eine Bewegung des Druckgasanschlusses oder durch eine Bewegung eines Behälters für eine Druckgaspatrone verschließbar ist.

Hiermit ist der Innenraum der Monomeraufnahme für ein sterilisierendes Gas wie Ethylenoxid zugängig.

Des Weiteren kann vorgesehen sein, dass an der der Austragsöffnung zugewandten Vorderseite des Austragskolbens ein Hohlzylinder angeordnet ist, wobei der Hohlzylinder an seiner der Austragsöffnung zugewandten Vorderseite offen ist und der Hohlzylinder sich vorzugsweise von der Vorderseite des Austragskolbens zumindest 3 mm in den Innenraum der Kartusche erstreckt.

Mit dem Hohlzylinder an der Vorderseite des Austragskolbens gelingt es, die Monomerflüssigkeit beim Einpressen in das Zementpulver im Innenraum der Kartusche über eine größere Strecke durch das Zementpulver fließen zu lassen beziehungsweise zu leiten, bevor die Monomerflüssigkeit die Innenwand der Kartusche erreicht. Dadurch kann die Ausbildung von Monomerflüssigkeitsblasen beziehungsweise Einschlüssen der Monomerflüssigkeit in dem gebildeten Knochenzementteig vermieden beziehungsweise reduziert werden. So kann ein homogenerer Knochenzementteig erzeugt werden. Ferner wurde gefunden, dass es durch das Zurückhalten eines kleinen Rests des in der Kartusche entstandenen Knochenzementteigs als Mischung des Zementpulvers mit der Monomerflüssigkeit in dem Innenraum der Kartusche gelingt, dass am Ende des Auspressvorgangs kein Knochenzementteig mit einer veränderten Konsistenz ausgetragen wird, da der restliche Knochenzementteig in der Kartusche zurückgehalten wird und die Austragsöffnung verschlossen wird.

Dabei kann vorgesehen sein, dass der Hohlzylinder eine weitere Bewegung des Austragskolbens in Richtung der Vorderseite der Kartusche blockiert, wenn die Vorderseite des Hohlzylinders an der Vorderseite des Innenraums der Kartusche anliegt, so dass der Austragskolben von der Vorderseite des Innenraums der Kartusche beabstandet ist und ein Totvolumen in dem Innenraum der Kartusche verbleibt.

Der Hohlzylinder ist im Innenraum der Kartusche angeordnet. Bevorzugt ist die Vorderseite des Austragskolbens bis auf den Hohlzylinder eben.

Es kann bevorzugt vorgesehen sein, dass der Austragskolben gegen die Innenwand des Innenraums der Kartusche dicht ist oder abgedichtet ist, insbesondere mit zumindest einer umlaufenden Dichtung abgedichtet ist.

Ferner kann vorgesehen sein, dass der Hohlzylinder zumindest einen Schlitz aufweist, vorzugsweise zumindest einen parallel zur Zylinderachse des Hohlzylinders verlaufenden aufweist, besonders bevorzugt zumindest einen von der Vorderseite bis zum Austragskolben reichenden Schlitz aufweist.

Hierdurch kann die Passung des Hohlzylinders an die Innenwand der Kartusche leichter angepasst werden und die Gefahr einer Blockade der Bewegung des Austragskolbens mit dem Hohlzylinder wird reduziert. Der zumindest Schlitz kann alternativ zu einem Verlauf parallel zur Zylinderachse des Hohlzylinders auch Spiralförmig in der Wandung des Hohlzylinders verlaufen.

Es kann auch vorgesehen sein, dass in dem Austragskolben zumindest eine Verbindung von der Rückseite des Austragskolbens zur Vorderseite des Austragskolbens zum Einleiten der Monomerflüssigkeit in den Innenraum der Kartusche vorgesehen ist, wobei die zumindest eine Verbindung für die Monomerflüssigkeit und Gase durchlässig ist und für das Zementpulver undurchlässig ist.

Es kann vorgesehen sein, dass das Zementpulver an der Vorderseite des Austragskolbens anliegt, insbesondere vollflächig anliegt, wobei vorzugsweise das Zementpulver in den Innenraum der Kartusche eingepresst ist.

Hierdurch wird verhindert, dass in der Kartusche größere Gaseinschlüsse verbleiben, die beim Mischen der Monomerflüssigkeit mit dem Zementpulver zu Gaseinschlüssen im Knochenzementteig oder zur Ausbildung von Monomerflüssigkeitsblasen führen könnten. Dies kann bei einem dicht geschütteten oder vorzugsweise gepressten Zementpulver nicht passieren, da die Monomerflüssigkeit die Partikel des Zementpulvers gut benetzt und die Oberflächenspannung der Monomerflüssigkeit dann keine oder zumindest keine relevanten Gaseinschlüsse zwischen den Partikeln des Zementpulvers erlaubt.

Es kann auch vorgesehen sein, dass das Zementpulver den Innenraum der Kartusche zwischen der geschlossenen Vorderseite und dem Austragskolben vollständig ausfüllt, wobei vorzugsweise das Zementpulver in den Innenraum der Kartusche eingepresst ist.

Hierdurch wird erreicht, dass die Monomerflüssigkeit in die Zwischenräume zwischen den Pulverpartikeln des Zementpulvers vordringt und dort aufgrund von Kapillarkräften durch das Zementpulver hindurch geleitet wird und sich so gut verteilen kann. Dadurch wird also eine schnelle und gleichmäßige Verteilung der Monomerflüssigkeit in dem Zementpulver erreicht.

Die der vorliegenden Erfindung zugrundeliegenden Aufgaben werden auch gelöst durch ein Verfahren zur Herstellung eines Knochenzementteigs, insbesondere eines pastenförmigen Polymethylmethacrylat-Knochenzementteigs, wobei der Knochenzementteig aus einem Zementpulver und einer Monomerflüssigkeit hergestellt wird, wobei die Monomerflüssigkeit in einem Monomerflüssigkeitsbehälter enthalten ist, der in einer Monomeraufnahme angeordnet ist, und wobei das Zementpulver in einer Kartusche enthalten ist, gekennzeichnet durch die folgenden nacheinander ablaufenden Schritte:
a) Antreiben und Bewegen eines Förderkolbens in der Monomeraufnahme mit einem Druckgas, wobei mit der Bewegung des Förderkolbens die Monomerflüssigkeit aus dem Monomerflüssigkeitsbehälter und aus der Monomeraufnahme in den Innenraum einer Kartusche gedrückt wird, so dass sich die Monomerflüssigkeit mit dem Zementpulver in der Kartusche mischt und dort den Knochenzementteig bildet,
b) der gemischte Knochenzementteig wird mit einem Austragskolben aus einer Austragsöffnung an einer dem Austragskolben gegenüberliegende Seite der Kartusche herausgedrückt.

Dabei kann vorgesehen sein, dass der Knochenzementteig mit einer erfindungsgemäßen Vorrichtung hergestellt wird.

Weiterhin kann vorgesehen sein, dass die Monomerflüssigkeit in dem Zementpulver mit Hilfe eines die Monomerflüssigkeit leitenden Additivs verteilt wird, wobei Partikel des Zementpulvere mit dem Additiv beschichtet sind oder mit dem Additiv vermischt sind.

Hiermit kann erreicht werden, dass die Monomerflüssigkeit sich schnell in dem Zementpulver verteilt und so eine vollständige Mischung entsteht, bevor das anquellende Zementpulver eine weitere Ausbreitung der Monomerflüssigkeit verhindern würde. Dadurch ist es möglich die Monomerflüssigkeit auch über längere Strecken durch das Zementpulver zu leiten und so einen homogenen Knochenzementteig zu erzeugen.

Zu dem gleichen Zweck kann vorgesehen sein, dass das Zementpulver vollständig von der Monomerflüssigkeit benetzt wird, wobei hierzu vorzugsweise ein die Monomerflüssigkeit anziehendes Additiv in dem Zementpulver verteilt ist.

Es kann auch vorgesehen sein, dass der Monomerflüssigkeitsbehälter durch die Bewegung des vom Druckgas angetriebenen Förderkolbens geöffnet wird.

Hierdurch wird der bereits vorhandene Antrieb durch das Druckgas auch dazu genutzt, den Monomerflüssigkeitsbehälter zu öffnen. Dadurch wird eine weitere Automatisierung des Verfahrens erreicht.

Ferner kann vorgesehen sein, dass der gemischte Knochenzementteig aus der Kartusche in einen Applikator oder eine Spritze gefüllt wird.

Auf diese Weise kann der Knochenzementteig später bequem mit dem Applikator oder der Spritze angewendet werden.

Es kann ferner vorgesehen sein, dass in Schritt b) der Austragskolben von dem vom Druckgas angetriebenen Förderkolben in Richtung der Austragsöffnung gedrückt wird.

Hierdurch wird der durch das Druckgas bereitgestellte Antrieb auch zum Auspressen des Knochenzementteigs aus der Kartusche genutzt und so das Verfahren weiter automatisiert.

Es kann auch vorgesehen sein, dass die beim Eindrücken der Monomerflüssigkeit in die Kartusche Gas aus den Zwischenräumen von Pulverpartikeln des Zementpulvers verdrängt und durch die Austragsöffnung herausgedrückt wird, insbesondere durch einen für Gas durchlässigen aber für das Zementpulver undurchlässigen Verschluss in der Austragsöffnung.

Hiermit wird das Vordringen der Monomerflüssigkeit in dem Zementpulver erleichtert.

Es kann auch vorgesehen sein, dass der Förderkolben in den Innenraum der Kartusche gedrückt wird und am Ende seiner Bewegung eine Öffnung zu einem Bypass oder eine Nut in der seitlichen Innenwand der Kartusche freilegt, wobei das Druckgas durch den Bypass oder die Nut am Förderkolben und am Austragskolben vorbei nach außen strömt, insbesondere durch die Austragsöffnung entweicht.

Dadurch entweicht am Ende des Auspressvorgangs das Druckgas durch den Bypass oder die Nut aus der Vorrichtung und die Vorrichtung wird damit druckfrei und kann gefahrlos entsorgt werden.

Es kann erfindungsgemäß vorgesehen sein, dass ein Verschluss durch den von dem Knochenzementteig auf den Verschluss wirkenden Druck in eine Verschlussaufnahme gedrückt und dabei die Austragsöffnung geöffnet wird, wobei vorzugsweise beim Herausdrücken des Knochenzementteigs aus der Austragöffnung der Knochenzementteig durch einen durch das Öffnen des Verschlusses entstandenen freien Leitungsquerschnitt fließt und aus der Vorrichtung ausgetragen wird.

Hiermit kann das Zementpulver zunächst verschlossen gelagert werden und die Kartusche wird selbstständig von dem angetriebenen Knochenzementteig geöffnet.

Ferner kann vorgesehen sein, dass vor Schritt a) die Druckgaspatrone geöffnet wird und das Druckgas aus der Druckgaspatrone durch einen Druckgasanschluss in die Monomeraufnahme zum Förderkolben geleitet wird.

Damit kann das Verfahren unabhängig von externen Druckgasquellen angewendet werden.

Es kann vorgesehen sein, dass an der der Austragsöffnung zugewandten Vorderseite des Austragskolbens ein Hohlzylinder angeordnet ist, wobei die Monomerflüssigkeit den Hohlzylinder umfließt, bevor sie bis zur Innenwand der Kartusche gelangt und/oder der Austragskolben auf die Vorderseite der Kartusche trifft, wobei mit dem Hohlzylinder eine weitere Bewegung des Austragskolbens in Richtung der Austragsöffnung blockiert wird und eine Restmenge des Knochenzementteigs in dem von dem Hohlzylinder begrenzten Teil des Innenraums der Kartusche verbleibt.

Bei der Anwendung mit Hohlzylinder wird sichergestellt, dass am Ende des Auspressvorgangs ein schlechter gemischter Rest des Knochenzementteigs oder ein Teil des Knochenzementteigs, der eine veränderte Zusammensetzung aufweist, in der Kartusche zurückgehalten wird und nicht zu Applikation verwendet wird.

Es wird mit dem erfindungsgemäßen Verfahren auch vorgeschlagen, dass in Schritt a) die Monomerflüssigkeit durch zumindest eine für das Zementpulver undurchlässige aber für Gase und die Monomerflüssigkeit durchlässige Verbindung in die Kartusche gepresst wird, vorzugsweise durch eine Bewegung eines Förderkolbens, der mit dem Druckgas angetrieben wird, in die Kartusche gepresst wird.

Hiermit wird verhindert, dass sich die Monomerflüssigkeit frühzeitig mit dem Zementpulver mischt.

Der Erfindung liegt die überraschende Erkenntnis zugrunde, dass es mit einem durch ein Druckgas angetriebenen Förderkolben gelingt, eine Monomerflüssigkeit derart tief in ein Zementpulver in einer Kartusche zu pressen, dass sich die Monomerflüssigkeit gleichmäßig in dem Zementpulver verteilt und dabei ein homogener Knochenzementteig gebildet wird. Gleichzeitig kann die mit dem Druckgas angetriebene Bewegung des Förderkolbens auch zum Öffnen eines Monomerflüssigkeitsbehälters, in dem die Monomerflüssigkeit enthalten ist, verwendet werden. In einer bevorzugten Ausführung kann die mit dem Druckgas angetriebene Bewegung auch zusätzlich noch zum Austreiben des Knochenzementteigs aus der Kartusche verwendet werden, wobei hierzu ein Austragskolben von der Bewegung des Förderkolbens angetrieben wird.

Der besondere Vorteil der Erfindung besteht darin, dass dem Anwender innerhalb von wenigen Sekunden Knochenzementteig zur Verfügung steht, ohne dass der Anwender komplexe Schritte zum Öffnen des Monomerflüssigkeitsbehälters und zur Vermischung der Ausgangskomponenten sowie zum Auspressen des Zementteigs in Spritzen oder in Kyphoplastie-Systeme durchführen muss.

Die Vorrichtung kann als hygienisches Wegwerfprodukt verwendet werden, da sie sehr weitgehend aus Kunststoff gefertigt werden kann und weil alle Teile einschließlich der Innenräume und des Zementpulvers mit Hilfe von Ethylenoxid sterilisierbar sind.

Eine beispielhafte erfindungsgemäße Vorrichtung zum Lagern, Vermischen und Austragen von Polymethylmethacrylat-Knochenzementteig kann beispielsweise aufweisen:
a) eine hohlzylinderförmige Kartusche,
b) einen für Gase undurchlässigen, axial beweglichen Förderkolben, der in der Kartusche angeordnet ist,
c) einen für Pulverpartikel undurchlässigen, für Gase durchlässigen axial beweglichen Austragskolben, der in der Kartusche angeordnet ist,
d) einen Monomerflüssigkeitsbehälter mit Monomerflüssigkeit, der in einem ersten Hohlraum, der durch die hohlzylinderförmige Kartusche, den Förderkolben und den Austragskolben begrenzt ist, angeordnet ist,
e) einen für Gase durchlässigen aber für Pulverpartikel undurchlässigen ersten Kartuschenverschluss, der axial beweglich in der Kartusche angeordnet ist,
f) Zementpulver, das in einem zweiten Hohlraum, der durch den Innenraum der Kartusche, den Austragskolben und den ersten Kartuschenverschluss begrenzt wird, angeordnet ist,
g) einen zweiten Kartuschenverschluss, der die Kartusche unterhalb des Förderkolbens abschließt,
h) einen dritten Hohlraum, der durch die Kartusche, den Förderkolben und den zweiten Kartuschenverschluss begrenzt wird,
i) eine Gaspatrone mit einem manuell zu betätigendem Öffnungselement, wobei die Gaspatrone komprimiertes Gas enthält, und
j) eine gasdurchlässige Druckgasleitung, die die Gaspatrone mit dem dritten Hohlraum gasdurchlässig verbindet.

Ein erfindungsgemäßes Verfahren kann beispielsweise mit der beispielhaften Vorrichtung zur Vermischung des Zementpulvers mit der Monomerflüssigkeit unter Bildung von Knochenzementteig mit den folgenden aufeinanderfolgenden Schritten umgesetzt werden:
a) manuelle Betätigung des Öffnungselements der Gaspatrone,
b) Öffnung der Gaspatrone,
c) Ausströmen des Druckgases aus der Gaspatrone durch die Druckgasleitung in den dritten Hohlraum,
d) Schieben des Förderkolbens in Richtung einer Austragsöffnung in einem vorderen Kartuschenkopf,
e) Zerbersten des Monomerflüssigkeitsbehälters durch die Bewegung des Förderkolbens,
f) Auspressen der Monomerflüssigkeit aus dem ersten Hohlraum und durch den Austragskolben in das Zementpulver im zweiten Hohlraum,
g) Verdrängen der Luft aus den Zwischenräumen der Zementpulverpartikel,
h) Entweichen der verdrängten Luft durch den ersten Kartuschenverschluss,
i) Vollständiges Benetzen der Zementpulverpartikel unter gleichzeitiger Bildung des Knochenzementteigs,
j) Schieben des Förderkolbens auf den zerborstenen Monomerflüssigkeitsbehälter und den Austragskolben,
k) Pressen des Knochenzementteigs in Richtung des ersten Kartuschenverschlusses,
l) Herausrücken oder Öffnen des ersten Kartuschenverschlusses aus der Kartusche und
m) Ausfließen des Knochenzementteigs aus dem Kartuschenkopf.

Im Folgenden werden weitere Ausführungsbeispiele der Erfindung anhand von fünfzehn schematisch dargestellten Figuren erläutert, ohne jedoch dabei die Erfindung zu beschränken. Dabei zeigt:
Figur 1: fünf schematische Querschnittansichten einer ersten erfindungsgemäßen Vorrichtung zum Lagern und Mischen einer Monomerflüssigkeit und eines Zementpulvers übereinander zur Verdeutlichung des Ablaufs eines erfindungsgemäßen Verfahrens;
Figur 2: eine schematische perspektivische Außenansicht der ersten Vorrichtung nach Figur 1 mit einer Spritze zur Anwendung des Knochenzementteigs;
Figur 3: zwei schematische Querschnittansichten als Ausschnittvergrößerungen der ersten Vorrichtung nach den Figuren 1 und 2 übereinander beim Auspressen des Knochenzementteigs, wobei die Querschnittebenen senkrecht zueinander gewählt sind;
Figur 4: eine schematische perspektivische Außenansicht einer zweiten erfindungsgemäßen Vorrichtung mit einer Spritze zur Anwendung des Knochenzementteigs;
Figur 5: eine schematische Querschnittansicht der zweiten erfindungsgemäßen Vorrichtung nach Figur 4 mit geöffneten Belüftungsöffnungen;
Figur 6: eine schematische Querschnittansicht der zweiten erfindungsgemäßen Vorrichtung nach den Figuren 4 und 5 im Ausgangszustand und mit geschlossenen Belüftungsöffnungen;
Figur 7: eine schematische Querschnittansicht der zweiten erfindungsgemäßen Vorrichtung nach den Figuren 4 bis 6 mit geöffneter Gaspatrone und geöffneter Glasampulle;
Figur 8: eine schematische Querschnittansicht der zweiten erfindungsgemäßen Vorrichtung nach den Figuren 4 bis 7 mit vollständig komprimierter Glasampulle;
Figur 9: eine schematische Querschnittansicht der zweiten erfindungsgemäßen Vorrichtung nach den Figuren 4 bis 8 mit geöffnetem Verschluss beim Austragen des Knochenzementteigs;
Figur 10: zwei schematische Querschnittansichten als Ausschnittvergrößerungen der zweiten Vorrichtung nach den Figuren 4 bis 9 nebeneinander beim Auspressen des Knochenzementteigs, wobei die Querschnittebenen senkrecht zueinander gewählt sind;
Figur 11: eine schematische perspektivische Außenansicht einer dritten erfindungsgemäßen Vorrichtung;
Figur 12: eine schematische perspektivische Seitenansicht der dritten Vorrichtung nach Figur 11 mit geöffnetem Gehäuse;
Figur 13: eine schematische Querschnittansicht der dritten erfindungsgemäßen Vorrichtung nach den Figuren 11 und 12 im Ausgangszustand;
Figur 14: zwei schematische Querschnittansichten der dritten erfindungsgemäßen Vorrichtung nach den Figuren 11 bis 13 übereinander zur Verdeutlichung des Ablaufs eines erfindungsgemäßen Verfahrens; und
Figur 15: zwei weitere schematische Querschnittansichten der dritten erfindungsgemäßen Vorrichtung nach den Figuren 11 bis 14 übereinander zur Verdeutlichung des weiteren Ablaufs eines erfindungsgemäßen Verfahrens.

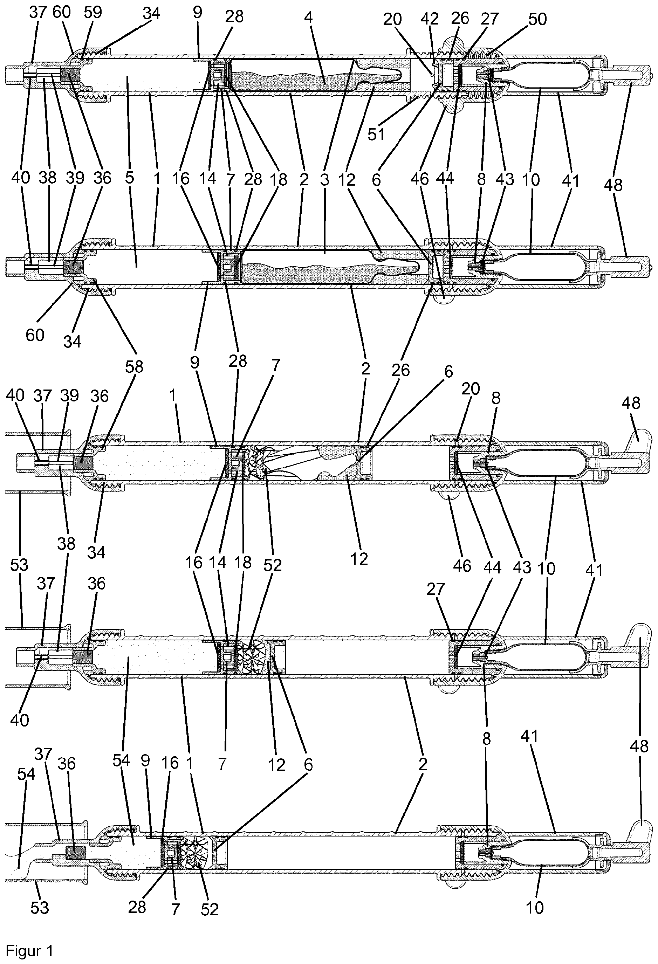

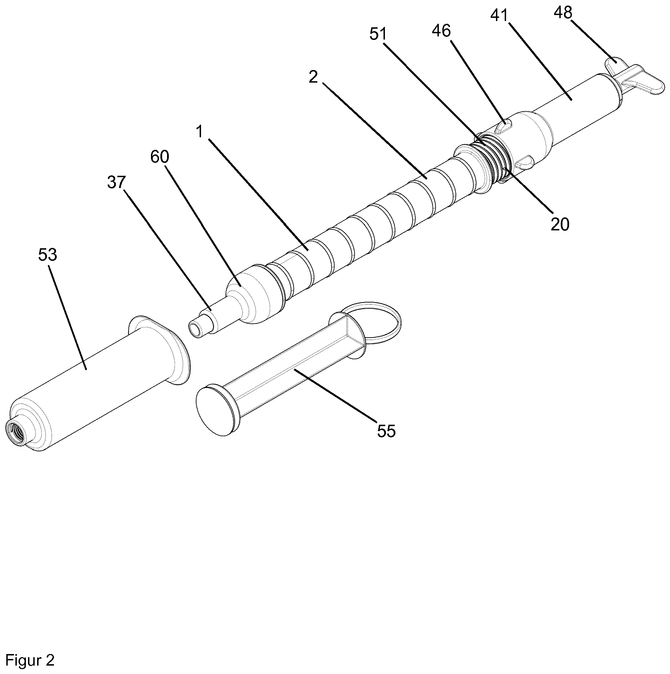

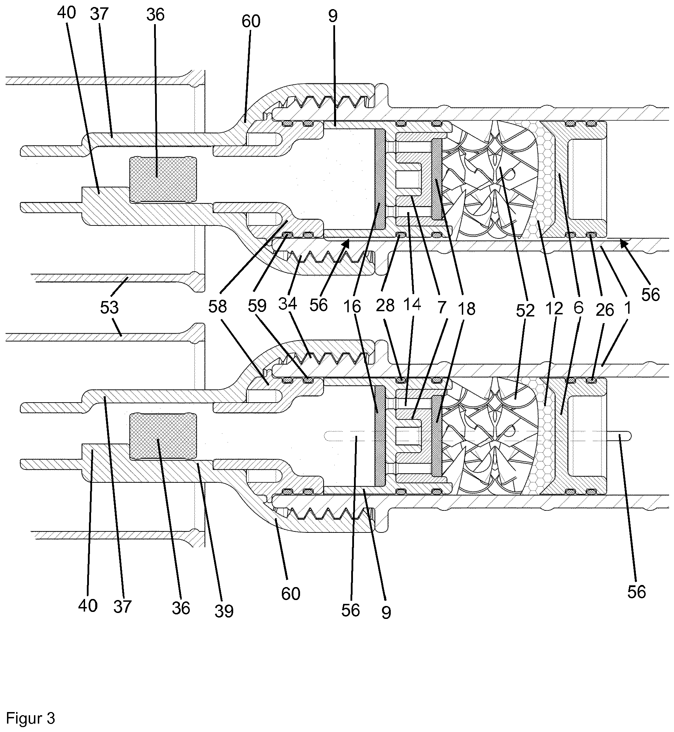

In den Figuren 1 bis 3 sind Abbildungen einer ersten erfindungsgemäßen Vorrichtung zum Lagern und Mischen einer Monomerflüssigkeit und eines Zementpulvers zur Herstellung eines Knochenzementteigs gezeigt. Die Figur 1 zeigt dabei fünf Querschnittansichten übereinander zur Erläuterung des Ablaufs eines erfindungsgemäßen Verfahrens, wobei sich der Zustand der Vorrichtung von oben nach unten während des Verfahrens ändert. Figur 2 zeigt eine perspektivische Außenansicht und Figur 3 zwei Querschnittansichten als Ausschnittvergrößerungen mit senkrecht zueinander liegenden Querschnittebenen.

Die erste erfindungsgemäße Vorrichtung besteht im Wesentlichen aus einem röhrenförmigen Behälter aus Kunststoff, der als vorderen Teil (in Figur 1 und 2 links) eine Kartusche 1 mit einem zylindrischen Innenraum bildet und der als hinteren Teil eine Monomeraufnahme 2 für eine Glasampulle 3 als Monomerflüssigkeitsbehälter bildet. Anstelle der Glasampulle 3 kann auch ohne weiteres eine aufbrechbare Kunststoffampulle verwendet werden oder es kann durch geringe Umbaumaßnahmen auch ein aufreißbarer Folienbeutel aus einem Metall-beschichteten Kunststoff anstelle der Glasampulle 3 verwendet werden.

Die Rückseite der Vorrichtung ist in den Figuren 1 und 2 rechts gezeigt. Die Röhrenform des Behälters ist gut in den Querschnittansichten der Figur 1 sowie der perspektivischen Ansicht nach Figur 2 zu erkennen. Sowohl der Innenraum der Kartusche 1 als auch der Innenraum der Monomeraufnahme 2 sind zylindrisch mit kreisförmiger Grundfläche. Dabei sind die Durchmesser des Innenraums der Kartusche 1 und der Durchmesser des Innenraums der Monomeraufnahme 2 gleich groß und fluchten. Der Behälter mit der Monomeraufnahme 2 und der Kartusche 1 wird vorzugsweise mit Hilfe einer Spritzgusstechnik aus Kunststoff hergestellt. Die Monomeraufnahme 2 weist also einen zylindrischen Innenraum auf, in den die Glasampulle 3 eingesteckt ist. In der Glasampulle 3 befindet sich die Monomerflüssigkeit 4. In den Innenraum der Kartusche 1 ist ein Zementpulver 5 eingefüllt oder vorzugsweise eingepresst. Die Monomerflüssigkeit 4 und das Zementpulver 5 bilden die Ausgangskomponenten für einen PMMA-Knochenzement, der mit der Vorrichtung herstellbar ist. Aufgrund der Glasampulle 3 kann die Monomerflüssigkeit 4 sehr lange in der Monomeraufnahme 2 und dadurch in der Vorrichtung gelagert werden. Das Zementpulver 5 kann ebenfalls über längere Zeiträume in der Vorrichtung gelagert werden. Die Vorrichtung ist damit zum Lagern der Monomerflüssigkeit 4 und des Zementpulvers 5 als Ausgangskomponenten eines Knochenzementteigs 54 des PMMA-Knochenzements geeignet. Die Vorrichtung ist aber auch zum Mischen des Knochenzementteigs 54 aus den Ausgangskomponenten und zum Austragen des gemischten Knochenzementteigs 54 geeignet und vorgesehen.

In der Monomeraufnahme 2 ist ein im zylindrischen Innenraum der Monomeraufnahme 2 in Längsrichtung beweglicher Förderkolben 6 aus Kunststoff angeordnet. Der Förderkolben 6 ist im Bereich der Rückseite der Monomeraufnahme 2 angeordnet. Die Glasampulle 3 kann mit dem Förderkolben 6 in der Monomeraufnahme 2 zusammengedrückt und dabei zersplittert werden, indem der Förderkolben 6 in Richtung der Vorderseite, also in Richtung der Kartusche 1 gedrückt wird. Der Förderkolben 6 weist an der Vorderseite Abstreifer auf, mit denen Splitter der Glasampulle 3 von der Innenwand der Monomeraufnahme 2 abgestreift werden. Die Abstreifer liegen hierzu seitlich an der Innenwand des Innenraums der Monomeraufnahme 2 an.

In dem Innenraum der Kartusche 1 ist in dessen Rückseite ein Austragskolben 7 aus Kunststoff angeordnet. Hinter dem Förderkolben 6 und der Rückseite der Monomeraufnahme 2 ist ein Druckgasanschluss 8 vorgesehen, mit dem eine Druckgaspatrone 10 als Druckgasquelle an den Innenraum der Monomeraufnahme 2 angeschlossen werden kann, so dass ein Druckgas aus der Druckgaspatrone 10 hinter dem Förderkolben 6 in den Innenraum der Monomeraufnahme 2 einströmen und den Förderkolben 6 mittels des Gasdrucks in Richtung der Vorderseite der Kartusche 1 vortreiben kann.

Der Austragskolben 7 weist an seiner Vorderseite einen Hohlzylinder 9 zum Verlängern der Strecke auf, die die Monomerflüssigkeit 4 durch das Zementpulver 5 fließen muss, bis sie zur Innenwand der Kartusche 1 gelangt. Zudem dient der Hohlzylinder 9 zur Beabstandung des Austragskolbens 7 von einer Austragsöffnung an der Vorderseite des Innenraums der Kartusche 1 sowie zum Schaffen eins Totvolumens zwischen dem Austragskolben 7 und der Vorderseite des Innenraums der Kartusche 1, wenn der Austragskolben 7 beziehungsweise der Hohlzylinder 9 maximal an die Vorderseite des Innenraums der Kartusche 1 gedrückt ist. Der Hohlzylinder 9 ist vorliegend rotationssymmetrisch und ist nach Art eines Rohrstücks geformt. Der Hohlzylinder 9 kann aber auch parallel zur Zylinderachse des Hohlzylinders 9 verlaufende Längsschnitte aufweisen. An der Vorderseite ist der Hohlzylinder 9 eben.

In dem Innenraum der Monomeraufnahme 2 ist eine Lagerung 12 aus Schaumstoff vorgesehen, die als Transportsicherung und als Stoßsicherung für die Glasampulle 3 dient. Damit soll verhindert werden, dass die Glasampulle 3 bei Erschütterungen oder Stößen ungewollt aufbricht. Der Schaumstoff und damit die Lagerung 12 sind durchlässig für Gase.

Die Kartusche 1 und die Monomeraufnahme 2 sind einteilig als gemeinsames Kunststoffteil ausgeführt. Die Monomeraufnahme 2 und die Kartusche 1 sind für die Monomerflüssigkeit 4 flüssigkeitsdurchlässig miteinander über eine Verbindung 14 im Austragskolben 7 verbunden. Die Verbindung 14 durch den Austragskolben 7 mündet durch einen für das Zementpulver 5 undurchlässigen aber für die Monomerflüssigkeit 4 durchlässigen Porenfilter 16 in den Innenraum der Kartusche 1.

In der Einmündung zur Verbindung 14 ist in dem Austragskolben 7 ein Filter 18 angeordnet, mit dem die Splitter der Glasampulle 3 zurückgehalten werden können. Statt dem Filter 18 oder zusätzlich zum Filter 18 kann auch ein Sieb vorgesehen sein.

Mehrere Belüftungsöffnungen 20 sind in der Wandung der Monomeraufnahme 2 im Bereich der Rückseite vorgesehen, durch die der Innenraum der Monomeraufnahme 2 mit Hilfe eines sterilisierenden Gases wie Ethylenoxid sterilisiert werden kann. Die Belüftungsöffnungen 20 sind unmittelbar benachbart zum Förderkolben 6 und zum Druckgasanschluss 8 angeordnet, so dass der Förderkolben 6 und der Druckgasanschluss 8 sich unmittelbar vor die Belüftungsöffnungen 20 schieben und somit die Belüftungsöffnungen 20 verschließt, wenn der Förderkolben 6 und der Druckgasanschluss 8 in Richtung der Kartusche 1 verschoben werden. Dadurch wird verhindert, dass Monomerflüssigkeit 4 durch die Belüftungsöffnungen 20 austreten kann, wenn die Glasampulle 3 in der Monomeraufnahme 2 geöffnet wurde.

Der zylindrische Förderkolben 6 hat einen zur Zylindergeometrie des Innenraums der Monomeraufnahme 2 passenden Außenumfang und ist über zwei umlaufende Dichtungen 26 gegen die Innenwand der Monomeraufnahme 2 flüssigkeitsdicht und druckdicht abgedichtet. Ebenso ist ein äußerer Einsatz des Druckgasanschlusses 8 über zwei äußere umlaufende Dichtungen 27 druckdicht gegen die Innenwand der Monomeraufnahme 2 abgedichtet und der äußere Einsatz gegen ein inneres Teil des Druckgasanschlusses 8 über eine innere umlaufende Dichtung 27 abgedichtet. Ferner ist der Austragskolben 7 über zwei umlaufende Dichtungen 28 gegen die Innenwand der Kartusche 1 flüssigkeitsdicht abgedichtet. Diese Dichtungen 26, 27 dienen dazu, dass der Gasdruck aus der Gasdruckpatrone 10 nicht entweichen kann und für den Vortrieb des Förderkolbens 6 zur Verfügung steht. Die Dichtungen 28 dienen dazu, einen Austritt von Monomerflüssigkeit 4 oder von Knochenzementteig 54 zu verhindern, um eine Kontamination der Umgebung (des OP-Saals und des Anwenders) zu verhindern. Die Dichtungen 26, 27, 28 können hierzu aus Gummi bestehen.

An der Vorderseite der Kartusche 1 ist ein Anschluss 34 in Form eines Außengewindes vorgesehen, auf das ein Kartuschenkopf 60 als Abschluss der Kartusche 1 aufgeschraubt ist. In dem Kartuschenkopf 60 ist die Austragsöffnung gebildet und im Ausgangszustand (siehe die obersten beiden Abbildungen der Figur 1) mit einem Verschluss 36 verschlossen, der in der Austragsöffnung steckt und diese verschließt. Der Verschluss 36 wird erst zum Austragen des gemischten Knochenzementteigs 54 geöffnet (siehe die unterste Abbildung der Figur 1 sowie die beiden Abbildungen nach Figur 3). Der Verschluss 36 ist ein für das Zementpulver 5 undurchlässiger aber für Gase durchlässiger Porenfilter und hat eine zylindrische Form. Der Verschluss 36 ist vorzugsweise aus Porex oder einem anderen offenporigen Kunststoff gefertigt.

An dem Außengewinde 34 an der Vorderseite der Kartusche 1 ist der Kartuschenkopf 60 aufgeschraubt, der ein Leitungselement 37 mit einer Verschlussaufnahme 38 zur Aufnahme des Verschlusses 36 umfasst. Die Verschlussaufnahme 38 ist nach Art einer Hülse geformt und weist vier in Längsrichtung ausgerichtete, sich in die Verschlussaufnahme 38 hinein erhebende Leisten 39 auf. Die Leisten 39 beabstanden den Verschluss 36 von der Innenwand der Verschlussaufnahme 38, wenn der Verschluss 36 in die Verschlussaufnahme 38 hineingedrückt ist. Vor der Verschlussaufnahme 38 verengt sich das Leitungselement 37. In diesem Bereich sind vier weitere Leisten 40 angeordnet, die einen Anschlag 40 für die Bewegung des Verschlusses 36 bilden und also die Bewegung des Verschlusses 36 in die Verschlussaufnahme 38 begrenzen. Zwischen den Leisten 39, 40 ist ein ausreichender freier Leitungsquerschnitt vorgesehen, so dass ein aus den Ausgangskomponenten 4, 5 hergestellter Knochenzementteig 54 (siehe die unteren beiden Abbildungen der Figur 1) zwischen den Leisten 39, der Wandung der Verschlussaufnahme 38 und dem eingeschobenen Verschluss 36 sowie zwischen den Leisten 40 im vorderen Teil des Leitungselements 37 hindurch strömen kann.

An der Rückseite der Monomeraufnahme 2 ist ein Behälter 41 für die Druckgaspatrone 10 aufgeschraubt. Der Behälter 41 umfasst an seiner Vorderseite, die der Monomeraufnahme 2 zugewandt ist, den Druckgasanschluss 8 auf. Die Druckgaspatrone 10 wird von der Rückseite in den Behälter 41 für die Druckgaspatrone 10 eingesteckt und der Behälter 41 mit einem rückseitigen Deckel geschlossen.

An der Vorderseite des Förderkolbens 6 sind vorspringende Keile 42 angeordnet, die einen punktuellen oder linearen Krafteintrag in die Glasampulle 3 ermöglichen und so das Aufbrechen der Glasampulle 3 begünstigen. Die Keile 42 sind zum Spalten oder Brechen der Glasampulle 3 bei Vortreiben des Förderkolbens 6 vorgesehen.

Durch den als Porenfilter ausgeführten Verschluss 36 hindurch können das Innere der Kartusche 1 und das Zementpulver 5 mit Hilfe von Ethylenoxid sterilisiert werden, da das Leitungselement 37 offen ist und der Verschluss 36 und die Zwischenräume zwischen den Pulverpartikeln des Zementpulvers 5 luftdurchlässig sind. Gleichzeitig kann Luft aus der Monomeraufnahme 2 durch das Zementpulver 5, den Verschluss 36 und das offene Leitungselement 37 herausgepresst werden, wenn der Förderkolben 6 in Richtung der Monomeraufnahme 2 gepresst wird.

Das Zementpulver 5 ist in der Kartusche 1 eingeschlossen, da alle Öffnungen und Verbindungen 14 mit Hilfe der Porenfilter 16, 36 für das Zementpulver 5 undurchlässig verschlossen sind. Der Inhalt der Kartusche 1 kann dabei durch Evakuieren und Spülen mit Ethylenoxid sterilisiert werden. Dadurch ist die Vorrichtung auch zum langfristigen Lagern des Zementpulvers 5 geeignet. Das Ethylenoxid kann in dem in der obersten Abbildung von Figur 1 Zustand durch die Vorrichtung hindurchgespült werden, da eine durchgängige gasdurchlässige Verbindung zwischen der Austragsöffnung im Kartuschenkopf 60 und den Belüftungsöffnungen 20 vorhanden ist. Nach dem Sterilisieren mit Ethylenoxid werden die Belüftungsöffnungen 20 verschlossen, in dem der Behälter 41 für die Druckgaspatrone 10 weiter auf die Monomeraufnahme 2 aufgeschraubt wird, so dass die Belüftungsöffnungen 20 von Innen von dem Förderkolben 6 und den äußeren Dichtungen 27 an dem Einsatz des Druckgasanschlusses 8 überfahren und dadurch abgedichtet und verschlossen werden. Hierzu weist der Druckgasanschluss 8 beziehungsweise der Behälter 41 für die Druckgaspatrone 10 ein Innengewinde 50 auf und die Rückseite der Monomeraufnahme 2 ein dazu passendes Außengewinde 51. Die Belüftungsöffnungen 20 sind im Bereich des Außengewindes 51 angeordnet.

Der Druckgasanschluss 8 weist eine Hohlnadel 43 auf, mit der die Druckgaspatrone 10 zu öffnen ist, wenn diese mit einer Membran an ihrer Vorderseite auf die Hohlnadel 43 geschoben wird. Dann strömt das Druckgas aus der Druckgaspatrone 10 durch die Hohlnadel 43 und einen Sterilfilter 44 an der Rückseite der Monomeraufnahme 2 hinter dem Förderkolben 6 in den Innenraum der Monomeraufnahme 2.

Um den Behälter 41 für die Druckgaspatrone 10 bequem auf die Monomeraufnahme 2 aufschrauben zu können, sind an dem Behälter 41 außen Flügel 46 angeordnet, so dass der Behälter 41 nach Art einer Flügelmutter manuell bis zu einem Anschlag an der Monomeraufnahme 2 aufgeschraubt werden kann, um die Belüftungsöffnungen 20 zu verschließen.

In gleicher Weise kann auch die Druckgaspatrone 10 geöffnet werden. Dazu ist ein Flügelschraubkopf 48, der durch eine Durchführung im rückseitigen Deckel des Behälters 41 für die Druckgaspatrone 10 reicht, am Boden der Druckgaspatrone 10 befestigt. Die Druckgaspatrone 10 weist an ihrer der Hohlnadel 43 zugewandten Vorderseite ein Außengewinde auf und der Druckgasanschluss 8 ein dazu passendes Innengewinde. Mit dem Flügelschraubenkopf 48 kann die Druckgaspatrone 10 innerhalb des Behälters 41 nach vorne tiefer in den Druckgasanschluss 8 geschraubt werden, so dass die Membran an der Vorderseite der Druckgaspatrone 10 von der Hohlnadel 43 durchstochen wird und das Druckgas innerhalb der Vorrichtung zur Verfügung steht (siehe mittlere der Abbildungen von Figur 1).

Durch den auf die Rückseite des Förderkolbens 6 wirkenden Druck wird der Förderkolben 6 in Richtung des Kartuschenkopfs 60 gedrückt und die Glasampulle 3 zwischen dem Förderkolben 6 und dem Austragskolben 7 zersplittert und dadurch geöffnet. Die Splitter 52 der Glasampulle 3 werden weiter komprimiert und die freigewordene Monomerflüssigkeit 4 wird durch den Filter 18 oder das Sieb, durch die Verbindungen 14 im Austragskolben 7 und durch den Porenfilter 16 in den Innenraum der Kartusche 1 und damit in das Zementpulver 5 gepresst. Der Austragskolben 7 wird dabei von dem Zementpulver 5 gehalten, da das Zementpulver 5 im trockenen Zustand nicht fließfähig ist. Im Innenraum der Kartusche 1 mischt sich die Monomerflüssigkeit 4 mit dem Zementpulver 5, da in dem Zementpulver 5 ein Additiv verteilt ist, das die Monomerflüssigkeit 4 leitet und dadurch in dem Zementpulver 5 verteilt, bevor die Monomerflüssigkeit 4 mit dem Zementpulver 5 derart reagiert und quillt, dass eine weitere Ausbreitung der Monomerflüssigkeit 4 unterbunden wird. Zusätzlich kann die Monomerflüssigkeit 4 entlang des Hohlzylinders 9 tief in das Zementpulver 5 eindringen. In dem Innenraum der Kartusche 1 wird so der Knochenzementteig 54 gebildet (siehe vierte Abbildung von oben der Figur 1).

Der mit der Vorrichtung erzeugte Knochenzementteig 54 kann mit der Vorrichtung in eine Spritze 53 gefüllt werden, mit der der Knochenzementteig 54 durch den Anwender (den Operateur) beim Patienten appliziert werden kann. Hierzu kann der Knochenzementteig 54 aus der Spritze 53 mit einem Kolben 55 der Spritze 53 nach vorne ausgetrieben werden.

An der Vorderseite des Innenraums der Kartusche 1 ist in der seitlichen Innenwand eine Nut 56 vorgesehen, über die der Gasdruck aus dem Innenraum der Kartusche 1 und dem Innenraum der Monomeraufnahme 2 abgeblasen werden kann, wenn das rückseitige Ende des Förderkolbens 6 an dem hinteren Ende der Nut 56 vorbei geschoben ist (siehe die beiden Abbildungen nach Figur 3). Das Druckgas an der Rückseite der Monomeraufnahme 2 kann dann an dem Förderkolben 6, den Splittern 52 der Glasampulle 3 und dem Austragskolben 7 vorbei strömen und durch die Austragsöffnung entweichen. Dadurch wird die Vorrichtung drucklos und kann gefahrlos entsorgt werden.

Der Verschluss 36 ist im geschlossenen Zustand in einem Einsatz 58 am Kartuschenkopf 60 angeordnet, der in den Innenraum der Kartusche 1 ragt und der mit zwei umlaufenden Dichtungen 59 gegen die Innenwand der Kartusche 1 abgedichtet ist. Der Einsatz 58 bildet dabei einen Anschlag für den Hohlzylinder 9 an der Vorderseite des Austragskolbens 7 und damit für den Austragskolben 7.

Figur 1 zeigt fünf schematische Querschnittansichten der erfindungsgemäßen Vorrichtung übereinander zur Verdeutlichung des Ablaufs eines erfindungsgemäßen Verfahrens. Dazu zeigt Figur 3 eine Ausschnittvergrößerung der letzten Abbildung von oben der Figur 1 und Figur 2 eine Außenansicht der Vorrichtung im Ausgangszustand. Zu Beginn des Verfahrens liegt die Vorrichtung im Ausgangszustand vor, so wie sie auch in der obersten Abbildung von Figur 1 gezeigt ist. In diesem Zustand wird die Vorrichtung sterilisiert. Anschließend wird der Behälter 41 für die Druckgaspatrone 10 mit den Flügeln 46 auf die Monomeraufnahme 2 geschraubt und dadurch die Belüftungsöffnungen 20 verschlossen. Dieser Zustand ist in der zweiten Abbildung von oben der Figur 1 gezeigt.

Anschließend wird die Druckgaspatrone 10 geöffnet, indem die Druckgaspatrone 10 mit der Flügelkopfschraube 48 auf die Hohlnadel 43 geschraubt wird. Diese Situation ist in der dritten Abbildung von oben der Figur 1 gezeigt.

Dann beginnt der wesentliche Teil des erfindungsgemäßen Verfahrens:
Das aus der Druckgaspatrone 10 austretende Gas strömt durch den Sterilfilter 44 und drückt auf den Förderkolben 6 und diesen in Richtung der Kartusche 1 nach vorne. Durch das weiter aus der Druckgaspatrone 10 in den rückseitigen Innenraum der Monomeraufnahme 2 strömende Druckgas wird der Förderkolben 6 in Richtung der Kartusche 1 vorgetrieben. Die Lagerung 12 wird komprimiert und der Förderkolben 6 trifft auf den Kopf der Glasampulle 3. Da die Glasampulle 3 an der Vorderseite an dem Austragskolben 7 anliegt und sich der Innenraum der Monomeraufnahme 2 weiter verkleinert, wird die Glasampulle 3 zerbrochen. Die Monomerflüssigkeit 4 tritt aus der Glasampulle 3 in den Innenraum der Monomeraufnahme 2 aus. Der Austragskolben 7 kann nicht oder nicht weit von der Glasampulle 3 in Richtung des Verschlusses 36 geschoben werden, wenn das Zementpulver 5 trocken ist, also nicht von der Monomerflüssigkeit 4 benetzt ist, da das trockene Zementpulver 5 nicht fließfähig ist und eine Bewegung des Austragskolbens 7 blockiert. Diese Situation ist in Figur 1, dritte Abbildung von oben gezeigt. Überstehende Luft aus der Monomeraufnahme 2 wird durch den Filter 18, die Verbindung 14, den Porenfilter 16, durch die Zwischenräume zwischen den Partikeln des Zementpulvers 5, durch den Verschluss 36 und aus dem Leitungselement 37 aus der Vorrichtung herausgedrückt.

Von der Glasampulle 3 bleiben schließlich nur kleine Splitter 52 zurück, die von dem Filter 18 zurückgehalten werden und in dem röhrenförmigen Behälter verbleiben. Die Monomerflüssigkeit 4 wird durch den Filter 18, die Verbindung 14 und den Porenfilter 16 in das Zementpulver 5 gepresst und beginnt dort mit dem Zementpulver 5 zu reagieren, so dass sich aus dem Gemisch der Knochenzementteig 54 bildet. Dabei kann die Monomerflüssigkeit 4 nicht direkt von dem Porenfilter 16 aus zur Innenwand der Kartusche 1 fließen, da diese vollständig oder im Fall eines geschlitzten Hohlzylinders 9 weitgehend von dem Hohlzylinder 9 abgedeckt ist. Dadurch wird die Monomerflüssigkeit 4 gezwungen sich einen Weg durch das Zementpulver 5 zu bahnen. Monomerflüssigkeitsblasen oder Monomerflüssigkeitsansammlungen können so verhindert werden.

Die Menge der Monomerflüssigkeit 4 ist so gewählt, dass das Zementpulver 5 bis in die vorderste Spitze der Kartusche 1, das heißt, bis an den Verschluss 36 heran mit der Monomerflüssigkeit 4 benetzt wird. Der Verschluss 36 wird, sobald das Gemisch also der Knochenzementteig 54 entstanden ist, von dem auf den Knochenzementteig 54 aufgrund des Drucks auf den Austragskolben 7 wirkenden Druck nach vorne getrieben und in die Verschlussaufnahme 38 hineingedrückt, bis der Verschluss 36 auf den Anschlag 40 trifft, wo die Bewegung des Verschlusses 36 endet. Diese Situation ist in Figur 1, vierte Abbildung von oben gezeigt. Der Knochenzementteig 54 umfließt den Verschluss 36, indem er zwischen den Leisten 39 und zwischen den Leisten 40 hindurchfließt. Schließlich tritt der Knochenzementteig 54 an der Vorderseite der Vorrichtung aus.

Spätestens in diesem Zustand (oder bevorzugt bereits beim Öffnen der Druckgaspatrone 10) wird eine Spritze 53 bereitgestellt, die den Knochenzementteig 54 aufnehmen kann. Durch weiteres Vortreiben des Förderkolbens 6, der Scherben 52 und des davor angeordnete Austragskolbens 7 mit dem Druckgas wird der Knochenzementteig 54 aus der Kartusche 1 ausgetrieben und in die Spritze 53 zur weiteren Verwendung gefüllt. Diese Situation ist in der letzten Abbildung von oben der Figur 1 sowie den beiden Abbildungen nach Figur 3 gezeigt.

Schließlich trifft der Hohlzylinder 9 auf den Kartuschenkopf 60 beziehungsweise den Einsatz 58 im Kartuschenkopf 60 an der Vorderseite des Innenraums der Kartusche 1. Dabei wird die Nut 56 freigelegt und der Druck an der Rückseite des Förderkolbens 6 entweicht durch die Nut 56 und durch die Austragsöffnung im Kartuschenkopf 60.

Der Hohlzylinder 9 weist eine Höhe von 3 mm, bevorzugt von 5 mm, oder größer auf, so dass durch den dadurch erzeugten Abstand gewährleistet ist, dass die Vorderseite des Austragskolbens 7 von der Vorderseite des Innenraums der Kartusche 1 beabstandet ist, wenn der Austragskolben 7 so weit nach vorne gedrückt es, wie es möglich ist. Dadurch entsteht im Innenraum der Kartusche 1 und zwar in dem von dem Hohlzylinder 9 begrenzten Bereich ein Totvolumen, das nicht aus der Kartusche 1 durch die Austragsöffnung und das Leitungselement 37 ausgetrieben werden kann.

In diesem Totvolumen befindet sich nun ein Teil des Knochenzementteigs 54, der gegebenenfalls einen zu großen Anteil an Monomerflüssigkeit 4 enthält. Dieser Teil kann des Knochenzementteigs 54 kann nicht aus dem Totvolumen aus der Vorrichtung ausgepresst werden. Durch diesen Aufbau wird sichergestellt, dass kein Knochenzementteig 54 mit einer sich ändernden Konsistenz aufgrund sich ändernder Zusammensetzung mit der Vorrichtung appliziert werden kann.

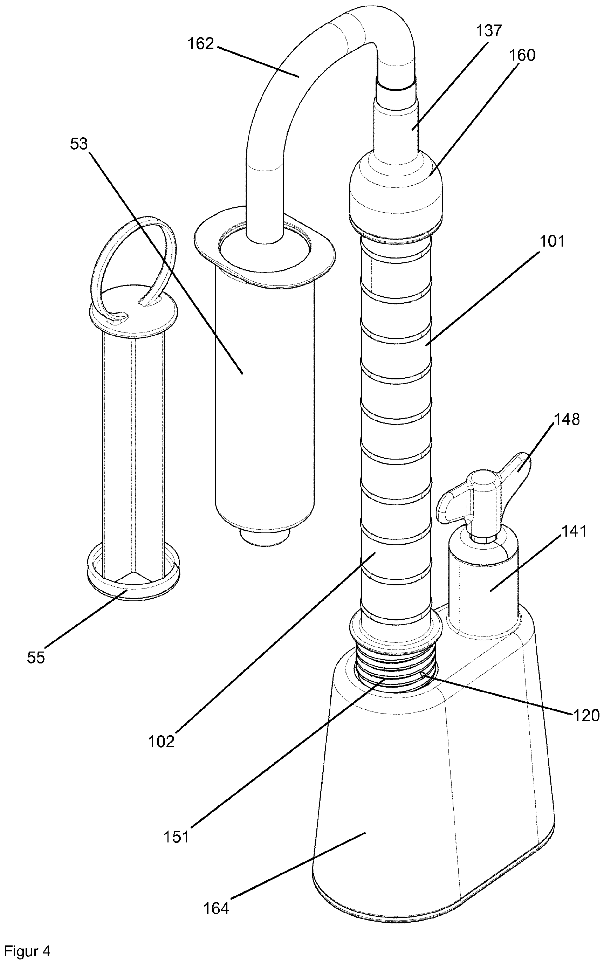

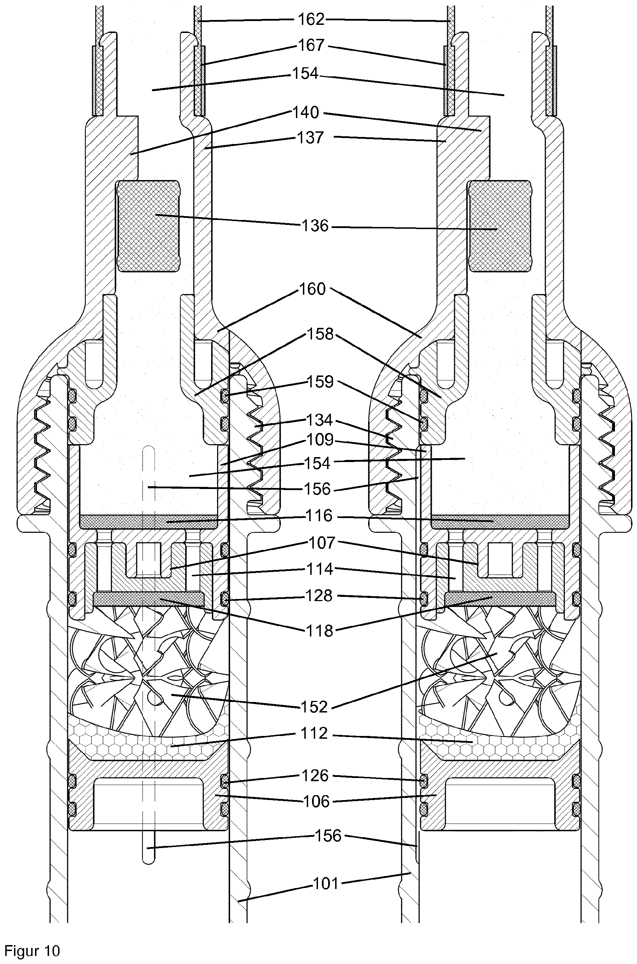

In den Figuren 4 bis 10 sind Abbildungen einer zweiten alternativen erfindungsgemäßen Vorrichtung gezeigt. Die Figuren 4 bis 9 zeigen verschiedene schematische Gesamtansichten der beispielhaften zweiten erfindungsgemäßen Vorrichtung. Die Figur 10 zeigt zwei schematische Querschnittansichten als Detailansichten in Form von Ausschnittvergrößerungen durch den vorderen Bereich der zweiten erfindungsgemäßen Vorrichtung.

Diese zweite erfindungsgemäße Vorrichtung unterscheidet sich von der ersten vor allem dadurch, dass sie aufstellbar ist und dass der Druckgasanschluss nicht direkt an der Monomeraufnahme platziert ist.

Die erste erfindungsgemäße Vorrichtung besteht im Wesentlichen aus einem röhrenförmigen Behälter aus Kunststoff, der als vorderen Teil (in Figur 4 bis 9 oben) eine Kartusche 101 mit einem zylindrischen Innenraum bildet und der als hinteren Teil eine Monomeraufnahme 102 für eine Kunststoff- oder Glasampulle 103 als Monomerflüssigkeitsbehälter bildet. Anstelle der Kunststoff- oder Glasampulle 103 kann durch geringe Umbaumaßnahmen auch ein aufreißbarer Folienbeutel aus einem Metall-beschichteten Kunststoff verwendet werden.

Die Rückseite der Vorrichtung ist in den Figuren 4 bis 9 unten gezeigt. Die Röhrenform des Behälters ist gut in den Querschnittansichten sowie der perspektivischen Ansicht nach Figur 4 zu erkennen. Sowohl der Innenraum der Kartusche 101 als auch der Innenraum der Monomeraufnahme 102 sind zylindrisch mit kreisförmiger Grundfläche. Dabei sind die Durchmesser des Innenraums der Kartusche 101 und der Durchmesser des Innenraums der Monomeraufnahme 102 gleich groß und fluchten. Der Behälter mit der Monomeraufnahme 102 und der Kartusche 101 wird vorzugsweise mit Hilfe einer Spritzgusstechnik aus Kunststoff hergestellt. Die Monomeraufnahme 102 weist also einen zylindrischen Innenraum auf, in den die Kunststoff- oder Glasampulle 103 eingesteckt ist. In der Kunststoff- oder Glasampulle 103 befindet sich die Monomerflüssigkeit 104. In den Innenraum der Kartusche 101 ist ein Zementpulver 105 eingefüllt oder vorzugsweise eingepresst. Die Monomerflüssigkeit 104 und das Zementpulver 105 bilden die Ausgangskomponenten für einen PMMA-Knochenzement, der mit der Vorrichtung herstellbar ist. Aufgrund der Kunststoff- oder Glasampulle 103 kann die Monomerflüssigkeit 104 sehr lange in der Monomeraufnahme 102 und dadurch in der Vorrichtung gelagert werden. Das Zementpulver 105 kann ebenfalls über längere Zeiträume in der Vorrichtung gelagert werden. Die Vorrichtung ist damit zum Lagern der Monomerflüssigkeit 104 und des Zementpulvers 105 als Ausgangskomponenten eines Knochenzementteigs 154 des PMMA-Knochenzements geeignet. Die Vorrichtung ist aber auch zum Mischen des Knochenzementteigs 154 aus den Ausgangskomponenten und zum Austragen des gemischten Knochenzementteigs 154 geeignet und vorgesehen.

Im zylindrischen Innenraum der Monomeraufnahme 102 ist ein in Längsrichtung beweglicher Förderkolben 106 aus Kunststoff angeordnet. Der Förderkolben 106 ist im Bereich der Rückseite der Monomeraufnahme 102 angeordnet. Die Kunststoff- oder Glasampulle 103 kann mit dem Förderkolben 106 in der Monomeraufnahme 102 zusammengedrückt und dabei zersplittert werden, indem der Förderkolben 106 in Richtung der Vorderseite, also in Richtung der Kartusche 101 gedrückt wird. Der Förderkolben 106 weist an der Vorderseite Abstreifer auf, mit denen Splitter 152 der Kunststoff- oder Glasampulle 103 von der Innenwand der Monomeraufnahme 102 abgestreift werden. Die Abstreifer liegen hierzu seitlich an der Innenwand des Innenraums der Monomeraufnahme 102 an.

In dem Innenraum der Kartusche 101 ist in dessen Rückseite ein Austragskolben 107 aus Kunststoff angeordnet. Ein Druckgasanschluss 108 ist seitlich neben der Monomeraufnahme 102 und parallel zur Monomeraufnahme 102 in einem Standfuß 164 angeordnet. Der Standfuß 164 weist eine ebene Unterseite auf, so dass die Vorrichtung auf einer ebenen Unterlage, wie einem Tisch, aufgestellt werden kann. Über den Standfuß 164 sind die Monomeraufnahme 102 mit der Kartusche 101 neben dem Druckgasanschluss 108 angeordnet. Der Druckgasanschluss 108 ist über eine Druckgasleitung 166 mit dem Förderkolben 106 und der Rückseite der Monomeraufnahme 102 gasdurchlässig verbunden. Mit dem Druckgasanschluss 108 und über die Druckgasleitung 166 kann eine Druckgaspatrone 110 als Druckgasquelle an den Innenraum der Monomeraufnahme 102 angeschlossen werden, so dass ein Druckgas aus der Druckgaspatrone 110 hinter dem Förderkolben 106 in den Innenraum der Monomeraufnahme 102 einströmen und den Förderkolben 106 mittels des Gasdrucks in Richtung der Vorderseite der Kartusche 101 vortreiben kann.

Der Austragskolben 107 weist an seiner Vorderseite einen Hohlzylinder 109 zum Verlängern der Strecke auf, die die Monomerflüssigkeit 104 durch das Zementpulver 105 fließen muss, bis sie zur Innenwand der Kartusche 101 gelangt. Zudem dient der Hohlzylinder 109 zur Beabstandung des Austragskolbens 107 von einer Austragsöffnung an der Vorderseite des Innenraums der Kartusche 101 sowie zum Schaffen eins Totvolumens zwischen dem Austragskolben 107 und der Vorderseite des Innenraums der Kartusche 101, wenn der Austragskolben 107 beziehungsweise der Hohlzylinder 109 maximal an die Vorderseite des Innenraums der Kartusche 101 gedrückt ist. Der Hohlzylinder 109 ist vorliegend rotationssymmetrisch und ist nach Art eines geschlitzten Rohrstücks geformt. Der Hohlzylinder 109 hat dazu parallel zur Zylinderachse des Hohlzylinders 109 verlaufende Längsschnitte.

In dem Innenraum der Monomeraufnahme 102 ist eine Lagerung 112 aus Schaumstoff vorgesehen, die als Transportsicherung und als Stoßsicherung für die Kunststoff- oder Glasampulle 103 dient. Damit soll verhindert werden, dass die Kunststoff- oder Glasampulle 103 bei Erschütterungen oder Stößen ungewollt aufbricht.

Die Kartusche 101 und die Monomeraufnahme 102 sind einteilig als gemeinsames Kunststoffteil ausgeführt. Die Monomeraufnahme 102 und die Kartusche 101 sind für die Monomerflüssigkeit 104 flüssigkeitsdurchlässig miteinander über eine Verbindung 114 im Austragskolben 107 verbunden. Die Verbindung 114 durch den Austragskolben 107 mündet durch einen für das Zementpulver 105 undurchlässigen aber für die Monomerflüssigkeit 104 durchlässigen Porenfilter 116 in den Innenraum der Kartusche 101.

In der Einmündung zur Verbindung 114 ist in dem Austragskolben 107 ein Filter 118 angeordnet, mit dem die Splitter 152 der Kunststoff- oder Glasampulle 103 zurückgehalten werden können. Statt dem Filter 118 oder zusätzlich zum Filter 118 kann auch ein Sieb vorgesehen sein.

Mehrere Belüftungsöffnungen 120 sind in der Wandung der Monomeraufnahme 102 im Bereich der Rückseite vorgesehen, durch die der Innenraum der Monomeraufnahme 102 mit Hilfe eines sterilisierenden Gases wie Ethylenoxid sterilisiert werden kann. Die Belüftungsöffnungen 120 sind unmittelbar benachbart zum Förderkolben 106 und zum einem Einsatz mit umlaufenden Dichtungen 127 angeordnet, so dass der Förderkolben 106 und der Einsatz mit den Dichtungen 127 sich unmittelbar vor die Belüftungsöffnungen 120 schieben und somit die Belüftungsöffnungen 120 verschließt, wenn der Förderkolben 106 und der Einsatz mit den Dichtungen 127 in Richtung der Kartusche 101 verschoben werden, indem die Monomeraufnahme 102 in den Standfuß 164 mit einem Innengewinde 150 geschraubt wird. Hierzu weist die Monomeraufnahme 102 an der Rückseite ein Außengewinde 151 auf. Durch das Schließen der Belüftungsöffnungen 120 wird verhindert, dass Monomerflüssigkeit 104 durch die Belüftungsöffnungen 120 austreten kann, wenn die Kunststoff- oder Glasampulle 103 in der Monomeraufnahme 102 geöffnet wurde.

Der zylindrische Förderkolben 106 hat einen zur Zylindergeometrie des Innenraums der Monomeraufnahme 102 passenden Außenumfang und ist über zwei umlaufende Dichtungen 126 gegen die Innenwand der Monomeraufnahme 102 flüssigkeitsdicht und druckdicht abgedichtet. Ebenso ist der Einsatz über die zwei äußeren umlaufenden Dichtungen 127 druckdicht gegen die Innenwand der Monomeraufnahme 102 abgedichtet und der Einsatz gegen eine Einmündung der Druckgasleitung 166 über eine innere umlaufende Dichtung 127 abgedichtet. Ferner ist der Austragskolben 107 über zwei umlaufende Dichtungen 128 gegen die Innenwand der Kartusche 101 flüssigkeitsdicht abgedichtet. Diese Dichtungen 126, 127 dienen dazu, dass der Gasdruck aus der Gasdruckpatrone 110 nicht entweichen kann und für den Vortrieb des Förderkolbens 106 zur Verfügung steht. Die Dichtungen 128 dienen dazu, einen Austritt von Monomerflüssigkeit 104 oder von Knochenzementteig 154 zu verhindern, um eine Kontamination der Umgebung (des OP-Saals und des Anwenders) zu verhindern. Die Dichtungen 126, 127, 128 können hierzu aus Gummi bestehen.

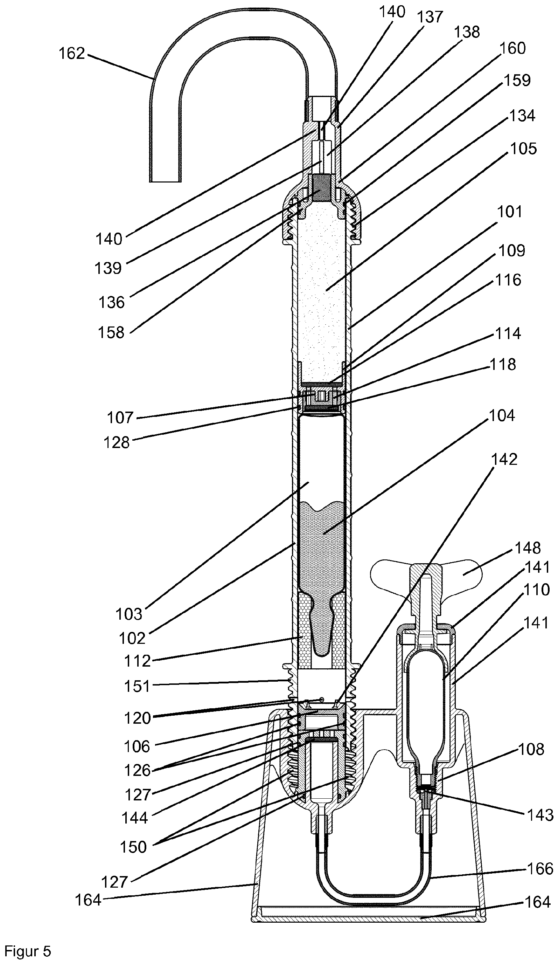

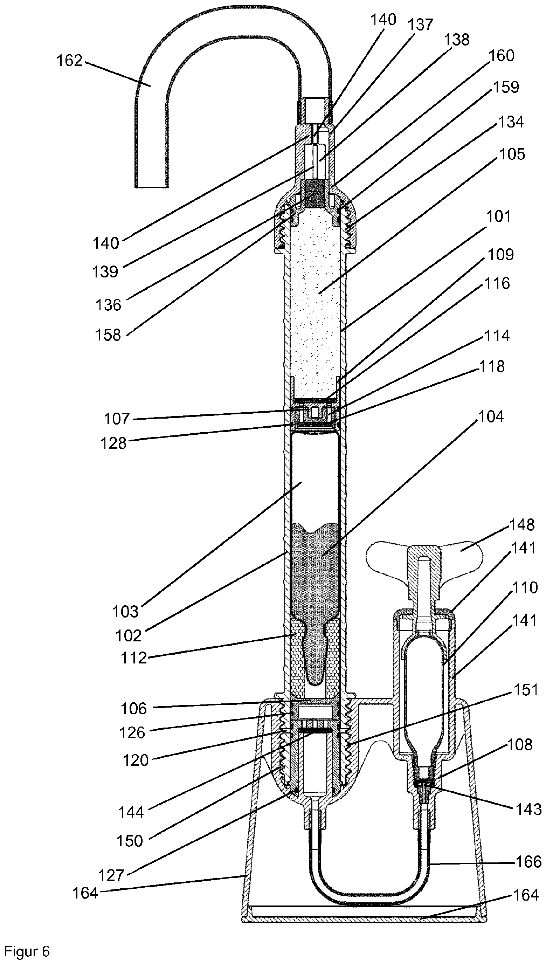

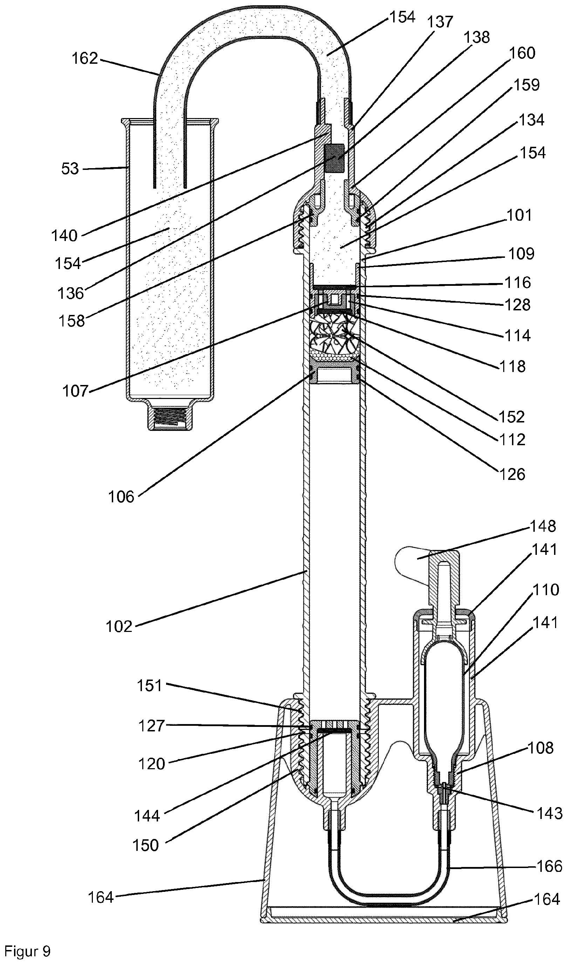

An der Vorderseite der Kartusche 101 ist ein Anschluss 134 in Form eines Außengewindes vorgesehen, auf das ein Kartuschenkopf 160 als Abschluss der Kartusche 101 aufgeschraubt ist. In dem Kartuschenkopf 160 ist die Austragsöffnung gebildet und im Ausgangszustand (siehe Figur 5 und 6) mit einem Verschluss 136 verschlossen, der in der Austragsöffnung steckt und diese verschließt. Der Verschluss 136 wird erst zum Austragen des gemischten Knochenzementteigs 154 geöffnet (siehe Figur 9 sowie die beiden Abbildungen nach Figur 10). Der Verschluss 136 ist ein für das Zementpulver 105 undurchlässiger aber für Gase durchlässiger Porenfilter und hat eine zylindrische Form. Der Verschluss 136 ist vorzugsweise aus Porex oder einem anderen offenporigen Kunststoff gefertigt.

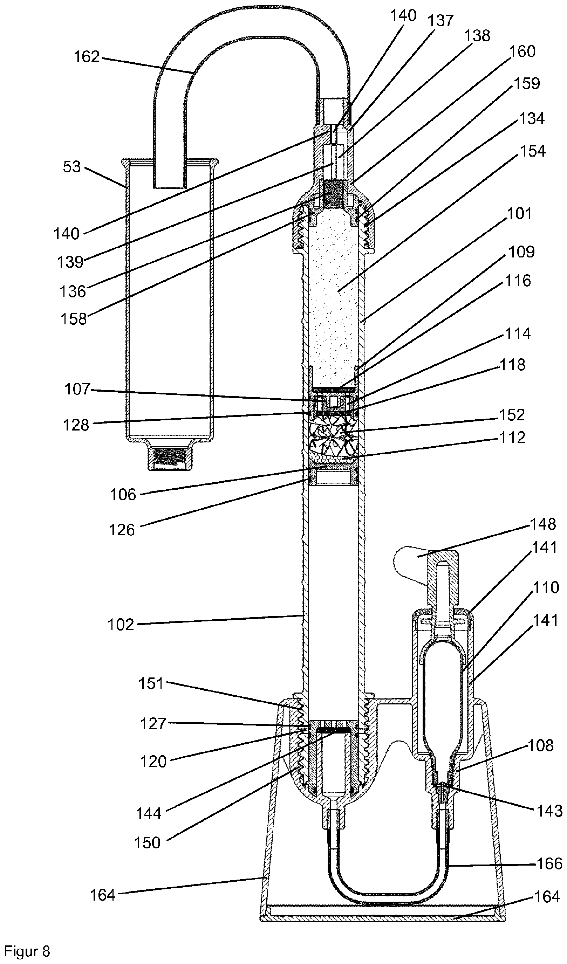

An dem Außengewinde 134 an der Vorderseite der Kartusche 101 ist der Kartuschenkopf 160 aufgeschraubt, der ein Leitungselement 137 mit einer Verschlussaufnahme 138 zur Aufnahme des Verschlusses 136 umfasst. Die Verschlussaufnahme 138 ist nach Art einer Hülse geformt und weist vier in Längsrichtung ausgerichtete, sich in die Verschlussaufnahme 138 hinein erhebende Leisten 139 auf. Die Leisten 139 beabstanden den Verschluss 136 von der Innenwand der Verschlussaufnahme 138, wenn der Verschluss 136 in die Verschlussaufnahme 138 hineingedrückt ist. Vor der Verschlussaufnahme 138 verengt sich das Leitungselement 137. In diesem Bereich sind vier weitere Leisten 140 angeordnet, die einen Anschlag 140 für die Bewegung des Verschlusses 136 bilden und also die Bewegung des Verschlusses 136 in die Verschlussaufnahme 138 begrenzen. Zwischen den Leisten 139, 140 ist ein ausreichender freier Leitungsquerschnitt vorgesehen, so dass ein aus den Ausgangskomponenten 104, 105 hergestellter Knochenzementteig 154 (siehe Figuren 8 und 9) zwischen den Leisten 139, der Wandung der Verschlussaufnahme 138 und dem eingeschobenen Verschluss 136 sowie zwischen den Leisten 140 im vorderen Teil des Leitungselements 137 hindurch strömen kann.

An der Vorderseite des Kartuschenkopfs 160 ist ein Rohr 162 mit einer Krimphülse 167 befestigt, durch das der Knochenzementteig 154 abgefüllt werden kann. Hierzu weist das Rohr 162 eine Biegung auf, so dass der Knochenzementteig 154 bei einer Aufstellung mit dem Standfuß 164 auf einer horizontalen Unterlage, wie einem Tisch, nach unten abgegeben wird (siehe Figur 9). Durch die Gravitation kann dann der Knochenzementteig 154 nach unten auslaufen.

An dem Standfuß 164 ist ein Behälter 141 für die Druckgaspatrone 110 ausgeformt. Der Behälter 141 für die Druckgaspatrone 110 ist dabei größtenteils als einteiliges Spritzgussteil zusammen mit dem Standfuß 164 aus Kunststoff ausgeformt. Der Behälter 141 umfasst an seiner Vorderseite, die der Druckgasleitung 166 zugewandt ist, den Druckgasanschluss 108 auf. Die Druckgaspatrone 110 wird von der Rückseite in den Behälter 141 für die Druckgaspatrone 110 eingesteckt und der Behälter 141 mit einem rückseitigen Deckel geschlossen.

An der Vorderseite des Förderkolbens 106 sind vorspringende Keile 142 angeordnet, die einen punktuellen oder linearen Krafteintrag in die Kunststoff- oder Glasampulle 103 ermöglichen und so das Aufbrechen der Kunststoff- oder Glasampulle 103 begünstigen. Die Keile 142 sind zum Spalten oder Brechen der Kunststoff- oder Glasampulle 103 bei Vortreiben des Förderkolbens 106 vorgesehen.

Durch den als Porenfilter ausgeführten Verschluss 136 hindurch können das Innere der Kartusche 101 und das Zementpulver 105 mit Hilfe von Ethylenoxid sterilisiert werden, da das Leitungselement 137 offen ist und der Verschluss 136 und die Zwischenräume zwischen den Pulverpartikeln des Zementpulvers 105 luftdurchlässig sind. Gleichzeitig kann Luft aus der Monomeraufnahme 102 durch das Zementpulver 105, den Verschluss 136 und das offene Leitungselement 137 herausgepresst werden, wenn der Förderkolben 106 in Richtung der Monomeraufnahme 102 gepresst wird.

Das Zementpulver 105 ist in der Kartusche 101 eingeschlossen, da alle Öffnungen und Verbindungen 114 mit Hilfe der Porenfilter 116, 136 für das Zementpulver 105 undurchlässig verschlossen sind. Der Inhalt der Kartusche 101 kann dabei durch Evakuieren und Spülen mit Ethylenoxid sterilisiert werden. Dadurch ist die Vorrichtung auch zum langfristigen Lagern des Zementpulvers 105 geeignet. Das Ethylenoxid kann in Figur 4 und 5 gezeigten Zustand durch die Vorrichtung hindurchgespült werden, da eine durchgängige gasdurchlässige Verbindung zwischen der Austragsöffnung im Kartuschenkopf 160 und den Belüftungsöffnungen 120 vorhanden ist. Nach dem Sterilisieren mit Ethylenoxid werden die Belüftungsöffnungen 120 verschlossen, in dem der die Monomeraufnahme 102 weiter in den Standfuß 164 und dadurch auf den Einsatz mit den Dichtungen 127 eingeschraubt wird, so dass die Belüftungsöffnungen 120 von Innen von dem Förderkolben 106 und den äußeren Dichtungen 127 an dem Einsatz überfahren und dadurch abgedichtet und verschlossen werden. Hierzu weist der Standfuß 164 das Innengewinde 150 auf und die Rückseite der Monomeraufnahme 102 das dazu passende Außengewinde 151. Die Belüftungsöffnungen 120 sind im Bereich des Außengewindes 151 angeordnet.

Der Druckgasanschluss 108 weist eine Hohlnadel 143 auf, mit der die Druckgaspatrone 110 zu öffnen ist, wenn diese mit einer Membran an ihrer Vorderseite auf die Hohlnadel 143 geschoben wird. Dann strömt das Druckgas aus der Druckgaspatrone 110 durch die Hohlnadel 143, die Druckgasleitung 166 und einen Sterilfilter 144 in die Rückseite des Innenraums der Monomeraufnahme 102 hinter dem Förderkolben 106.

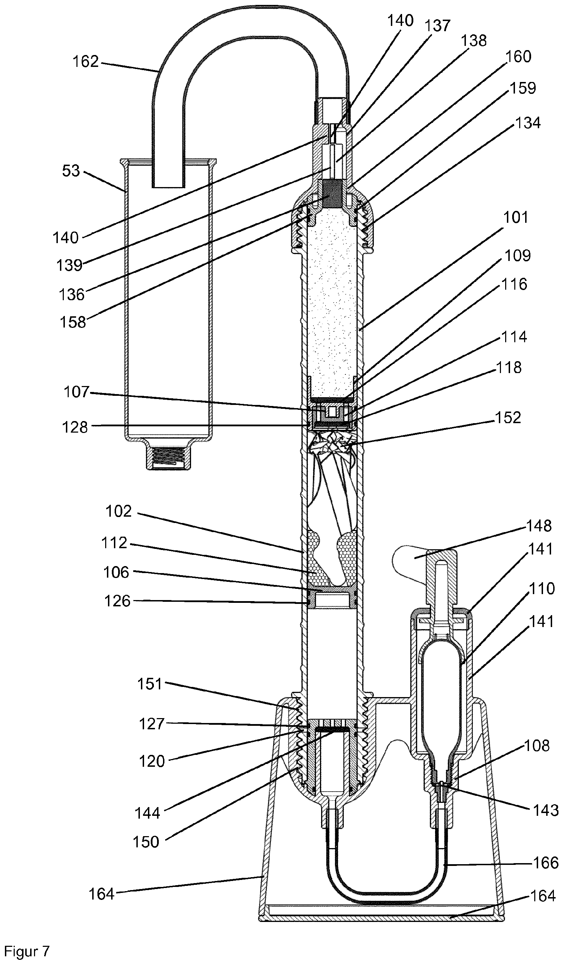

Die Druckgaspatrone 110 kann geöffnet werden, indem ein Flügelschraubkopf 148 gedreht wird, der durch eine Durchführung im rückseitigen Deckel des Behälters 141 für die Druckgaspatrone 110 reicht und am Boden der Druckgaspatrone 110 befestigt ist. Die Druckgaspatrone 110 weist an ihrer der Hohlnadel 143 zugewandten Vorderseite ein Außengewinde auf und der Druckgasanschluss 108 ein dazu passendes Innengewinde. Mit dem Flügelschraubenkopf 148 kann die Druckgaspatrone 110 innerhalb des Behälters 141 nach vorne tiefer in den Druckgasanschluss 108 geschraubt werden, so dass die Membran an der Vorderseite der Druckgaspatrone 110 von der Hohlnadel 143 durchstochen wird und das Druckgas innerhalb der Vorrichtung zur Verfügung steht (siehe Figur 7).

Durch den auf die Rückseite des Förderkolbens 106 wirkenden Druck wird der Förderkolben 106 in Richtung des Kartuschenkopfs 160 gedrückt und die Kunststoff- oder Glasampulle 103 zwischen dem Förderkolben 106 und dem Austragskolben 107 zersplittert und dadurch geöffnet. Die Splitter 152 der Kunststoff- oder Glasampulle 103 werden weiter komprimiert und die freigewordene Monomerflüssigkeit 104 wird durch den Filter 118, durch die Verbindungen 114 im Austragskolben 107 und durch den Porenfilter 116 in den Innenraum der Kartusche 101 und damit in das Zementpulver 105 gepresst. Der Austragskolben 107 wird dabei von dem Zementpulver 105 gehalten, da das Zementpulver 105 im trockenen Zustand nicht fließfähig ist. Im Innenraum der Kartusche 101 mischt sich die Monomerflüssigkeit 104 mit dem Zementpulver 105, da in dem Zementpulver 105 ein Additiv verteilt ist, das die Monomerflüssigkeit 104 leitet und dadurch in dem Zementpulver 105 verteilt, bevor die Monomerflüssigkeit 104 mit dem Zementpulver 105 derart reagiert und quillt, dass eine weitere Ausbreitung der Monomerflüssigkeit 104 unterbunden wird. Zusätzlich kann die Monomerflüssigkeit 104 entlang des Hohlzylinders 109 tief in das Zementpulver 105 eindringen. In dem Innenraum der Kartusche 101 wird so der Knochenzementteig 154 gebildet (siehe Figur 8).

Der mit der Vorrichtung erzeugte Knochenzementteig 154 kann mit der Vorrichtung in eine Spritze 53 gefüllt werden, mit der der Knochenzementteig 154 durch den Anwender (den Operateur) beim Patienten appliziert werden kann. Hierzu kann der Knochenzementteig 154 aus der Spritze 53 mit einem Kolben 55 der Spritze 53 nach vorne ausgetrieben werden.

An der Vorderseite des Innenraums der Kartusche 101 ist in der seitlichen Innenwand eine Nut 156 vorgesehen, über die der Gasdruck aus dem Innenraum der Kartusche 101 und dem Innenraum der Monomeraufnahme 102 abgeblasen werden kann, wenn das rückseitige Ende des Förderkolbens 106 an dem hinteren Ende der Nut 156 vorbei geschoben ist (siehe die beiden Abbildungen nach Figur 10). Das Druckgas an der Rückseite der Monomeraufnahme 102 kann dann an dem Förderkolben 106, den Splittern 152 der Kunststoff- oder Glasampulle 103 und dem Austragskolben 107 vorbei strömen und durch die Austragsöffnung entweichen. Dadurch wird die Vorrichtung drucklos und kann gefahrlos entsorgt werden.

Der Verschluss 136 ist im geschlossenen Zustand in einem Einsatz 158 am Kartuschenkopf 160 angeordnet, der vorne in den Innenraum der Kartusche 101 ragt und der mit zwei umlaufenden Dichtungen 159 gegen die Innenwand der Kartusche 101 abgedichtet ist. Der Einsatz 158 bildet dabei einen Anschlag für den Hohlzylinder 109 an der Vorderseite des Austragskolbens 107 und damit für den Austragskolben 107.

Die Figuren 5 bis 9 zeigen fünf schematische Querschnittansichten der erfindungsgemäßen Vorrichtung zur Verdeutlichung des Ablaufs eines erfindungsgemäßen Verfahrens. Dazu zeigt Figur 10 eine Ausschnittvergrößerung der Figur 9 und Figur 4 eine Außenansicht der Vorrichtung im Ausgangszustand. Zu Beginn des Verfahrens liegt die Vorrichtung im Ausgangszustand vor, so wie sie auch in Figur 4 und 5 gezeigt ist. In diesem Zustand wird die Vorrichtung sterilisiert. Anschließend wird die Monomeraufnahme 102 in den Standfuß 164 geschraubt und dadurch die Belüftungsöffnungen 120 verschlossen. Dieser Zustand ist in der zweiten Abbildung von oben der Figur 6 gezeigt.

Anschließend wird die Druckgaspatrone 110 geöffnet, indem die Druckgaspatrone 110 mit der Flügelkopfschraube 148 auf die Hohlnadel 143 geschraubt wird. Diese Situation ist in Figur 7 gezeigt.

Dann beginnt der wesentliche Teil des erfindungsgemäßen Verfahrens:
Das aus der Druckgaspatrone 110 austretende Gas strömt durch die Druckgasleitung 166 und durch den Sterilfilter 144 und drückt auf den Förderkolben 106 und diesen in Richtung der Kartusche 101 nach vorne. Durch das weiter aus der Druckgaspatrone 110 in den rückseitigen Innenraum der Monomeraufnahme 102 strömende Druckgas wird der Förderkolben 106 in Richtung der Kartusche 101 vorgetrieben. Die Lagerung 112 wird komprimiert und der Förderkolben 106 trifft auf den Kopf der Kunststoff- oder Glasampulle 103. Da die Kunststoff- oder Glasampulle 103 an der Vorderseite an dem Austragskolben 107 anliegt und sich der Innenraum der Monomeraufnahme 102 weiter verkleinert, wird die Kunststoff- oder Glasampulle 103 zerbrochen. Die Monomerflüssigkeit 104 tritt aus der Kunststoff- oder Glasampulle 103 in den Innenraum der Monomeraufnahme 102 aus. Der Austragskolben 107 kann nicht oder nicht weit von der Kunststoff- oder Glasampulle 103 in Richtung des Verschlusses 136 geschoben werden, wenn das Zementpulver 105 trocken ist, also nicht von der Monomerflüssigkeit 104 benetzt ist, da das trockene Zementpulver 105 nicht fließfähig ist und eine Bewegung des Austragskolbens 107 blockiert. Diese Situation ist in Figur 7 gezeigt. Überstehende Luft aus der Monomeraufnahme 102 wird durch den Filter 118, die Verbindung 114, den Porenfilter 116, durch die Zwischenräume zwischen den Partikeln des Zementpulvers 105, durch den Verschluss 136 und aus dem Leitungselement 137 aus der Vorrichtung herausgedrückt.

Von der Kunststoff- oder Glasampulle 103 bleiben schließlich nur kleine Splitter 152 zurück, die von dem Filter 118 zurückgehalten werden und in dem röhrenförmigen Behälter verbleiben. Die Monomerflüssigkeit 104 wird durch den Filter 118, die Verbindung 114 und den Porenfilter 116 in das Zementpulver 105 gepresst und beginnt dort mit dem Zementpulver 105 zu reagieren, so dass sich aus dem Gemisch der Knochenzementteig 154 bildet. Dabei kann die Monomerflüssigkeit 104 nicht direkt von dem Porenfilter 116 aus zur Innenwand der Kartusche 101 fließen, da diese von dem geschlitzten Hohlzylinder 109 abgedeckt ist. Dadurch wird die Monomerflüssigkeit 104 gezwungen sich einen Weg durch das Zementpulver 105 zu bahnen. Monomerflüssigkeitsblasen oder Monomerflüssigkeitsansammlungen können so verhindert werden.

Die Menge der Monomerflüssigkeit 104 ist so gewählt, dass das Zementpulver 105 bis in die vorderste Spitze der Kartusche 101, das heißt, bis an den Verschluss 136 heran mit der Monomerflüssigkeit 104 benetzt wird. Der Verschluss 136 wird, sobald das Gemisch also der Knochenzementteig 154 entstanden ist, von dem auf den Knochenzementteig 154 aufgrund des Drucks auf den Austragskolben 107 wirkenden Druck nach vorne getrieben und in die Verschlussaufnahme 138 hineingedrückt, bis der Verschluss 136 auf den Anschlag 140 trifft, wo die Bewegung des Verschlusses 136 endet. Diese Situation ist in Figur 9 und 10 gezeigt. Der Knochenzementteig 154 umfließt den Verschluss 136, indem er zwischen den Leisten 139 und zwischen den Leisten 140 hindurchfließt. Schließlich tritt der Knochenzementteig 154 an der Vorderseite der Vorrichtung durch das Rohr 162 aus.

Spätestens in diesem Zustand (oder bevorzugt bereits beim Öffnen der Druckgaspatrone 110) wird eine Spritze 53 bereitgestellt, die den Knochenzementteig 154 aufnehmen kann. Durch weiteres Vortreiben des Förderkolbens 106, der Scherben 152 und des davor angeordnete Austragskolbens 107 mit dem Druckgas wird der Knochenzementteig 154 aus der Kartusche 101 ausgetrieben und in die Spritze 53 zur weiteren Verwendung gefüllt. Diese Situation ist in Figur 9 sowie den beiden Abbildungen nach Figur 10 gezeigt.

Schließlich trifft der Hohlzylinder 109 auf den Kartuschenkopf 160 beziehungsweise den Einsatz 158 im Kartuschenkopf 160 an der Vorderseite des Innenraums der Kartusche 101. Dabei wird die Nut 156 freigelegt und der Druck an der Rückseite des Förderkolbens 106 entweicht durch die Nut 156 und durch die Austragsöffnung im Kartuschenkopf 160.

Der Hohlzylinder 109 weist eine Höhe von 3 mm, bevorzugt von 5 mm, oder größer auf, so dass durch den dadurch erzeugten Abstand gewährleistet ist, dass die Vorderseite des Austragskolbens 107 von der Vorderseite des Innenraums der Kartusche 101 beabstandet ist, wenn der Austragskolben 107 so weit nach vorne gedrückt es, wie es möglich ist. Dadurch entsteht im Innenraum der Kartusche 101 und zwar in dem von dem Hohlzylinder 109 begrenzten Bereich ein Totvolumen, das nicht aus der Kartusche 101 durch die Austragsöffnung, das Leitungselement 137 und das Rohr 162 ausgetrieben werden kann.

In diesem Totvolumen befindet sich nun ein Teil des Knochenzementteigs 154, der gegebenenfalls einen zu großen Anteil an Monomerflüssigkeit 104 enthält. Dieser Teil kann des Knochenzementteigs 154 kann nicht aus dem Totvolumen aus der Vorrichtung ausgepresst werden. Durch diesen Aufbau wird sichergestellt, dass kein Knochenzementteig 154 mit einer sich ändernden Konsistenz aufgrund sich ändernder Zusammensetzung mit der Vorrichtung appliziert werden kann.

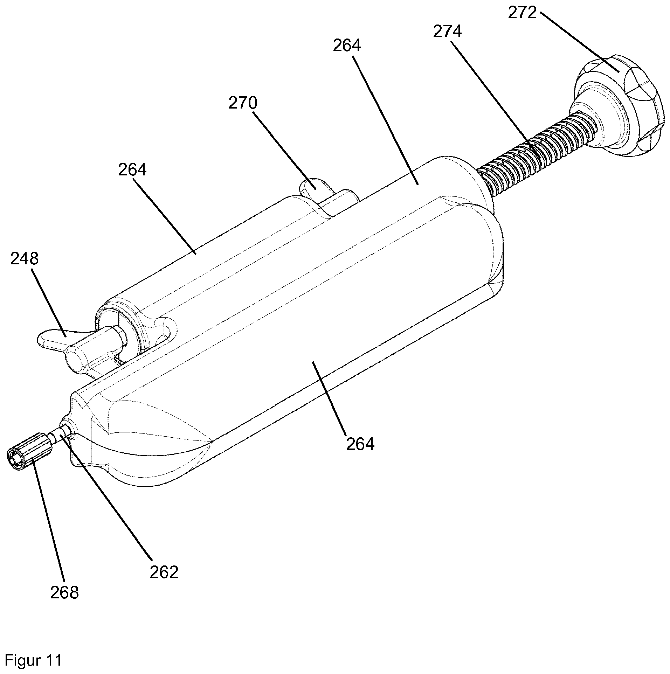

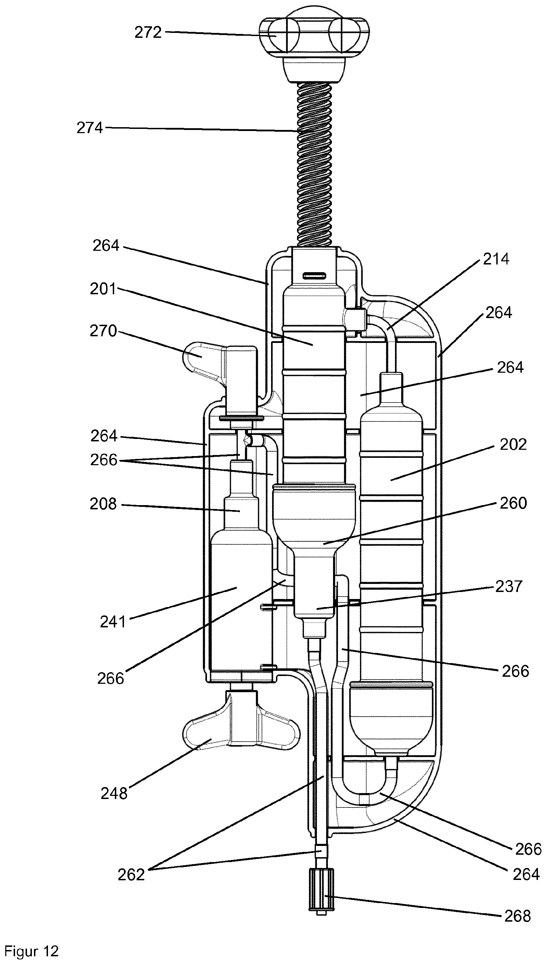

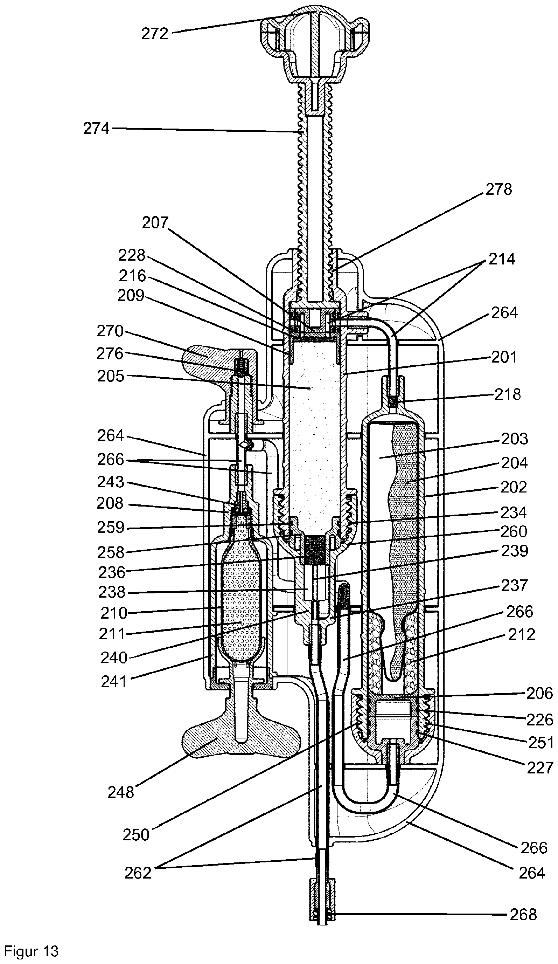

In den Figuren 11 bis 15 sind Abbildungen einer dritten alternativen erfindungsgemäßen Vorrichtung gezeigt. Die Figuren 11 bis 15 zeigen verschiedene schematische Gesamtansichten der beispielhaften zweiten erfindungsgemäßen Vorrichtung. In Figur 11 ist die dritte Vorrichtung von außen mit einem geschlossenen Gehäuse 264 gezeigt, in Figur 12 mit geöffnetem Gehäuse 264 und in den Figuren 13 bis 15 sind Querschnittansichten der Vorrichtung während des Ablaufs eines erfindungsgemäßen Verfahrens gezeigt.

Die erste erfindungsgemäße Vorrichtung umfasst eine Kartusche 201 mit einem zylindrischen Innenraum und eine Monomeraufnahme 202 für eine Glasampulle 203 als Monomerflüssigkeitsbehälter bildet. Anstelle der Glasampulle 203 kann auch ein Kunststoffbehälter oder durch geringe Umbaumaßnahmen auch ein aufreißbarer Folienbeutel aus einem Metall-beschichteten Kunststoff verwendet werden.

Sowohl der Innenraum der Kartusche 201 als auch der Innenraum der Monomeraufnahme 202 sind zylindrisch mit kreisförmiger Grundfläche. Die Monomeraufnahme 202 und die Kartusche 201 werden vorzugsweise mit Hilfe einer Spritzgusstechnik aus Kunststoff hergestellt. Die Monomeraufnahme 202 weist also einen zylindrischen Innenraum auf, in den die Glasampulle 203 eingesteckt ist. In der Glasampulle 203 befindet sich die Monomerflüssigkeit 204. In den Innenraum der Kartusche 201 ist ein Zementpulver 205 eingefüllt oder vorzugsweise eingepresst. Die Monomerflüssigkeit 204 und das Zementpulver 205 bilden die Ausgangskomponenten für einen PMMA-Knochenzement, der mit der Vorrichtung herstellbar ist. Aufgrund der Glasampulle 203 kann die Monomerflüssigkeit 204 sehr lange in der Monomeraufnahme 202 und dadurch in der Vorrichtung gelagert werden. Das Zementpulver 205 kann ebenfalls über längere Zeiträume in der Vorrichtung gelagert werden. Die Vorrichtung ist damit zum Lagern der Monomerflüssigkeit 204 und des Zementpulvers 205 als Ausgangskomponenten eines Knochenzementteigs 254 des PMMA-Knochenzements geeignet. Die Vorrichtung ist aber auch zum Mischen des Knochenzementteigs 254 aus den Ausgangskomponenten und zum Austragen des gemischten Knochenzementteigs 254 geeignet und vorgesehen.

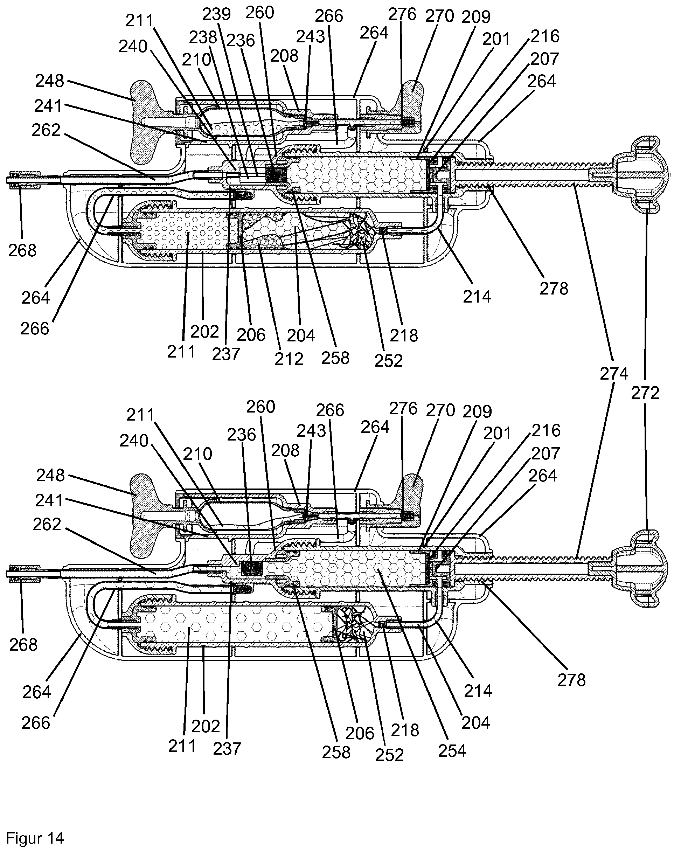

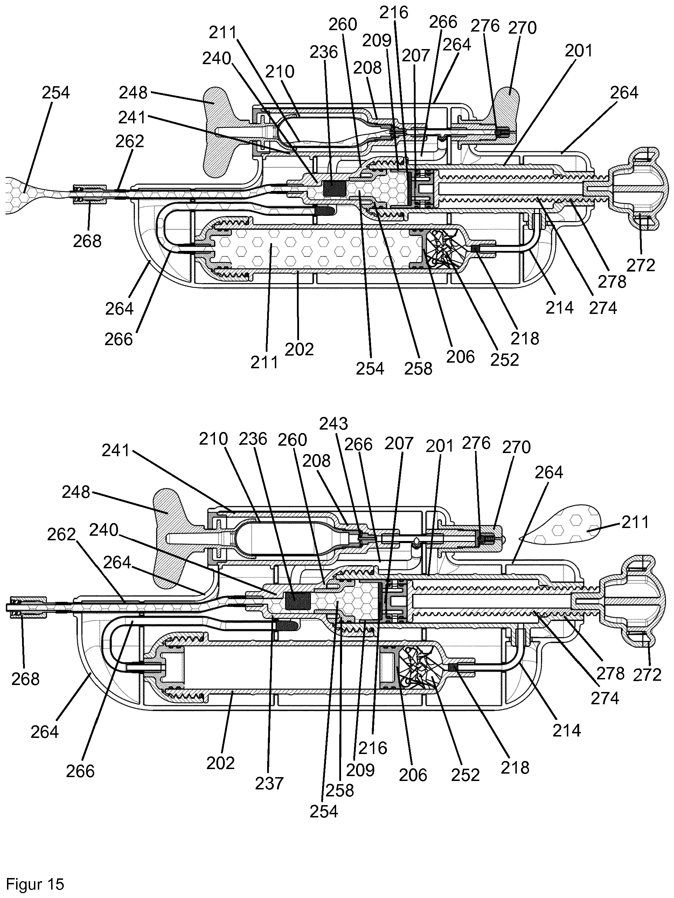

Im zylindrischen Innenraum der Monomeraufnahme 202 ist ein in Längsrichtung beweglicher Förderkolben 206 aus Kunststoff angeordnet. Der Förderkolben 206 ist im Bereich der Rückseite der Monomeraufnahme 202 (in den Figuren 12 und 13 unten und in den Figuren 14 und 15 links) angeordnet. Die Glasampulle 203 kann mit dem Förderkolben 206 in der Monomeraufnahme 202 zusammengedrückt und dabei zersplittert werden, indem der Förderkolben 206 in Richtung der Vorderseite der Monomeraufnahme 202 gedrückt wird. Der Förderkolben 206 weist an der Vorderseite Abstreifer auf, mit denen Splitter 252 der Glasampulle 203 von der Innenwand der Monomeraufnahme 202 abgestreift werden. Die Abstreifer liegen hierzu seitlich an der Innenwand des Innenraums der Monomeraufnahme 202 an.

In dem Innenraum der Kartusche 201 ist in dessen Rückseite (in den Figuren 12 und 13 oben und in den Figuren 14 und 15 rechts) ein Austragskolben 207 aus Kunststoff angeordnet. Ein Druckgasanschluss 208 ist seitlich neben der Kartusche 201 und parallel zur Kartusche 201 in einem Gehäuse 264 angeordnet. Das Gehäuse 264 schließt die Kartusche 201, die Monomeraufnahme 202, den Druckgasanschluss 208 und weitere Teile der Vorrichtung nach außen ab. Über den Standfuß 264 sind die Monomeraufnahme 202, die Kartusche 201 und der Druckgasanschluss 208 nebeneinander angeordnet und gehalten. Die Kartusche 201 und die Monomeraufnahme 202 sind über eine Verbindung 214 in Form einer Leitung 214 und eines Durchgangs 214 durch den Austragskolben 207 miteinander flüssigkeitsdurchlässig verbunden. Der Druckgasanschluss 208 ist über eine Druckgasleitung 266 mit der Rückseite des Förderkolbens 206 und der Rückseite der Monomeraufnahme 202 gasdurchlässig verbunden. Mit dem Druckgasanschluss 208 und über die Druckgasleitung 266 kann eine Druckgaspatrone 210 als Druckgasquelle mit dem Innenraum der Monomeraufnahme 202 verbunden werden, so dass ein Druckgas 211 aus der Druckgaspatrone 210 hinter dem Förderkolben 206 in den Innenraum der Monomeraufnahme 202 einströmen und den Förderkolben 206 mittels des Gasdrucks in Richtung eines Kartuschenkopfs 260 and der Kartusche 201 vortreiben kann.

Der Austragskolben 207 weist an seiner Vorderseite einen Hohlzylinder 209 zum Verlängern der Strecke auf, die die Monomerflüssigkeit 204 durch das Zementpulver 205 fließen muss, bis sie zur Innenwand der Kartusche 201 gelangt. Zudem dient der Hohlzylinder 209 zur Beabstandung des Austragskolbens 207 von einer Austragsöffnung an der Vorderseite des Innenraums der Kartusche 201 sowie zum Schaffen eins Totvolumens zwischen dem Austragskolben 207 und der Vorderseite des Innenraums der Kartusche 201, wenn der Austragskolben 207 beziehungsweise der Hohlzylinder 209 maximal an die Vorderseite des Innenraums der Kartusche 201 gedrückt ist. Der Hohlzylinder 209 ist vorliegend rotationssymmetrisch und ist nach Art eines geschlitzten Rohrstücks geformt. Der Hohlzylinder 209 hat dazu parallel zur Zylinderachse des Hohlzylinders 209 verlaufende Längsschnitte.

In dem Innenraum der Monomeraufnahme 202 ist eine Lagerung 212 aus Schaumstoff vorgesehen, die als Transportsicherung und als Stoßsicherung für die Glasampulle 203 dient. Damit soll verhindert werden, dass die Glasampulle 203 bei Erschütterungen oder Stößen ungewollt aufbricht.

Die Kartusche 201 und die Monomeraufnahme 202 sind separat voneinander als nebeneinander angeordnete Kunststoffteile ausgeführt. Die Monomeraufnahme 202 und die Kartusche 201 sind für die Monomerflüssigkeit 204 flüssigkeitsdurchlässig miteinander über die Verbindung 214 im Austragskolben 207 die Leitung und eine Öffnung in der Seitenwand der Kartusche 201 verbunden. Die Verbindung 214 mündet im Austragskolben 207 durch einen für das Zementpulver 205 undurchlässigen aber für die Monomerflüssigkeit 204 durchlässigen Porenfilter 216 in den Innenraum der Kartusche 201.

In der Einmündung zur Verbindung 214 ist in dem Austragskolben 207 ein Filter 218 angeordnet, mit dem die Splitter 252 der Glasampulle 203 zurückgehalten werden können. Statt dem Filter 218 oder zusätzlich zum Filter 218 kann auch ein Sieb vorgesehen sein.

Der zylindrische Förderkolben 206 hat einen zur Zylindergeometrie des Innenraums der Monomeraufnahme 202 passenden Außenumfang und ist über zwei umlaufende Dichtungen 226 gegen die Innenwand der Monomeraufnahme 202 flüssigkeitsdicht und druckdicht abgedichtet. Ebenso ist der Einsatz der Einmündung der Druckgasleitung 266 über die zwei äußeren umlaufenden Dichtungen 227 druckdicht gegen die Innenwand der Monomeraufnahme 202 abgedichtet. Ferner ist der Austragskolben 207 über zwei umlaufende Dichtungen 228 gegen die Innenwand der Kartusche 201 flüssigkeitsdicht abgedichtet. Die Dichtungen 226, 227 dienen dazu, dass der Gasdruck aus der Gasdruckpatrone 210 nicht entweichen kann und für den Vortrieb des Förderkolbens 206 zur Verfügung steht. Die Dichtungen 228 dienen dazu, einen Austritt von Monomerflüssigkeit 204 oder von Knochenzementteig 254 zu verhindern, um eine Kontamination der Umgebung (des OP-Saals und des Anwenders) zu verhindern. Die Dichtungen 226, 227, 228 können hierzu aus Gummi bestehen.

An der Vorderseite der Kartusche 201 ist ein Anschluss 234 in Form eines Außengewindes vorgesehen, auf das der Kartuschenkopf 260 als Abschluss der Kartusche 201 aufgeschraubt ist. In dem Kartuschenkopf 260 ist die Austragsöffnung gebildet und im Ausgangszustand (siehe Figuren 12 und 13) mit einem Verschluss 236 verschlossen, der in der Austragsöffnung steckt und diese verschließt. Der Verschluss 236 wird erst zum Austragen des gemischten Knochenzementteigs 254 geöffnet (siehe Figur 14 unten und Figur 15 oben). Der Verschluss 236 ist ein für das Zementpulver 205 undurchlässiger aber für Gase durchlässiger Porenfilter und hat eine zylindrische Form. Der Verschluss 236 ist vorzugsweise aus Porex oder einem anderen offenporigen Kunststoff gefertigt.

An dem Außengewinde 234 an der Vorderseite der Kartusche 201 ist der Kartuschenkopf 260 aufgeschraubt, der ein Leitungselement 237 mit einer Verschlussaufnahme 238 zur Aufnahme des Verschlusses 236 umfasst. Die Verschlussaufnahme 238 ist nach Art einer Hülse geformt und weist vier in Längsrichtung ausgerichtete, sich in die Verschlussaufnahme 238 hinein erhebende Leisten 239 auf. Die Leisten 239 beabstanden den Verschluss 236 von der Innenwand der Verschlussaufnahme 238, wenn der Verschluss 236 in die Verschlussaufnahme 238 hineingedrückt ist. Vor der Verschlussaufnahme 238 verengt sich das Leitungselement 237. In diesem Bereich sind vier weitere Leisten 240 angeordnet, die einen Anschlag 240 für die Bewegung des Verschlusses 236 bilden und also die Bewegung des Verschlusses 236 in die Verschlussaufnahme 238 begrenzen. Zwischen den Leisten 239, 240 ist ein ausreichender freier Leitungsquerschnitt vorgesehen, so dass ein aus den Ausgangskomponenten 204, 205 hergestellter Knochenzementteig 254 (siehe Figur 14 unten und Figur 15 oben) zwischen den Leisten 239, der Wandung der Verschlussaufnahme 238 und dem eingeschobenen Verschluss 236 sowie zwischen den Leisten 240 im vorderen Teil des Leitungselements 237 hindurch strömen kann.

An der Vorderseite des Kartuschenkopfs 260 ist ein Rohr 262 angeschlossen, das in einem Luer-Lock-Adapter 268 endet und durch das der Knochenzementteig 254 abgefüllt werden kann oder an ein Kyphoplastie-System (nicht gezeigt) oder einen Spine-Applikator (nicht gezeigt) weitergeleitet werden kann, das oder der an den Luer-Lock-Adapter 268 angeschlossen werden kann.

In dem Gehäuse 264 ist ein Behälter 241 für die Druckgaspatrone 210 vorgesehen, der ebenfalls als Kunststoffteil durch Spritzguss gefertigt werden kann und der parallel zu der Kartusche 201 und der Monomeraufnahme 202 in dem Gehäuse 264 angeordnet ist. Der Behälter 241 umfasst an seiner Vorderseite, die der Druckgasleitung 266 zugewandt ist, den Druckgasanschluss 208 auf. Die Druckgaspatrone 210 wird von der Rückseite in den Behälter 241 für die Druckgaspatrone 210 eingesteckt und der Behälter 241 mit einem rückseitigen Deckel geschlossen.

Durch den als Porenfilter ausgeführten Verschluss 236 hindurch können das Innere der Kartusche 201 und das Zementpulver 205 mit Hilfe von Ethylenoxid sterilisiert werden, da das Leitungselement 237 offen ist und der Verschluss 236 und die Zwischenräume zwischen den Pulverpartikeln des Zementpulvers 205 luftdurchlässig sind. Gleichzeitig kann Luft aus der Monomeraufnahme 202 durch die Verbindung 214, das Zementpulver 205, den Verschluss 236 und das offene Leitungselement 237 herausgepresst werden, wenn der Förderkolben 206 in Richtung der Monomeraufnahme 202 gepresst wird.

Das Zementpulver 205 ist in der Kartusche 201 eingeschlossen, da alle Öffnungen und Verbindungen 214 mit Hilfe der Porenfilter 216, 236 für das Zementpulver 205 undurchlässig verschlossen sind. Der Inhalt der Kartusche 201 kann dabei durch Evakuieren und Spülen mit Ethylenoxid sterilisiert werden. Dadurch ist die Vorrichtung auch zum langfristigen Lagern des Zementpulvers 205 geeignet.

Der Druckgasanschluss 208 weist eine Hohlnadel 243 auf, mit der die Druckgaspatrone 210 zu öffnen ist, wenn diese mit einer Membran an ihrer Vorderseite auf die Hohlnadel 243 geschoben wird. Dann strömt das Druckgas 211 aus der Druckgaspatrone 210 durch die Hohlnadel 243, die Druckgasleitung 266 und einen Sterilfilter in der Druckgasleitung 266 in den Innenraum der Monomeraufnahme 202 hinter dem Förderkolben 206.

Die Druckgaspatrone 210 kann geöffnet werden, indem ein Flügelschraubkopf 248 gedreht wird, der durch eine Durchführung im rückseitigen Deckel des Behälters 241 für die Druckgaspatrone 210 reicht und am Boden der Druckgaspatrone 210 befestigt ist. Die Druckgaspatrone 210 weist an ihrer der Hohlnadel 243 zugewandten Vorderseite ein Außengewinde auf und der Druckgasanschluss 208 ein dazu passendes Innengewinde. Mit dem Flügelschraubenkopf 248 kann die Druckgaspatrone 210 innerhalb des Behälters 241 nach vorne tiefer in den Druckgasanschluss 208 geschraubt werden, so dass die Membran an der Vorderseite der Druckgaspatrone 210 von der Hohlnadel 243 durchstochen wird und das Druckgas 211 innerhalb der Vorrichtung zur Verfügung steht (siehe Figur 14 oben).

Durch den auf die Rückseite des Förderkolbens 206 wirkenden Druck wird der Förderkolben 206 in Richtung des Kartuschenkopfs 260 gedrückt und die Glasampulle 203 zwischen dem Förderkolben 206 und dem Austragskolben 207 zersplittert und dadurch geöffnet. Die Splitter 252 der Glasampulle 203 werden weiter komprimiert und die freigewordene Monomerflüssigkeit 204 wird durch den Filter 218, durch die Verbindungen 214 und durch den Porenfilter 216 in den Innenraum der Kartusche 201 und damit in das Zementpulver 205 gepresst. Der Austragskolben 207 wird dabei von dem Zementpulver 205 gehalten, da das Zementpulver 205 im trockenen Zustand nicht fließfähig ist. Im Innenraum der Kartusche 201 mischt sich die Monomerflüssigkeit 204 mit dem Zementpulver 205, da in dem Zementpulver 205 ein Additiv verteilt ist, das die Monomerflüssigkeit 204 leitet und dadurch in dem Zementpulver 205 verteilt, bevor die Monomerflüssigkeit 204 mit dem Zementpulver 205 derart reagiert und quillt, dass eine weitere Ausbreitung der Monomerflüssigkeit 204 unterbunden wird. Zusätzlich kann die Monomerflüssigkeit 204 entlang des Hohlzylinders 209 tief in das Zementpulver 205 eindringen. In dem Innenraum der Kartusche 201 wird so der Knochenzementteig 254 gebildet (siehe Figur 14 unten).

Der mit der Vorrichtung erzeugte Knochenzementteig 254 kann mit der Vorrichtung in eine Spritze (nicht gezeigt) gefüllt werden, mit der der Knochenzementteig 254 durch den Anwender (den Operateur) beim Patienten appliziert werden kann. Alternativ kann der Knochenzementteig 254 über ein Applikationssystem, das an dem Luer-Lock-Adapter 268 angeschlossen ist, appliziert werden.

Um die Vorrichtung druckfrei zu machen, ist in der Druckgasleitung 266 ein Abzweig vorgesehen, der zu einem Druckablassventil 276 führt, das mit einem Ventilgriff 270 von außen bedient werden kann. Wenn die Monomerflüssigkeit 204 in die Kartusche 201 gepresst worden ist, kann das Druckablassventil 276 geöffnet werden und das Druckgas 211 nach außen abgeblasen werden. Dadurch wird die Vorrichtung drucklos und kann gefahrlos weiterverwendet und anschließend entsorgt werden.

Der Verschluss 236 ist im geschlossenen Zustand in einem Einsatz 258 am Kartuschenkopf 260 angeordnet, der vorne in den Innenraum der Kartusche 201 ragt und der mit zwei umlaufenden Dichtungen 259 gegen die Innenwand der Kartusche 201 abgedichtet ist. Der Einsatz 258 bildet dabei einen Anschlag für den Hohlzylinder 209 an der Vorderseite des Austragskolbens 207 und damit für den Austragskolben 207.

Die Figuren 13 bis 15 zeigen fünf schematische Querschnittansichten der erfindungsgemäßen Vorrichtung zur Verdeutlichung des Ablaufs eines erfindungsgemäßen Verfahrens. Dazu zeigen Figur 11 und 12 eine Außenansicht und eine Teilschnittansicht der Vorrichtung im Ausgangszustand. Zu Beginn des Verfahrens liegt die Vorrichtung im Ausgangszustand vor, so wie sie auch in Figur 13 gezeigt ist. In diesem Zustand wird die Vorrichtung sterilisiert.

Anschließend wird die Druckgaspatrone 210 geöffnet, indem die Druckgaspatrone 210 mit der Flügelkopfschraube 248 auf die Hohlnadel 243 geschraubt wird. Diese Situation ist in Figur 14 oben gezeigt.

Dann beginnt der wesentliche Teil des erfindungsgemäßen Verfahrens:
Das aus der Druckgaspatrone 210 austretende Druckgas 211 strömt durch die Druckgasleitung 266 und durch den Sterilfilter und drückt auf den Förderkolben 206 und diesen in Richtung der Verbindung 214 nach vorne. Durch das weiter aus der Druckgaspatrone 210 in den rückseitigen Innenraum der Monomeraufnahme 202 strömenden Druckgas 211 wird der Förderkolben 206 in Richtung der Verbindung 214 vorgetrieben. Die Lagerung 212 wird komprimiert und der Förderkolben 206 trifft auf den Kopf der Glasampulle 203. Da die Glasampulle 203 an der Vorderseite der Monomeraufnahme 202 anliegt und sich der Innenraum der Monomeraufnahme 202 weiter verkleinert, wird die Glasampulle 203 zerbrochen. Die Monomerflüssigkeit 204 tritt aus der Glasampulle 203 in den Innenraum der Monomeraufnahme 202 aus. Überstehende Luft aus der Monomeraufnahme 202 wird durch den Filter 218, die Verbindung 214, den Porenfilter 216, durch die Zwischenräume zwischen den Partikeln des Zementpulvers 205, durch den Verschluss 236 und aus dem Leitungselement 237 aus der Vorrichtung herausgedrückt.

Von der Glasampulle 203 bleiben schließlich nur kleine Splitter 252 zurück, die von dem Filter 218 zurückgehalten werden und in der Monomeraufnahme 202 verbleiben. Die Monomerflüssigkeit 204 wird durch den Filter 218, die Verbindung 214 und den Porenfilter 216 in das Zementpulver 205 gepresst und beginnt dort mit dem Zementpulver 205 zu reagieren, so dass sich aus dem Gemisch der Knochenzementteig 254 bildet. Dabei kann die Monomerflüssigkeit 204 nicht direkt von dem Porenfilter 216 aus zur Innenwand der Kartusche 201 fließen, da diese von dem geschlitzten Hohlzylinder 209 abgedeckt ist. Dadurch wird die Monomerflüssigkeit 204 gezwungen sich einen Weg durch das Zementpulver 205 zu bahnen. Monomerflüssigkeitsblasen oder Monomerflüssigkeitsansammlungen können so verhindert werden.

Die Menge der Monomerflüssigkeit 204 ist so gewählt, dass das Zementpulver 205 bis in die vorderste Spitze der Kartusche 201, das heißt, bis an den Verschluss 236 heran mit der Monomerflüssigkeit 204 benetzt wird (siehe Figur 14 unten).

Anschließend kann der Knochenzementteig 254 aus der Kartusche 201 durch Vortreiben des Austragskolbens 207 aus der Kartusche 201 und dem Rohr 262 ausgetrieben werden. Hierzu ist eine Gewindestange 274 vorgesehen, die mit einem Drehgriff 272 von außen bedient werden kann. In der Rückseite der Kartusche 201 ist hierzu ein Innengewinde 278 vorgesehen, in dem die Gewindestange 274 geschraubt werden kann. Durch Einschrauben der Gewindestange 274 wird der Austragskolben 207 in Richtung des Kartuschenkopfs 260 getrieben. Dabei wird die seitliche Öffnung in der Wand der Kartusche 201 durch den nach vorne getriebenen Austragskolben 207 und die Dichtungen 228 verschlossen.

Der Verschluss 236 wird von dem auf den Knochenzementteig 254 aufgrund des mit der Gewindestange 274 vermittelten Drucks auf den Austragskolben 207 nach vorne getrieben und in die Verschlussaufnahme 238 hineingedrückt, bis der Verschluss 236 auf den Anschlag 240 trifft, wo die Bewegung des Verschlusses 236 endet. Der Knochenzementteig 254 umfließt den Verschluss 236, indem er zwischen den Leisten 239 und zwischen den Leisten 240 hindurchfließt. Schließlich tritt der Knochenzementteig 254 an der Vorderseite der Vorrichtung durch das Rohr 262 aus.

Durch weiteres Vortreiben des Austragskolbens 207 mit der Gewindestange 274 wird der Knochenzementteig 254 aus der Kartusche 201 ausgetrieben. Diese Situation ist in Figur 15 oben gezeigt.

Schließlich trifft der Hohlzylinder 209 auf den Kartuschenkopf 260 beziehungsweise den Einsatz 258 im Kartuschenkopf 260 an der Vorderseite des Innenraums der Kartusche 201. Anschließend wird das Druckablassventil 276 mit dem Ventilgriff 270 geöffnet und das Druckgas 211 aus der Vorrichtung abgeblasen. Diese Situation ist in Figur 15 unten dargestellt.

Der Hohlzylinder 209 weist eine Höhe von 3 mm, bevorzugt von 5 mm, oder größer auf, so dass durch den dadurch erzeugten Abstand gewährleistet ist, dass die Vorderseite des Austragskolbens 207 von der Vorderseite des Innenraums der Kartusche 201 beabstandet ist, wenn der Austragskolben 207 so weit nach vorne gedrückt es, wie es möglich ist. Dadurch entsteht im Innenraum der Kartusche 201 und zwar in dem von dem Hohlzylinder 209 begrenzten Bereich ein Totvolumen, das nicht aus der Kartusche 201 durch die Austragsöffnung, das Leitungselement 237 und das Rohr 262 ausgetrieben werden kann.

In diesem Totvolumen befindet sich nun ein Teil des Knochenzementteigs 254, der gegebenenfalls einen zu großen Anteil an Monomerflüssigkeit 204 enthält. Dieser Teil kann des Knochenzementteigs 254 kann nicht aus dem Totvolumen aus der Vorrichtung ausgepresst werden. Durch diesen Aufbau wird sichergestellt, dass kein Knochenzementteig 254 mit einer sich ändernden Konsistenz aufgrund sich ändernder Zusammensetzung mit der Vorrichtung appliziert werden kann.

Die dritte erfindungsgemäße Vorrichtung nach den Figuren 11 bis 15 unterscheidet sich von der ersten nach den Figuren 1 bis 3 vor allem dadurch, dass der Druckgasanschluss 208 nicht direkt an der Monomeraufnahme 202 platziert ist, und von den ersten beiden Ausführungen nach den Figuren 1 bis 10 dadurch, dass die Kartusche 201 und die Monomeraufnahme 202 keinen gemeinsamen Behälter bilden, sondern wie auch der Behälter 241 parallel zueinander in dem gemeinsamen Gehäuse 264 angeordnet sind.

Die dritte erfindungsgemäße Vorrichtung kann zusammen mit einem sogenannten Spine-Applikator für die Spondylodese oder mit einem Kyphoplastie-System eingesetzt werden, indem das Kyphoplastie-System beziehungsweise der Spine-Applikator an den Luer-Lock-Adapter 268 angeschlossen wird. Der Spine-Applikator oder das Kyphoplastie-System wird zur Wirbelverblockung beziehungsweise Wirbelversteifung zweier Wirbelkörper oder zum Auffüllen von Hohlräumen in den Wirbelkörpern eingesetzt, indem Knochenzementteig 254 unter Röntgenkontrolle mit Hilfe eines Trokars (nicht gezeigt) im Bereich der Wirbel appliziert wird. Durch den Trokar muss der Arzt nicht in der Röntgenstrahlung arbeiten.

In einer weiteren alternativen vierten Ausführungsform umfasst das System nach der dritten Ausführungsform nach den Figuren 11 bis 15 noch eine Spritze 53 oder ein Kyphoplastie-System oder einen Spine-Applikator als zusätzliches Bauteil, das lösbar mit der Vorrichtung verbunden ist und das über das Rohr 262 gefüllt werden kann. Nachdem der Knochenzementteig 254 gemischt und durch das Rohr 262 beziehungsweise den Luer-Lock-Adapter 268 in das zusätzliche Bauteil überführt worden ist, kann dieses entnommen werden und der Knochenzementteig 254 mit diesem Bauteil angewendet werden.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1, 101, 201: Kartusche
- 2, 102, 202: Monomeraufnahme
- 3, 103, 203: Ampulle
- 4, 104, 204: Monomerflüssigkeit
- 5, 105, 205: Zementpulver
- 6, 106, 206: Förderkolben
- 7, 107, 207: Austragskolben
- 8, 108, 208: Druckgasanschluss
- 9, 109, 209: Hohlzylinder
- 10, 110, 210: Druckgaspatrone
- 12, 112, 212: Lagerung
- 14, 114, 214: Verbindung
- 16, 116, 216: Porenfilter
- 18, 118, 218: Filter
- 20, 120: Belüftungsöffnung
- 26, 126, 226: Dichtung
- 27, 127, 227: Dichtung
- 28, 128, 228: Dichtung
- 34, 134, 234: Anschluss
- 36, 136, 236: Verschluss / Porenfilter
- 37, 137, 237: Leitungselement
- 38, 138, 238: Verschlussaufnahme
- 39, 139, 239: Leiste / Abstandhalter
- 40, 140, 240: Anschlag / Leiste
- 41, 141, 241: Behälter für Druckgas
- 42, 142: Keil
- 43, 143, 243: Hohlnadel
- 44, 144: Sterilfilter
- 46: Flügel
- 48, 148, 248: Flügelschraubenkopf
- 50, 150, 250: Innengewinde
- 51, 151, 251: Außengewinde
- 52, 152, 252: Splitter
- 53: Spritze
- 54, 154, 254: Knochenzementteig
- 55: Kolben der Spritze
- 56, 156: Nut
- 58, 158, 258: Einsatz
- 59, 159, 259: Dichtung
- 60, 160: Kartuschenkopf
- 162, 262: Rohr
- 164: Standfuß / Gehäuse
- 166, 266: Druckgasleitung
- 167: Krimphülse
- 211: Druckgas
- 264: Gehäuse
- 268: Luer-Lock-Adapter
- 270: Ventilgriff
- 272: Drehgriff
- 274: Gewindestange
- 276: Druckablassventil
- 278: Innengewinde

## Patentansprüche

1. Vorrichtung zum Herstellen eines Knochenzementteigs (54, 154, 254) aus einer Monomerflüssigkeit (4, 104, 204) und einem Zementpulver (5, 105, 205) als Ausgangskomponenten des Knochenzementteigs (54, 154, 254), die Vorrichtung aufweisend
eine Kartusche (1, 101, 201) mit einem zylindrischen Innenraum zum Mischen der Ausgangskomponenten, wobei der Innenraum der Kartusche (1, 101, 201) an der Vorderseite bis auf eine Austragsöffnung zum Austreiben des Knochenzementteigs (54, 154, 254) aus dem Innenraum geschlossen ist,
einen Austragskolben (7, 107, 207), der im Innenraum der Kartusche (1, 101, 201) angeordnet ist und der in Richtung der Austragsöffnung linear bewegbar gelagert ist, das Zementpulver (5, 105, 205), das im Innenraum der Kartusche (1, 101, 201) zwischen der Austragsöffnung und dem Austragskolben (7, 107, 207) angeordnet ist, eine Monomeraufnahme (2, 102, 202) mit einem Innenraum, in dem ein Monomerflüssigkeitsbehälter (3, 103, 203) enthaltend die Monomerflüssigkeit (4, 104, 204) enthalten ist, wobei in der Monomeraufnahme (2, 102, 202) ein in Längsrichtung der Monomeraufnahme (2, 102, 202) beweglicher Förderkolben (6, 106, 206) angeordnet ist,
einen Druckgasanschluss (8, 108, 208), der direkt oder über eine Druckgasleitung (166, 266) druckdicht mit dem Innenraum der Monomeraufnahme (2, 102, 202) verbunden ist, wobei der Förderkolben (6, 106, 206) zwischen dem Monomerflüssigkeitsbehälter (3, 103, 203) und dem Druckgasanschluss (8, 108, 208) oder der Druckgasleitung (166, 266) in der Monomeraufnahme (2, 102, 202) angeordnet ist, und
eine Verbindung (14, 114, 214), die den Innenraum der Monomeraufnahme (2, 102, 202) und den Innenraum der Kartusche (1, 101, 201) für die Monomerflüssigkeit (4, 104, 204) durchlässig aber für das Zementpulver (5, 105, 205) undurchlässig miteinander verbindet, wobei der Monomerflüssigkeitsbehälter (3, 103, 203) zwischen dem Förderkolben (6, 106, 206) und der Verbindung (14, 114, 214) angeordnet ist.

2. Vorrichtung nach Anspruch 1, **dadurch gekennzeichnet, dass**
der Förderkolben (6, 106, 206) mit einem über den Druckgasanschluss (8, 108, 208) in den Innenraum der Monomeraufnahme (2, 102, 202) geleiteten Gasdruck in Richtung der Verbindung (14, 114, 214) drückbar ist und der Monomerflüssigkeitsbehälter (3, 103, 203) durch die Bewegung des Förderkolbens (6, 106, 206) zu öffnen ist, insbesondere aufbrechbar ist, und die Monomerflüssigkeit (4, 104, 204) aus dem Innenraum der Monomeraufnahme (2, 102, 202) durch die Verbindung (14, 114, 214) in den Innenraum der Kartusche (1, 101, 201) drückbar ist, wobei bevorzugt
der Förderkolben (6, 106, 206) für Gase undurchlässig ist und gegen die Innenwände der Monomeraufnahme (2, 102, 202) gasdicht abgedichtet ist, besonders bevorzugt mit wenigstens einer umlaufenden Dichtung (26, 126, 226).

3. Vorrichtung nach einem der vorangehenden Ansprüche, **dadurch gekennzeichnet, dass**
die Verbindung (14, 114, 214) wenigstens einen Durchgang im Austragskolben (7, 107, 207) aufweist, wobei der wenigstens eine Durchgang für die Monomerflüssigkeit (4, 104, 204) und für Gase durchlässig ist und für das Zementpulver (5, 105, 205) undurchlässig ist, wobei vorzugsweise die zum Zementpulver (5, 105, 205) ausgerichtete Oberfläche des Austragskolbens (7, 107, 207) für das Zementpulver (5, 105, 205) undurchlässig ist, und
der Monomerflüssigkeitsbehälter (3, 103, 203) enthaltend die Monomerflüssigkeit (4, 104, 204) zwischen dem Förderkolben (6, 106, 206) und dem Austragskolben (7, 107, 207) angeordnet ist.

4. Vorrichtung nach einem der vorangehenden Ansprüche, **dadurch gekennzeichnet, dass**
der Druckgasanschluss (8, 108, 208) ein Dichtmittel zum druckdichten Anschließen einer Druckgasquelle (10, 110, 210) aufweist, insbesondere zum druckdichten Anschließen einer Druckgaspatrone (10, 110, 210) aufweist, und
die Vorrichtung eine Druckgaspatrone (10, 110, 210) aufweist, die an dem Druckgasanschluss (8, 108, 208) druckdicht angeschlossen ist oder anschließbar ist, wobei vorzugsweise die Druckgaspatrone (10, 110, 210) eine CO₂-Patrone (10, 110, 210) ist.

5. Vorrichtung nach einem der vorangehenden Ansprüche, **dadurch gekennzeichnet, dass**
der Druckgasanschluss (8, 108, 208) ein Ventil oder eine Öffnungseinrichtung (43, 143, 243) umfasst, wobei die Öffnungseinrichtung (43, 143, 243) zum Öffnen einer verschlossenen Druckgaspatrone (10, 110, 210) und zum Herstellen einer druckdichten Verbindung zwischen dem Druckgasanschluss (8, 108, 208) und der Druckgaspatrone (10, 110, 210) geeignet ist, wobei vorzugsweise die Druckgaspatrone (10, 110, 210) und die Öffnungseinrichtung (43, 143, 243) gegeneinander verschiebbar in der Vorrichtung gelagert sind und sich durch Zusammenschieben der Druckgaspatrone (10, 110, 210) und der Öffnungseinrichtung (43, 143, 243) die Druckgaspatrone (10, 110, 210) in der Vorrichtung zu öffnen ist, so dass Druckgas (211) aus der Druckgaspatrone (10, 110, 210) in den Innenraum der Monomeraufnahme (2, 102, 202) strömt, und/oder
die Vorrichtung ferner einen Behälter (41, 141, 241) für eine Druckgaspatrone (10, 110, 210) aufweist, wobei eine in den Behälter (41, 141, 241) eingesetzte Druckgaspatrone (10, 110, 210) in der Vorrichtung durch eine Bewegung der Druckgaspatrone (10, 110, 210) gegen den Druckgasanschluss (8, 108, 208) derart zu öffnen ist, dass das Druckgas (211) aus der Druckgaspatrone (10, 110, 210) in den Druckgasanschluss (8, 108, 208) strömt, wobei vorzugsweise die Druckgaspatrone (10, 110, 210) durch eine Schraubbewegung gegen den Druckgasanschluss (8, 108, 208) zu bewegen ist.

6. Vorrichtung nach einem der vorangehenden Ansprüche, **dadurch gekennzeichnet, dass**
im Druckgasanschluss (8, 108, 208) oder in der Druckgasleitung (166, 266) ein Ablassventil (276) zum Ablassen eines Überdrucks an die Umgebung angeordnet ist, insbesondere ein geschlossenes von außen manuell bedienbares Ablassventil (276) oder ein geschlossenes mechanisch oder elektrisch öffenbares Ablassventil, und/oder zwischen der Verbindung (14, 114, 214) und dem Monomerflüssigkeitsbehälter (3, 103, 203) ein elastisch verformbarer Abstandhalter angeordnet ist, wobei bevorzugt der Abstandhalter den Monomerflüssigkeitsbehälter (3, 103, 203) zumindest 3 mm von der Verbindung (14, 114, 214) beabstandet, besonders bevorzugt zumindest 6 mm von der Verbindung (14, 114, 214) beabstandet, ganz besonders bevorzugt zumindest 10 mm von der Verbindung (14, 114, 214) beabstandet.

7. Vorrichtung nach einem der vorangehenden Ansprüche, **dadurch gekennzeichnet, dass**
die Verbindung (214) über eine Einmündung in einer Seitenwand der Kartusche (201) in den Innenraum der Kartusche (201) mündet, wobei die Einmündung von einer Seitenfläche des Austragskolbens (207), die parallel zur Bewegungsrichtung des Austragskolbens (207) liegt, abgedeckt ist, wobei der Austragskolben (207) eine für die Monomerflüssigkeit (204) durchlässige Durchführung in den Innenraum der Kartusche (201) zum Zementpulver (205) aufweist, die sich von einer Seitenfläche des Austragskolbens (207) bis zu einer vorderen Grundfläche des Austragskolbens (207) erstreckt, wobei die Grundfläche des Austragskolbens (207) senkrecht zur Bewegungsrichtung des Austragskolbens (207) liegt, wobei bevorzugt die Durchführung für das Zementpulver (205) undurchlässig ist und besonders bevorzugt für Gase durchlässig ist, und/oder
in dem Zementpulver (5, 105, 205) ein die Monomerflüssigkeit (4, 104, 204) leitendes Additiv verteilt ist, wobei vorzugsweise das Zementpulver (5, 105, 205) mit dem Additiv beschichtet oder mit dem Additiv gemischt ist.

8. Vorrichtung nach einem der vorangehenden Ansprüche, **dadurch gekennzeichnet, dass**
der Innenraum der Kartusche (1, 101, 201) und der Innenraum der Monomeraufnahme (2, 102, 202) einen gemeinsamen zylindrischen Innenraum bilden und miteinander fluchten, so dass der Austragskolben (7, 107, 207) mit dem Förderkolben (6, 106, 206) in dem Innenraum der Kartusche (1, 101, 201) vortreibbar ist und der Förderkolben (6, 106, 206) in den Innenraum der Kartusche (1, 101, 201) drückbar ist, wobei vorzugsweise
an der Innenwand der Kartusche (1, 101, 201) im Bereich der Vorderseite ein Bypass oder eine Nut (56, 156) vorgesehen ist, durch den oder durch die das Druckgas (211) an dem Förderkolben (6, 106, 206) und dem Austragskolben (7, 107, 207) vorbei strömen kann, wenn der Förderkolben (6, 106, 206) eine Öffnung zum Bypass oder die Nut (56, 156) zumindest bereichsweise überfahren hat und dadurch zum Druckgasanschluss (8, 108, 208) freilegt.

9. Vorrichtung nach einem der vorangehenden Ansprüche, **dadurch gekennzeichnet, dass**
an dem Druckgasanschluss (8, 108, 208) oder in der Druckgasleitung (166, 266) ein geschlossenes Überdruckventil angeordnet ist, das bei Überschreiten eines Grenzdrucks den Druckgasanschluss (8, 108, 208) oder die Druckgasleitung (166, 266) nach außen zur Umgebung öffnet, und
die Vorrichtung einen Verschluss (36, 136, 236) aufweist, der die Austragsöffnung verschließt und der gegen die Austragsöffnung beweglich gelagert ist, wobei ein Leitungselement (37, 137, 237) an der Vorderseite der Austragsöffnung angeordnet ist, wobei das Leitungselement (37, 137, 237) eine Verschlussaufnahme (38, 138, 238) zum Aufnehmen zumindest eines Teils des Verschlusses (36, 136, 236) umfasst, und wobei der Verschluss (36, 136, 236) durch einen Druck auf den Knochenzementteig (54, 154, 254) derart in die Verschlussaufnahme (38, 138, 238) hinein drückbar ist, dass die Austragsöffnung geöffnet ist, wobei das Leitungselement (37, 137, 237) mit dem in die Verschlussaufnahme (38, 138, 238) gedrückten Verschluss (36, 136, 236) einen freien Leitungsquerschnitt bereitstellt, durch den der Knochenzementteig (54, 154, 254) aus der Austragsöffnung hindurch und aus der Vorrichtung heraus drückbar ist, wobei vorzugsweise
der Verschluss (36, 136, 236) für Gase durchlässig aber für das Zementpulver (5, 105, 205) undurchlässig ist.

10. Vorrichtung nach einem der vorangehenden Ansprüche, **dadurch gekennzeichnet, dass**
die Rückseite der Kartusche (1, 101, 201) mit der Vorderseite der Monomeraufnahme (2, 102, 202) verbunden ist, vorzugsweise derart verbunden ist, dass der Innenraum der Kartusche (1, 101, 201) mit dem Innenraum der Monomeraufnahme (2, 102, 202) fluchtet, und
in dem Druckgasanschluss (8, 108, 208) oder in der Monomeraufnahme (2, 102, 202) eine Belüftungsöffnung (20, 120) vorgesehen ist, wobei die Belüftungsöffnung (20, 120) durch eine Bewegung des Druckgasanschlusses (8, 108, 208) oder durch eine Bewegung eines Behälters (41, 141, 241) für eine Druckgaspatrone (10, 110, 210) verschließbar ist, und
an der der Austragsöffnung zugewandten Vorderseite des Austragskolbens (7, 107, 207) ein Hohlzylinder (9, 109, 209) angeordnet ist, wobei der Hohlzylinder (9, 109, 209) an seiner der Austragsöffnung zugewandten Vorderseite offen ist und der Hohlzylinder (9, 109, 209) sich vorzugsweise von der Vorderseite des Austragskolbens (7, 107, 207) zumindest 3 mm in den Innenraum der Kartusche (1, 101, 201) erstreckt.

11. Vorrichtung nach einem der vorangehenden Ansprüche, **dadurch gekennzeichnet, dass**
das Zementpulver (5, 105, 205) an der Vorderseite des Austragskolbens (7, 107, 207) anliegt, insbesondere vollflächig anliegt, wobei vorzugsweise das Zementpulver (5, 105, 205) in den Innenraum der Kartusche (1, 101, 201) eingepresst ist, und/oder das Zementpulver (5, 105, 205) den Innenraum der Kartusche (1, 101, 201) zwischen der geschlossenen Vorderseite und dem Austragskolben (7, 107, 207) vollständig ausfüllt, wobei vorzugsweise das Zementpulver (5, 105, 205) in den Innenraum der Kartusche eingepresst ist.

12. Verfahren zur Herstellung eines Knochenzementteigs (54, 154, 254), vorzugsweise mit einer Vorrichtung nach einem der Ansprüche 1 bis 11, wobei der Knochenzementteig (54, 154, 254) aus einem Zementpulver (5, 105, 205) und einer Monomerflüssigkeit (4, 104, 204) hergestellt wird, wobei die Monomerflüssigkeit (4, 104, 204) in einem Monomerflüssigkeitsbehälter (3, 103, 203) enthalten ist, der in einer Monomeraufnahme (2, 102, 202) angeordnet ist, und wobei das Zementpulver (5, 105, 205) in einer Kartusche (1, 101, 201) enthalten ist, **gekennzeichnet durch** die folgenden nacheinander ablaufenden Schritte:
a) Antreiben und Bewegen eines Förderkolbens (6, 106, 206) in der Monomeraufnahme (2, 102, 202) mit einem Druckgas (211), wobei mit der Bewegung des Förderkolbens (6, 106, 206) die Monomerflüssigkeit (4, 104, 204) aus dem Monomerflüssigkeitsbehälter (3, 103, 203) und aus der Monomeraufnahme (2, 102, 202) in den Innenraum einer Kartusche (1, 101, 201) gedrückt wird, so dass sich die Monomerflüssigkeit (4, 104, 204) mit dem Zementpulver (5, 105, 205) in der Kartusche (1, 101, 201) mischt und dort den Knochenzementteig (54, 154, 254) bildet,
b) der gemischte Knochenzementteig (54, 154, 254) wird mit einem Austragskolben (7, 107, 207) aus einer Austragsöffnung an einer dem Austragskolben (7, 107, 207) gegenüberliegende Seite der Kartusche (1, 101, 201) herausgedrückt.

13. Verfahren nach Anspruch 12, **dadurch gekennzeichnet, dass**
die Monomerflüssigkeit (4, 104, 204) in dem Zementpulver (5, 105, 205) mit Hilfe eines die Monomerflüssigkeit (4, 104, 204) leitenden Additivs verteilt wird, wobei Partikel des Zementpulvers (5, 105, 205) mit dem Additiv beschichtet sind oder mit dem Additiv vermischt sind, und/oder
der Monomerflüssigkeitsbehälter (3, 103, 203) durch die Bewegung des vom Druckgas (211) angetriebenen Förderkolbens (6, 106, 206) geöffnet wird.

14. Verfahren nach einem der Ansprüche 12 oder 13, **dadurch gekennzeichnet, dass** der gemischte Knochenzementteig (54, 154, 254) aus der Kartusche (1, 101, 201) in einen Applikator (53) oder eine Spritze (53) gefüllt wird und/oder
in Schritt b) der Austragskolben (7, 107, 207) von dem vom Druckgas (211) angetriebenen Förderkolben (6, 106, 206) in Richtung der Austragsöffnung gedrückt wird.

15. Verfahren nach einem der Ansprüche 12 bis 14, **dadurch gekennzeichnet, dass** ein Verschluss (36, 136, 236) durch den von dem Knochenzementteig (54, 154, 254) auf den Verschluss (36, 136, 236) wirkenden Druck in eine Verschlussaufnahme (38, 138, 238) gedrückt und dabei die Austragsöffnung geöffnet wird, wobei vorzugsweise beim Herausdrücken des Knochenzementteigs (54, 154, 254) aus der Austragöffnung der Knochenzementteig (54, 154, 254) durch einen durch das Öffnen des Verschlusses (36, 136, 236) entstandenen freien Leitungsquerschnitt fließt und aus der Vorrichtung ausgetragen wird, und
vor Schritt a) eine Druckgaspatrone (10, 110, 210) geöffnet wird und das Druckgas (211) aus der Druckgaspatrone (10, 110, 210) durch einen Druckgasanschluss (8, 108, 208) in die Monomeraufnahme (2, 102, 202) zum Förderkolben (6, 106, 206) geleitet wird.

## Claims

1. A device for producing a bone cement paste (54, 154, 254) from a monomer liquid (4, 104, 204) and a cement powder (5, 105, 205) as parent components of the bone cement paste (54, 154, 254), the device having
a cartridge (1, 101, 201) with a cylindrical interior chamber for mixing the parent components, whereby the interior chamber of the cartridge (1, 101, 201) is closed on the front side up to a delivery opening for expelling the bone cement paste (54, 154, 254) from the interior chamber,
a delivery plunger (7, 107, 207) which is arranged in the interior chamber of the cartridge (1, 101, 201) and which is supported in a linearly movable manner in the direction of the delivery opening,
the cement powder (5, 105, 205) which is arranged in the interior chamber of the cartridge (1, 101, 201) between the delivery opening and the delivery plunger (7, 107, 207),
a monomer receptacle (2, 102, 202) with an interior chamber in which a monomer liquid container (3, 103, 203) containing the monomer liquid (4, 104, 204) is contained, whereby in the monomer receptacle (2, 102, 202) a conveying plunger (6, 106, 206) is arranged that is movable in the longitudinal direction of the monomer receptacle (2, 102, 202),
a compressed gas connection (8, 108, 208) which is directly connected or connected via a compressed gas line (166, 266) in a pressure-tight manner with the interior chamber of the monomer receptacle (2, 102, 202), whereby the conveying plunger (6, 106, 206) is arranged between the monomer liquid container (3, 103, 203) and the compressed gas connection (8, 108, 208) or the compressed gas line (166, 266) in the monomer receptacle (2, 102, 202), and
a connection (14, 114, 214) which connects the interior chamber of the monomer receptacle (2, 102, 202) and the interior chamber of the cartridge (1, 101, 201) which is permeable for the monomer liquid (4, 104, 204) but impermeable for the cement powder (5, 105, 205), whereby the monomer liquid container (3, 103, 203) is arranged between the conveying plunger (6, 106, 206) and the connection (14, 114, 214).

2. The device according to Claim 1, **characterized in that**
the conveying plunger (6, 106, 206) is pressable with a gas pressure that is guided via the compressed gas connection (8, 108, 208) into the interior chamber of the monomer receptacle (2, 102, 202) in the direction of the connection (14, 114, 214), and the monomer liquid container (3, 103, 203) is openable by the movement of the conveying plunger (6, 106, 206), in particular is break-openable, and the monomer liquid (4, 104, 204) from the interior chamber of the monomer receptacle (2, 102, 202) is pressable through the connection (14, 114, 214) into the interior chamber of the cartridge (1, 101, 201), wherein preferably
the conveying plunger (6, 106, 206) is impermeable for gases and is sealed in a gas-tight manner against the interior walls of the monomer receptacle (2, 102, 202), particular preferably with at least one circumferential seal (26, 126, 226).

3. The device according to any one of the preceding Claims, **characterized in that**
the connection (14, 114, 214) comprises at least one passage inside the delivery plunger (7, 107, 207), whereby the at least one passage is permeable for the monomer liquid (4, 104, 204) and for gases, and is impermeable for the cement powder (5, 105, 205), whereby preferably, the surface of the delivery plunger (7, 107, 207) that is aligned for the cement powder (5, 105, 205) is impermeable for the cement powder (5, 105, 205), and
the monomer liquid container (3, 103, 203) containing the monomer liquid (4, 104, 204) is arranged between the conveying plunger (6, 106, 206) and the delivery plunger (7, 107, 207).

4. The device according to any one of the preceding Claims, **characterized in that**
the compressed gas connection (8, 108, 208) has a sealing means for the pressure-tight connection of a compressed gas source (10, 110, 210), in particular for the pressure-tight connection of a compressed gas cartridge (10, 110, 210) and
the device has a compressed gas cartridge (10, 110, 210) which is connected or connectable in a pressure-tight manner to the compressed gas connection (8, 108, 208), whereby preferably the compressed gas cartridge (10, 110, 210) is a CO₂ cartridge (10, 110, 210).

5. The device according to any one of the preceding Claims, **characterized in that**
the compressed gas connection (8, 108, 208) comprises a valve or an opening device (43, 143, 243), whereby the opening device (43, 143, 243) is suitable for opening a closed compressed gas cartridge (10, 110, 210) and for producing a pressure-tight connection between the compressed gas connection (8, 108, 208) and the compressed gas cartridge (10, 110, 210), whereby preferably the compressed gas cartridge (10, 110, 210) and the opening device (43, 143, 243) are supported in the device such that they are movable against each other and the compressed gas cartridge (10, 110, 210) is to be opened in the device through the pushing together of the compressed gas cartridge (10, 110, 210) and the opening device (43, 143, 243), so that compressed gas (211) flows from the compressed gas cartridge (10, 110, 210) into the interior chamber of the monomer receptacle (2, 102, 202), and/or
the device further has a container (41, 141, 241) for a compressed gas cartridge (10, 110, 210), whereby a compressed gas cartridge (10, 110, 210) inserted into the container (41, 141, 241) is to be opened in the device by a movement of the compressed gas cartridge (10, 110, 210) against the compressed gas connection (8, 108, 208) such that the compressed gas (211) flows out of the compressed gas cartridge (10, 110, 210) into the compressed gas connection (8, 108, 208), whereby preferably the compressed gas cartridge (10, 110, 210) is to be moved by a screw movement against the compressed gas connection (8, 108, 208).

6. The device according to any one of the preceding Claims, **characterized in that**
in the compressed gas connection (8, 108, 208) or in the compressed gas line (166, 266), a discharge valve (276) is arranged for discharging an overpressure into the environment, in particular a closed discharge valve (276) that is manually operatable from the outside or a closed discharge valve that is mechanically or electrically openable, and/or
between the connection (14, 114, 214) and the monomer liquid container (3, 103, 203), an elastically deformable spacer is arranged, whereby preferably, the spacer separates the monomer liquid container (3, 103, 203) from the connection (14, 114, 214) by at least 3 mm, particularly preferred at a distance of at least 6 mm from the connection (14, 114, 214), very particularly preferred at a distance of at least 10 mm from the connection (14, 114, 214).

7. The device according to any one of the preceding Claims, **characterized in that**
the connection (214) opens out into the interior chamber of the cartridge (201) via a confluence in a side wall of the cartridge (201), whereby the confluence is covered by a side area of the delivery plunger (207) which lies parallel to the direction of movement of the delivery plunger (207), whereby the delivery plunger (207) has a passage into the interior chamber of the cartridge (201) to the cement powder (205) which is permeable for the monomer liquid (204), which extends from a side area of the delivery plunger (207) up to a front base area of the delivery plunger (207), whereby the base area of the delivery plunger (207) lies vertical to the direction of movement of the delivery plunger (207), whereby preferably the passage is impermeable for the cement powder (205) and particularly preferrably is permeable for gases, and/or
an additive conducting the monomer liquid (4, 104, 204) is distributed in the cement powder (5, 105, 205), whereby preferably the cement powder (5, 105, 205) is coated with the additive or mixed with the additive.

8. The device according to any one of the preceding Claims, **characterized in that**
the interior chamber of the cartridge (1, 101, 201) and the interior chamber of the monomer receptacle (2, 102, 202) form a shared cylindrical interior chamber and align with each other, so that the delivery plunger (7, 107, 207) is forward-drivable with the conveying plunger (6, 106, 206) in the interior chamber of the cartridge and the delivery plunger (6, 106, 206) is pressable into the interior chamber of the cartridge (1, 101, 201), wherein preferably
on the interior wall of the cartridge (1, 101, 201) in the area of the front side, a bypass or a groove (56, 156) is provided, through which the compressed gas (211) can flow past the conveying plunger (6, 106, 206) and the delivery plunger (7, 107, 207) when the conveying plunger (6, 106, 206) has traversed an opening to the bypass or the groove (56, 156) at least in regions, and as a result has opened it up to the compressed gas connection (8, 108, 208).

9. The device according to any one of the preceding Claims, **characterized in that**
on the compressed gas connection (8, 108, 208) or in the compressed gas line (166, 266) a closed overpressure valve is arranged which when a threshold pressure is exceeded opens up the compressed gas connection (8, 108, 208) or the compressed gas line (166, 266) outwards to the environment, and
the device has a closure (36, 136, 236) that closes the delivery opening and which is movably supported against the delivery opening, whereby a line element (37, 137, 237) is arranged on the front side of the delivery opening, whereby the line element (37, 137, 237) comprises a closure holder (38, 138, 238) for holding at least a portion of the closure (36, 136, 236), and whereby the closure (36, 136, 236) is pressable into the closure holder (38, 138, 238) through a pressure onto the bone cement paste (54, 154, 254) such that the delivery opening is opened, whereby the line element (37, 137, 237) with the closure (36, 136, 236) pressed into the closure holder (38, 138, 238) provides a free line cross-section, through which the bone cement paste (54, 154, 254) is squeezable out through the delivery opening and out of the device, wherein preferably the closure (36, 136, 236) is permeable for gases but impermeable for the cement powder (5, 105, 205).

10. The device according to any one of the preceding Claims, **characterized in that** the rear side of the cartridge (1, 101, 201) is connected with the front side of the monomer receptacle (2, 102, 202), preferably connected such that the interior chamber of the cartridge (1, 101, 201) aligns with the interior chamber of the monomer receptacle (2, 102, 202), and
In the compressed gas connection (8, 108, 208) or in the monomer receptacle (2, 102, 202), a ventilation opening (20, 120) is provided, whereby the ventilation opening (20, 120) is closable through a movement of the compressed gas connection (8, 108, 208) or through a movement of a container (41, 141, 241) for a compressed gas cartridge (10, 110, 210), and
on the front side of the delivery plunger (7, 107, 207) facing towards the delivery opening, a hollow cylinder (9, 109, 209) is arranged, whereby the hollow cylinder (9, 109, 209) is open on its front side facing towards the delivery opening and the hollow cylinder (9, 109, 209) preferably extends from the front side of the delivery plunger (7, 107, 207) at least 3 mm into the interior chamber of the cartridge (1, 101, 201), and

11. The device according to any one of the preceding Claims, **characterized in that**
the cement powder (5, 105, 205) rests against the front side of the delivery plunger (7, 107, 207), in particular over its full surface, whereby preferably, the cement powder (5, 105, 205) is pressed into the interior chamber of the cartridge (1, 101, 201), and/or the cement powder (5, 105, 205) completely fills out the interior chamber of the cartridge (1, 101, 201) between the closed front side and the delivery plunger (7, 107, 207), whereby preferably the cement powder (5, 105, 205) is pressed into the interior chamber of the cartridge.

12. A method for producing a bone cement paste (54, 154, 254), preferably using a device according to any one of Claims 1 to 11, whereby the bone cement paste (54, 154, 254) is produced from a cement powder (5, 105, 205) and a monomer liquid (4, 104, 204), whereby the monomer liquid (4, 104, 204) is contained in a monomer liquid container (3, 103, 203), which is arranged in a monomer receptacle (2, 102, 202), and whereby the cement powder (5, 105, 205) is contained in a cartridge (1, 101, 201), **characterized by** the following sequential steps:
a) Driving and moving a conveying plunger (6, 106, 206) in the monomer receptacle (2, 102, 202) with a compressed gas (211), whereby with the movement of the conveying plunger (6, 106, 206) the monomer fluid (4, 104, 204) from the monomer fluid container (3, 103, 203) and from the monomer receptacle (2, 102, 202) is pressed into the interior chamber of a cartridge (1, 101, 201), so that the monomer fluid (4, 104, 204) mixes with the cement powder (5, 105, 205) in the cartridge (1, 101, 201) and there forms the bone cement paste (54, 154, 254),
b) The mixed bone cement paste (54, 154, 254) is pressed out of a delivery opening with a delivery plunger (7, 107, 207) on a side of the cartridge (1, 101, 201) opposite the delivery plunger (7, 107, 207).

13. The method according to Claim 12, **characterized in that**
the monomer liquid (4, 104, 204) is distributed in the cement powder (5, 105, 205) with the aid of an additive that conducts the monomer liquid (4, 104, 204), whereby particles of the cement powder (5, 105, 205) are coated with the additive or mixed with the additive, and/or
the monomer liquid container (3, 103, 203) is opened by the movement of the conveying plunger (6, 106, 206) driven by the compressed gas (211).

14. The method according to any one of Claims 12 or 13, **characterized in that**
the mixed bone cement paste (54, 154, 254) from the cartridge (1, 101, 201) is filled into an applicator (53) or a syringe (53), and/or
in step b), the delivery plunger (7, 107, 207) is pushed by the conveying plunger (6, 106, 206) driven by the compressed gas (211) in the direction of the delivery opening.

15. The method according to any one of Claims 12 to 14, **characterized in that**
a closure (36, 136, 236) is pressed by the pressure of the bone cement paste (54, 154, 254) acting on the closure (36, 136, 236) into a closure holder (38, 138, 238) and at the same time, the delivery opening is opened, whereby preferably, when pressing out the bone cement paste (54, 154, 254) from the delivery opening, the bone cement paste (54, 154, 254) flows through a free line cross-section created by the opening of the closure (36, 136, 236) and is delivered out of the device, and
before step a), a compressed gas cartridge (10, 110, 210) is opened and the compressed gas (211) is guided from the compressed gas cartridge (10, 110, 210) through a compressed gas connection (8, 108, 208) into the monomer receptacle (2, 102, 202) to the conveying plunger (6, 106, 206).

## Revendications

1. Dispositif de fabrication d'une pâte de ciment osseux (54, 154, 254) à partir d'un liquide de monomère (4, 104, 204) et d'une poudre de ciment (5, 105, 205) servant de composants de départ de la pâte de ciment osseux (54, 154, 254), le dispositif présentant
une cartouche (1, 101, 201) dotée d'un espace intérieur cylindrique permettant le mélange des composants de départ, où l'espace intérieur de la cartouche (1, 101, 201) est fermé au niveau du côté avant jusqu'à un orifice de sortie pour l'expulsion de la pâte de ciment osseux (54, 154, 254) hors de l'espace intérieur,
un piston d'évacuation (7, 107, 207) qui est disposé dans l'espace intérieur de la cartouche (1, 101, 201) et qui est logé en étant mobile linéairement en direction de l'orifice de sortie,
la poudre de ciment (5, 105, 205) qui est disposée dans l'espace intérieur de la cartouche (1, 101, 201) entre l'orifice de sortie et le piston d'évacuation (7, 107, 207),
un réceptacle de monomère (2, 102, 202) doté d'un espace intérieur dans lequel est contenu un récipient de liquide de monomère (3, 103, 203) contenant le liquide de monomère (4, 104, 204), où un piston de transport (6, 106, 206) mobile dans la direction longitudinale du réceptacle de monomère (2, 102, 202) est disposé dans le réceptacle de monomère (2, 201, 202),
un raccord de gaz sous pression (8, 108, 208) qui est relié de manière étanche en pression directement ou par le biais d'une conduite de gaz sous pression (166, 266) à l'espace intérieur du réceptacle de monomère (2, 102, 202), où le piston de transport (6, 106, 206) est disposé entre le récipient de liquide de monomère (3, 103, 203) et le raccord de gaz sous pression (8, 108, 208) ou la conduite de gaz sous pression (166, 266) dans le réceptacle de monomère (2, 102, 202), et
une connexion (14, 114, 214) qui relie l'espace intérieur du réceptacle de monomère (2, 102, 202) et l'espace intérieur de la cartouche (1, 101, 201) l'un à l'autre de manière à laisser passer le liquide de monomère (4, 104, 204), mais ne laissant pas passer la poudre de ciment (5, 105, 205), où le récipient de liquide de monomère (3, 103, 203) est disposé entre le piston de transport (6, 106, 206) et la connexion (14, 114, 214).

2. Dispositif selon la revendication 1, **caractérisé en ce que**
le piston de transport (6, 106, 206) peut être pressé avec une pression de gaz mené dans l'espace intérieur du réceptacle de monomère (2, 102, 202) en direction de la connexion (14, 114, 214) par le biais du raccord de gaz sous pression (8, 108, 208), et le récipient de liquide de monomère (3, 103, 203) peut être ouvert par le déplacement du piston de transport (6, 106, 206), notamment peut être cassé, et le liquide de monomère (4, 104, 204) peut être pressé hors de l'espace intérieur du réceptacle de monomère (2, 102, 202) à travers la connexion (14, 114, 214) dans l'espace intérieur de la cartouche (1, 101, 201),
où, de préférence,
le piston de transport (6, 106, 206) ne laisse pas passer les gaz et est rendu étanche vis-à-vis des gaz contre les parois intérieures du réceptacle de monomère (2, 102, 202), de manière particulièrement préférée à l'aide d'au moins un joint (26, 126, 226) périphérique.

3. Dispositif selon l'une des revendications précédentes, **caractérisé en ce que**
la connexion (14, 114, 214) présente au moins un passage dans le piston d'évacuation (7, 107, 207), où l'au moins un passage laisse passer le liquide de monomère (4, 104, 204) et les gaz et ne laisse pas passer la poudre de ciment (5, 105, 205), où, de préférence, la surface du piston d'évacuation (7, 107, 207) orientée vers la poudre de ciment (5, 105, 205) ne laisse pas passer la poudre de ciment (5, 105, 205), et
le récipient de liquide de monomère (3, 103, 203) contenant le liquide de monomère (4, 104, 204) est disposé entre le piston de transport (6, 106, 206) et le piston d'évacuation (7, 107, 207).

4. Dispositif selon l'une des revendications précédentes, **caractérisé en ce que**
le raccord de gaz sous pression (8, 108, 208) présente un joint pour effectuer un raccordement étanche en pression d'une source de gaz sous pression (10, 110, 210), présente notamment pour effectuer un raccordement étanche en pression d'une cartouche de gaz sous pression (10, 110, 210), et
le dispositif présente une cartouche de gaz sous pression (10, 110, 210) qui est raccordée ou peut être raccordée de manière étanche en pression au raccord de gaz sous pression (8, 108, 208), où, de préférence, la cartouche de gaz sous pression (10, 110, 210) est une cartouche de CO₂ (10, 110, 210).

5. Dispositif selon l'une des revendications précédentes, **caractérisé en ce que**
le raccord de gaz sous pression (8, 108, 208) comprend une soupape ou un dispositif d'ouverture (43, 143, 243), où le dispositif d'ouverture (43, 143, 243) est approprié pour l'ouverture d'une cartouche de gaz sous pression (10, 110, 210) fermée et pour établir une connexion étanche en pression entre le raccord de gaz sous pression (8, 108, 208) et la cartouche de gaz sous pression (10, 110, 210), où, de préférence, la cartouche de gaz sous pression (10, 110, 210) et le dispositif d'ouverture (43, 143, 243) sont logés dans le dispositif de manière à pouvoir être glissés l'un contre l'autre et la cartouche de gaz sous pression (10, 110, 210) peut être ouverte dans le dispositif par le glissement de la cartouche de gaz sous pression (10, 110, 210) et du dispositif d'ouverture (43, 143, 243) l'un contre l'autre de sorte que le gaz sous pression (211) s'écoule hors de la cartouche de gaz sous pression (10, 110, 210) dans l'espace intérieur du réceptacle de monomère (2, 102, 202), et/ou
le dispositif présente en outre un récipient (41, 141, 241) pour une cartouche de gaz sous pression (10, 110, 210), où une cartouche de gaz sous pression (10, 110, 210) insérée dans le récipient (41, 141, 241) peut être ouverte dans le dispositif par un déplacement de la cartouche de gaz sous pression (10, 110, 210) contre le raccord de gaz sous pression (8, 108, 208) de telle manière que le gaz sous pression (211) s'écoule hors de la cartouche de gaz sous pression (10, 110, 210) dans le raccord de gaz sous pression (8, 108, 208), où, de préférence, la cartouche de gaz sous pression (10, 110, 210) peut être déplacée par un mouvement de vissage contre le raccord de gaz sous pression (8, 108, 208).

6. Dispositif selon l'une des revendications précédentes, **caractérisé en ce**
**qu'**une soupape de sortie (276) pour l'évacuation d'une surpression vers l'environnement est disposée dans le raccord de gaz sous pression (8, 108, 208) ou dans la conduite de gaz sous pression (166, 266), notamment, une soupape de sortie (276) fermée et pouvant être actionnée manuellement de l'extérieur, ou une soupape de sortie fermée mécaniquement ou pouvant être ouverte électriquement, et/ou un espaceur déformable élastiquement est disposé entre la connexion (14, 114, 214) et le récipient de liquide de monomère (3, 103, 203), où, de préférence, l'espaceur sépare le récipient de liquide de monomère (3, 103, 203) d'au moins 3 mm de la connexion (14, 114, 214), de manière particulièrement préférée, le sépare d'au moins 6 mm de la connexion (14, 114, 214), le sépare idéalement d'au moins 10 mm de la connexion (14, 114, 214).

7. Dispositif selon l'une des revendications précédentes, **caractérisé en ce que**
la connexion (214) débouche dans l'espace intérieur de la cartouche (201) par une embouchure au niveau d'une paroi latérale de la cartouche (201), où l'embouchure est recouverte par une surface latérale du piston d'évacuation (207) qui se situe parallèlement par rapport au sens de déplacement du piston d'évacuation (207), où le piston d'évacuation (207) présente un passage, laissant passer le liquide de monomère (204) dans l'espace intérieur de la cartouche (201) vers la poudre de ciment (205), qui s'étend à partir d'une surface latérale du piston d'évacuation (207) jusqu'à une surface de base avant du piston d'évacuation (207), où la surface de base du piston d'évacuation (207) se situe perpendiculairement par rapport au sens de déplacement du piston d'évacuation (207), où, de préférence, le passage ne laisse pas passer la poudre de ciment (205) et de manière particulièrement préférée laisse passer les gaz, et/ou
un additif conduisant le liquide de monomère (4, 104, 204) est dispersé dans la poudre de ciment (5, 105, 205), où, de préférence, la poudre de ciment (5, 105, 205) est revêtue avec l'additif ou est mélangée avec l'additif.

8. Dispositif selon l'une des revendications précédentes, **caractérisé en ce que**
l'espace intérieur de la cartouche (1, 101, 201) et l'espace intérieur du réceptacle de monomère (2, 102, 202) forment ensemble un espace intérieur cylindrique commun et sont en alignement l'un avec l'autre de sorte que le piston d'évacuation (7, 107, 207) peut être entraîné avec le piston de transport (6, 106, 206) dans l'espace intérieur de la cartouche (1, 101, 201) et le piston de transport (6, 106, 206) peut être pressé dans l'espace intérieur de la cartouche (1, 101, 201), où, de préférence, une dérivation ou une rainure (56, 156) est prévue dans l'espace intérieur de la cartouche (1, 101, 201) dans la région du côté avant, par laquelle le gaz sous pression (211) peut s'écouler en passant à côté du piston de transport (6, 106, 206) et du piston d'évacuation (7, 107, 207) lorsque le piston de transport (6, 106, 206) a dépassé au moins par endroits l'orifice vers la dérivation ou la rainure et est ainsi libéré vers le raccord de gaz sous pression (8, 108, 208).

9. Dispositif selon l'une des revendications précédentes, **caractérisé en ce**
**qu'**un clapet de décharge est disposé au niveau du raccord de gaz sous pression (8, 108, 208) ou dans la conduite de gaz sous pression (166, 266), qui ouvre le raccord de gaz sous pression (8, 108, 208) ou la conduite de gaz sous pression (166, 266) vers l'extérieur vers l'environnement lors d'un dépassement d'une pression limite, et le dispositif présente un obturateur (36, 136, 236) qui ferme l'orifice de sortie et qui est logé mobile contre l'orifice de sortie, où un élément de guidage (37, 137, 237) est disposé sur le côté avant de l'orifice de sortie, où l'élément de guidage (37, 137, 237) comprend un support d'obturateur (38, 138, 238) pour la réception d'au moins une partie de l'obturateur (36, 136, 236), et où l'obturateur (36, 136, 236) peut être pressé par une pression sur la pâte de ciment osseux (54, 154, 254) à l'intérieur du support d'obturateur (38, 138, 238) de telle manière que l'orifice de sortie est ouvert, où l'élément de guidage (37, 137, 237) avec l'obturateur (36, 136, 236) pressé dans le support d'obturateur (38, 138, 238) fournit une section transversale de conduite par laquelle la pâte de ciment osseux (54, 154, 254)peut être pressée à travers l'orifice de sortie et hors du dispositif, où, de préférence,
l'obturateur (36, 136, 236) laisse passer les gaz, mais ne laisse pas passer la poudre de ciment (5, 105, 205).

10. Dispositif selon l'une des revendications précédentes, **caractérisé en ce que**
le côté arrière de la cartouche (1, 101, 201) est relié avec le côté avant du réceptacle de monomère (2, 102, 202), de préférence, est relié de telle manière que l'espace intérieur de la cartouche (1, 101, 201) est aligné avec l'espace intérieur du réceptacle de monomère (2, 102, 202), et
un orifice d'aération (20, 120) est prévu dans le raccord de gaz sous pression (8, 108, 208) ou dans le réceptacle de monomère (2, 102, 202), où l'orifice d'aération (20, 120) peut être fermé par un déplacement du raccord de gaz sous pression (8, 108, 208) ou par un déplacement d'un récipient (41, 141, 241) pour une cartouche de gaz sous pression (10, 110, 210), et
un cylindre creux (9, 109, 209) est disposé sur le côté avant du piston d'évacuation (7, 107, 207) orienté vers l'orifice de sortie, où le cylindre creux (9, 109, 209) est ouvert au niveau de son côté avant orienté vers l'orifice de sortie et le cylindre creux (9, 109, 209) s'étend de préférence du côté avant du piston d'évacuation (7, 107, 207) au moins de 3 mm à l'intérieur de l'espace intérieur de la cartouche (1, 101, 201).

11. Dispositif selon l'une des revendications précédentes, **caractérisé en ce que**
la poudre de ciment (5, 105, 205) se plaque sur le côté avant du piston d'évacuation (7, 107, 207), notamment se plaque sur toute la surface, où, de préférence, la poudre de ciment (5, 105, 205) est pressée à l'intérieur de l'espace intérieur de la cartouche (1, 101, 201), et/ou
la poudre de ciment (5, 105, 205) remplit totalement l'espace intérieur de la cartouche (1, 101, 201) entre le côté avant fermé et le piston d'évacuation (7, 107, 207), où, de préférence, la poudre de ciment (5, 105, 205) est pressée à l'intérieur de l'espace intérieur de la cartouche.

12. Procédé de fabrication d'une pâte de ciment osseux (54, 154, 254), de préférence, avec un dispositif selon l'une des revendications 1 à 11, dans lequel la pâte de ciment osseux (54, 154, 254) est fabriquée à partir d'une poudre de ciment (5, 105, 205) et d'un liquide de monomère (4, 104, 204), où le liquide de monomère (4, 104, 204) est contenu dans un récipient de liquide de monomère (3, 103, 203) qui est disposé dans un réceptacle de monomère (2, 102, 202), et où la poudre de ciment (5, 105, 205) est contenue dans une cartouche (1, 101, 201), **caractérisé par** les étapes se déroulant les unes après les autres :
a) d'entraînement et de déplacement d'un piston de transport (6, 106, 206) dans le réceptacle de monomère (2, 102, 202) avec un gaz sous pression (211), où le liquide de monomère (4, 104, 204) est pressé hors du récipient de liquide de monomère (3, 103, 203) et hors du réceptacle de monomère (2, 102, 202) dans l'espace intérieur d'une cartouche (1, 101, 201) de sorte que le liquide de monomère (4, 104, 204) se mélange avec la poudre de ciment (5, 105, 205) dans la cartouche (1, 101, 201) et y forme la pâte de ciment osseux (54, 154, 254),
b) la pâte de ciment osseux (54, 154, 254) mélangée est pressée avec un piston d'évacuation (7, 107, 207) hors d'un orifice de sortie sur un côté de la cartouche (1, 101, 201) se situant en face du piston d'évacuation (7, 107, 207).

13. Procédé selon la revendication 12, **caractérisé en ce que**
le liquide de monomère (4, 104, 204) est distribué dans la poudre de ciment (5, 105, 205) à l'aide d'un additif guidant le liquide de monomère (4, 104, 204), où des particules du ciment osseux (5, 105, 205) sont revêtues avec l'additif ou sont mélangées avec l'additif, et/ou
le récipient de liquide de monomère (3, 103, 203) est ouvert par un déplacement du piston de transport (6, 106, 206) entraîné par le gaz sous pression (211).

14. Procédé selon la revendication 12 ou la revendication 13, **caractérisé en ce que**
la pâte de ciment osseux (54, 154, 254) mélangée est remplie dans un applicateur (53) ou une seringue (53) à partir de la cartouche (1, 101, 201), et/ou
dans l'étape b), le piston d'évacuation (7, 107, 207) est pressé en direction de l'orifice de sortie par le piston de transport (6, 106, 206) entraîné par le gaz sous pression (211).

15. Procédé selon l'une des revendications 12 à 14, **caractérisé en ce**
**qu'**un obturateur (36, 136, 236) est pressé dans un support d'obturateur (38, 138, 238) par la pression agissant sur l'obturateur (36, 136, 236) par la pâte de ciment osseux (54, 154, 254), et ainsi l'orifice de sortie est ouvert, où, de préférence, lors du pressage de la pâte de ciment osseux (54, 154, 254) hors de l'orifice de sortie, la pâte de ciment osseux (54, 154, 254) s'écoule par une section transversale de guidage libre générée par l'ouverture de l'obturateur (36, 136, 236) et est évacuée hors du dispositif, et
avant l'étape a), une cartouche de gaz sous pression (10, 110, 210) est ouverte et le gaz sous pression (211) est mené vers le piston de transport (6, 106, 206) à travers un raccord de gaz sous pression (8, 108, 208) dans le réceptacle de monomère (2, 102, 202).
